# EUROPEAN PATENT SPECIFICATION

(11) **EP 3 101 704 B1**
(45) Date of publication and mention of the grant of the patent: **04.09.2019**
(21) Application number: 15740592.9
(22) Date of filing: 21.01.2015
(51) Int. Cl.: H01L 51/54, H01L 51/46, H01L 51/05, C07D 495/04, C07D 495/22, C07D 493/04

(54) **ORGANIC TRANSISTOR, COMPOUND, ORGANIC SEMICONDUCTOR MATERIAL FOR NON-LIGHT-EMITTING ORGANIC SEMICONDUCTOR DEVICES, MATERIAL FOR ORGANIC TRANSISTORS, COATING SOLUTION FOR NON-LIGHT-EMITTING ORGANIC SEMICONDUCTOR DEVICES, AND ORGANIC SEMICONDUCTOR FILM FOR NON-LIGHT-EMITTING ORGANIC SEMICONDUCTOR DEVICES**
ORGANISCHER TRANSISTOR, VERBINDUNG, ORGANISCHES HALBLEITERMATERIAL FÜR NICHT-LICHTEMITTIERENDE ORGANISCHE HALBEITERBAUELEMENTE, MATERIAL FÜR ORGANISCHE TRANSISTOREN, BESCHICHTUNGSLÖSUNG FÜR NICHT-LICHTEMITTIERENDE ORGANISCHE HALBLEITERBAUELEMENTE UND ORGANISCHER HALBLEITERFILM FÜR NICHT-LICHTEMITTIERENDE ORGANISCHE HALBLEITERBAUELEMENTE
TRANSISTOR OGRANIQUE, COMPOSÉ, MATÉRIAU DE SEMI-CONDUCTEUR ORGANIQUE, MATÉRIAU POUR DES DISPOSITIFS À SEMI-CONDUCTEURS ORGANIQUES NON ÉLECTROLUMINESCENTS, MATÉRIAU POUR DES TRANSISTORS ORGANIQUES, SOLUTION DE REVÊTEMENT POUR DES DISPOSITIFS À SEMI-CONDUCTEURS ORGANIQUES NON ÉLECTROLUMINESCENTS ET FILM DE SEMI-CONDUCTEUR ORGANIQUE POUR DES DISPOSITIFS À SEMI-CONDUCTEURS ORGANIQUES NON ÉLECTROLUMINESCENTS

(30) Priority: 27.01.2014 JP 2014012058
(43) Date of publication of application: 07.12.2016
(73) Proprietor: FUJIFILM Corporation, Tokyo 106-8620 (JP)
(72) Inventor: KOYANAGI Masashi, Ashigarakami-gun Kanagawa 258-8577 (JP); HIRAI Yuki, Ashigarakami-gun Kanagawa 258-8577 (JP); MASUI Kensuke, Ashigarakami-gun Kanagawa 258-8577 (JP)
(74) Representative: Hoffmann Eitle
(86) International application number: PCT/JP2015/051488
(87) International publication number: WO 2015/111605

(56) References cited:
- WO-A1-2013/078407
- WO-A1-2014/087300
- JP-A- 2011 054 749
- JP-A- 2013 087 118
- JP-A- 2013 512 975
- LAURE BINIEK: 'New Fused Bis- Thienobenzothienothiophene Copolymers and Their Use in Organic Solar Cells and Transistors' MACROMOLECULES vol. 46, no. ISSUE, 31 January 2013, pages 727 - 735, XP055102743
- RALPH RIEGER ET AL.: 'Tetrathiahexacene as Building Block for Solution-Processable Semiconducting Polymers: Exploring the Monomer Size Limit' MACROMOLECULES vol. 43, no. ISSUE, 14 July 2010, pages 6264 - 6267, XP002660902

## Description

### BACKGROUND OF THE INVENTION

### 1. Field of the Invention

The present invention relates to an organic transistor, an organic semiconductor film, or an organic semiconductor material. Specifically, the present invention relates to a compound having a skeletal structure of condensed rings, an organic transistor containing the compound, an organic semiconductor material for a non-light-emitting organic semiconductor device that contains the compound, a material for an organic transistor that contains the compound, a coating solution for a non-light-emitting organic semiconductor device that contains the compound, an organic semiconductor film for a non-light-emitting organic semiconductor device that contains the compound, and a compound which is an intermediate for synthesizing the aforementioned compound.

### 2. Description of the Related Art

The devices using organic semiconductor materials are drawing great attention because they are expected to be superior in various aspects to the devices using inorganic semiconductor materials of the related art such as silicon. Examples of the devices using organic semiconductor materials include a photoelectric conversion element such as an organic solar cell or a solid-state imaging element using organic semiconductor materials as photoelectric conversion materials, an organic transistor (referred to as an organic thin-film transistor in some cases) having non-light-emitting properties (in the present specification, "non-light-emitting" refers to properties by which a luminous efficiency of equal to or less than 1 lm/W is obtained in a case where electric currents are applied to a device at a current density of 0.1 mW/cm² at room temperature in the atmosphere; non-light-emitting organic semiconductor devices mean organic semiconductor devices excluding light-emitting organic semiconductor devices such as organic electroluminescence elements), and the like. The devices using organic semiconductor materials are likely to make it possible to prepare large area elements at lower temperature and lower costs compared to the devices using inorganic semiconductor materials. Furthermore, the characteristics of the materials can be easily changed by varying the molecular structure thereof. Therefore, the materials show a wide variation and can realize functions or elements that cannot be obtained by inorganic semiconductor materials.

For example, WO2013/078407A discloses the use of a compound, which has a pyrrole ring on both terminals or one terminal, as an organic transistor.

Furthermore, US2008/0142792A1 discloses the use of a heteroacene compound consisting of 6 rings as an organic transistor.

JP 2011-04749 describes an organic transistor having an organic semiconductor layer and containing at least one kind of compound having the following general formula in the organic semiconductor layer: 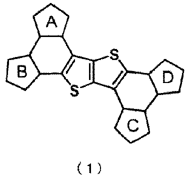 wherein the rings A to D represent a substituted or non-substituted thiophene ring.

WO 2011/067192 describes a dithienobenzo-thieno[3,2-b]thiophene-copolymer having a specified formula which is used as high performance solution processable semiconducting polymer.

JP 2013-512975 discloses the synthesis of polymers of condensed benzothiophene systems.

### SUMMARY OF THE INVENTION

Under the circumstances described above, the inventors of the present invention conducted investigation regarding the use of the compounds described in WO2013/078407A and US2008/0142792A1 in a semiconductor active layer of an organic transistor. As a result, the inventors found that the carrier mobility is low in most cases.

Through the investigation performed by the inventors of the present invention, it was confirmed that in the compounds disclosed in WO2013/078407A and US2008/0142792A1, overlapping of HOMO does not sufficiently occur, and accordingly, only a crystal structure that is undesirable as a transistor material is obtained, and thus the carrier mobility is lowered.

Therefore, in order to solve the above problems of the related art, the inventors of the present invention continued investigation. Objects of the present invention are to provide an organic transistor having high carrier mobility and to provide a compound which makes it possible to obtain an organic transistor having high carrier mobility by being used in a semiconductor active layer.

As a result of conducting thorough investigation for achieving the above objects, the inventors obtained knowledge that by condensing 2 heterocyclic rings with 2 thiophene rings, furan rings, or selenophene rings in specific positions, and substituting the resulting compound with a group represented by Formula (W), overlapping of HOMO occurs, the crystallinity is improved, and an organic transistor having high carrier mobility can be obtained. Based on the knowledge, the inventors accomplished the present invention.

The present invention as specific means for achieving the aforementioned objects is constituted as described in the claims.
[1] The compound of the present application is represented by the following Formula (1-1) or (1-2): 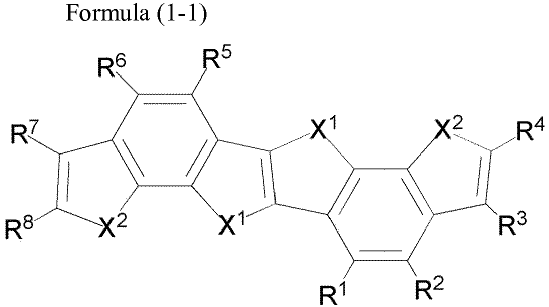 in Formula (1-1),
   each X¹ independently represents a sulfur atom, an oxygen atom, a selenium atom, or NR⁹,
   each X² independently represents a sulfur atom, an oxygen atom, or a selenium atom,
   each of R¹ to R⁹ independently represents a hydrogen atom or a substituent, and at least one of R¹, R², R³, R⁴, R⁵, R⁶, R⁷, R⁸, or R⁹ is a substituent represented by the following Formula (W) and at least one of R⁴ and R⁸ is a substituent represented by the Formula (W); wherein the substituents are as defined in claim 1; 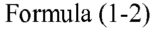 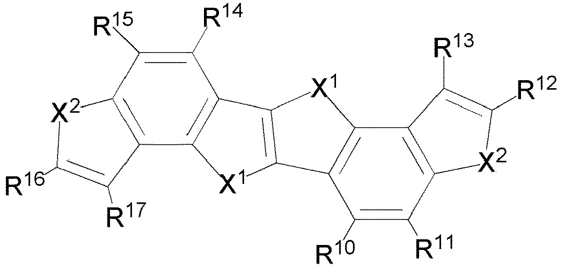
   in Formula (1-2),
   each X¹ independently represents a sulfur atom, an oxygen atom, a selenium atom, or NR⁹,
   each X² independently represents a sulfur atom, an oxygen atom, or a selenium atom,
   each of R⁹ to R¹⁷ independently represents a hydrogen atom or a substituent, and at least one of R⁹, R¹⁰, R¹¹, R¹², R¹³, R¹⁴, R¹⁵, R¹⁶, or R¹⁷ is a substituent represented by the following Formula (W) wherein the substituents are as defined in claim 1;

   -L-R Formula (W)

   in Formula (W), L represents a divalent linking group represented by any of the following Formulae (L-1) to (L-25) or a divalent linking group in which two or more divalent linking groups represented by any of the following Formulae (L-1) to (L-25) are bonded to each other, and
   R represents a substituted or unsubstituted alkyl group, a cyano group, a vinyl group, an ethynyl group, an oxyethylene group, an oligo-oxyethylene group in which a repetition number v of an oxyethylene unit is equal to or greater than 2, a siloxane group, an oligosiloxane group having two or more silicon atoms, or a substituted or unsubstituted trialkylsilyl group, wherein the total number of carbon atoms contained in L and R is 5 to 12; 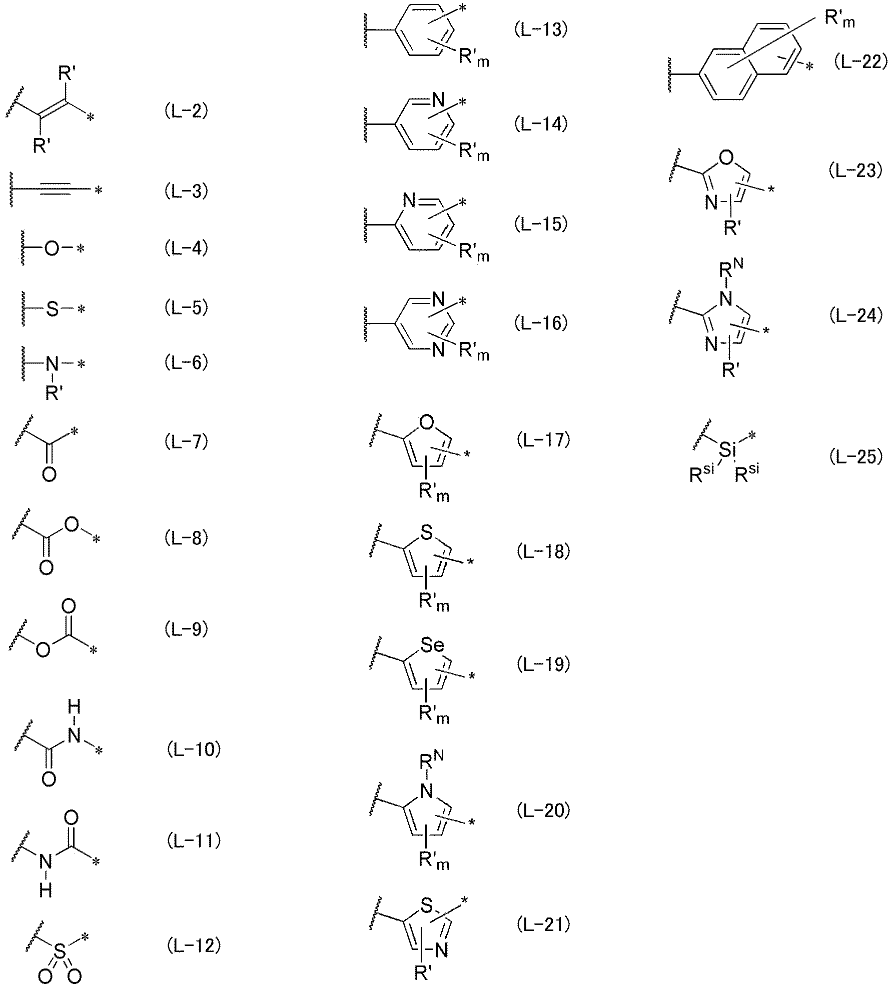
   in Formulae (L-1) to (L-25),
   the portion of a wavy line represents a position of bonding to a condensed cyclic skeleton,
   m in Formula (L-13) represents 4, m in Formulae (L-14) and (L-15) represents 3, m in Formulae (L-16) to (L-20) represents 2, m in Formula (L-22) represents 6,
   each R' in Formulae (L-1), (L-2), (L-6), and (L-13) to (L-24) independently represents a hydrogen atom or a substituent,
   R^{N} represents a hydrogen atom or a substituent, and
   each R^{si} independently represents a hydrogen atom, an alkyl group, an alkenyl group, or an alkynyl group. 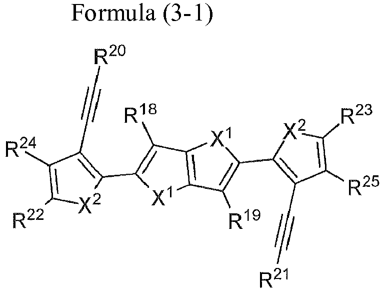
   in Formula (3-1),
   X¹ and X² have the same meaning as defined above,
   each of R⁹ and R¹⁸ to R²⁵ independently represents a hydrogen atom or a substituent, one or two of R⁹, R¹⁸, R¹⁹, R²⁰, R²¹, R²², R²³, R²⁴, and R²⁵ is a substituent represented by the Formula (W) as defined above and at least one of R²² and R²³ represents an alkyl group, an alkenyl group, an alkynyl group, a substituted or unsubstituted aryl group, a substituted or unsubstituted heteroaryl group, an alkylcarbonyl group, or a substituted or unsubstituted arylcarbonyl group; 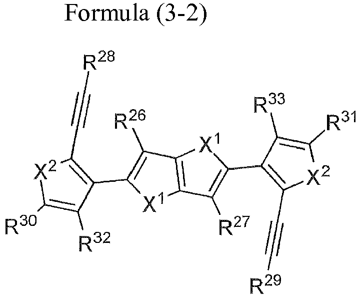
   in Formula (3-2),
   each X¹ independently represents a sulfur atom, an oxygen atom, a selenium atom, or NR⁹,
   each X² independently represents a sulfur atom, an oxygen atom, or a selenium atom,
   each of R⁹ and R²⁶ to R³³ independently represents a hydrogen atom or a substituent, and at least one of R³⁰ and R³¹ represents an alkyl group, an alkenyl group, an alkynyl group, a substituted or unsubstituted aryl group, a substituted or unsubstituted heteroaryl group, an alkylcarbonyl group, or a substituted or unsubstituted arylcarbonyl group.
[3] The compound represented by Formula (1-1) or (1-1) is preferably a compound represented by the following Formula (2-1-1) or (2-1-2), and
   the compound represented by Formula (1-2) is preferably a compound represented by the following Formula (2-2-1) or (2-2-2): 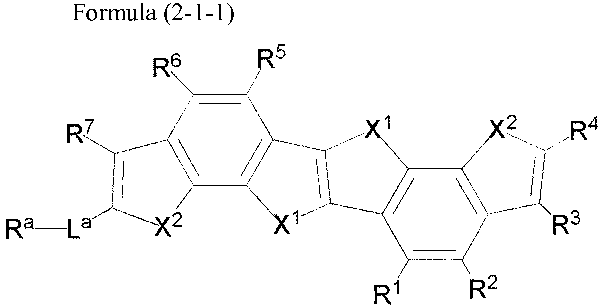 in Formula (2-1-1),
   each X¹, X², R¹ to R⁷ and R⁹ are as defined in claim 1, and when R¹, R², R³, R⁴, R⁵, R⁶, R⁷ or R⁹ is a substituent, the substituent is a substituent other than the substituents represented by the Formula (W);
   R⁴ is not a group represented by -L^{a}-R^{a},
   L^{a} and R^{a} have the same meaning as L and R as defined in claim 1; 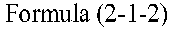 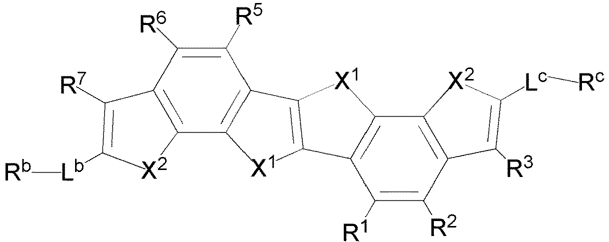
   in Formula (2-1-2),
   X¹ and X² and R¹ to R³, R⁵ to R⁷ and R⁹ have the same meaning as defined in claim 1, and wherein R1, R2, R3, R4, R5, R6, R7 or R9 is a substituent, the substituent is a substituent other than the substituents represented by Formula (W). each of L^{b} and L^{c}, R^{b} and R^{c} have the same meaning as L and R as defined in claim 1; 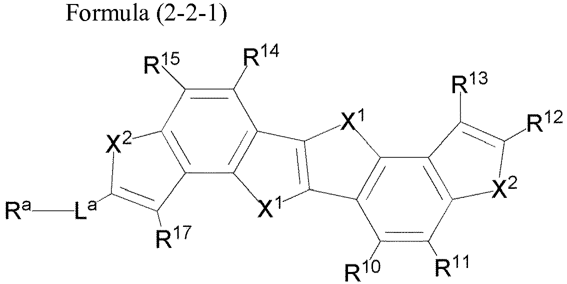 in Formula (2-2-1),
   each X¹ and X² have the same meaning as defined in claim 1,
   each of R¹⁰, R¹² to R¹⁵, and R¹⁷ independently represents a hydrogen atom or a substituent, R¹² is not a group represented by -L^{a}-R^{a},
   L^{a} and R^{a} have the same meaning as L and R as defined in claim 1; 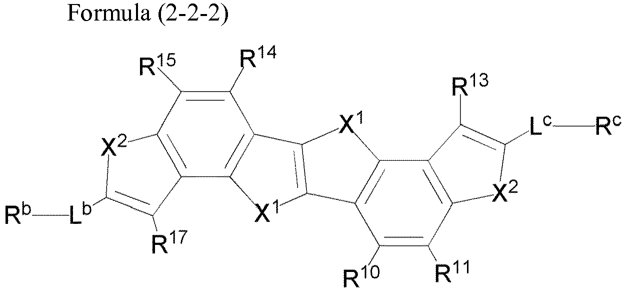
   in Formula (2-2-2),
   each X¹ and X² have the same meaning as defined in claim 1,
   each of R¹⁰, R¹¹, R¹³ to R¹⁵, and R¹⁷ independently represents a hydrogen atom or a substituent, R^{b} and R^{c} have the same meaning as L and R as defined in claim 1;
[3] The compound described in [1] or [2], in which in Formulae (1-1), (1-2), (1-1A), (2-1-1), (2-1-2), (2-2-1), and (2-2-2), each of L, L^{a}, L^{b}, and L^{c} is preferably independently a divalent linking group represented by any of Formulae (L-1) to (L-5), (L-13), (L-17), and (L-18) or a divalent linking group in which two or more divalent linking groups represented by any of Formulae (L-1) to (L-5), (L-13), (L-17), and (L-18) are bonded to each other.
[4] The compound described in any one of [1] to [3], in which in Formulae (1-1), (1-2), (1-1A), (2-1-1), (2-1-2), (2-2-1), and (2-2-2), each of L, L^{a}, L^{b}, and L^{c} is preferably independently a divalent linking group represented by any of Formulae (L-1), (L-3), (L-13), and (L-18) or a divalent linking group in which a divalent linking group represented by any one of Formulae (L-3), (L-13), and (L-18) is bonded to a divalent linking group represented by Formula (L-1).
[5] The compound described in any one of [1] to [4], in which in Formulae (1-1), (1-2), (1-1A), (2-1-1), (2-1-2), (2-2-1), and (2-2-2), each of R, R^{a}, R^{b}, and R^{c} is preferably independently a substituted or unsubstituted alkyl group.
[6] The compound described in any one of [1] to [5], in which in Formulae (1-1), (1-2), (1-1A), (2-1-1), (2-1-2), (2-2-1), and (2-2-2), each of R, R^{a}, R^{b}, and R^{c} is preferably independently a linear alkyl group.
[7] The compound described in any one of [1] to [6], in which in Formulae (1-1), (1-2), (1-1A), (2-1-1), (2-1-2), (2-2-1), and (2-2-2), both of X¹ and X² are preferably a sulfur atom.
[8] The compound according to any one of the item [1] to [4], wherein the substituents of each of R¹ to R³ in formula (1-1) and each of R¹⁰ to R¹⁷ in formula (1-2) are an alkyl group having 1 to 18 carbon atoms, an aryl group having 6 to 20 carbon atoms, an alkenyl group having 2 to 12 carbon atoms, an alkynyl group having 2 to 12 carbon atoms, an alkoxy group having 1 to 11 carbon atoms, a heterocyclic group having 5 to 12 carbon atoms, an alkyl thio group having 1 to 12 carbon atoms or the group of formula (W).
[9] An organic transistor or an organic semiconductor material for a non-light emitting organic semiconductor device or an organic semiconductor film for a non-light emitting organic semiconductor device, each comprising a compound represented by the Formula (1-1) or (1-2) as defined in any one of the items [1] to [5], wherein in the organic transistor said compound is comprised in a semiconductor active layer.
[10] A use for an organic transistor of a compound represented by the Formula (1-1) or (1-2) as defined in any one of the items [1] to [8].
[11] A coating solution for a non-light-emitting organic semiconductor device, comprising a compound represented by the Formula (1-1) or (1-2) as defined in any one of the items [1] to [8].

### BRIEF DESCRIPTION OF THE DRAWINGS

Fig. 1 is a schematic view showing a section of an exemplary structure of an organic transistor of the present invention.
Fig. 2 is a schematic view showing a section of a structure of the organic transistor manufactured as a substrate for measuring FET characteristics in examples of the present invention.
Fig. 3 is a ¹H-NMR chart of a compound g.
Fig. 4 is a ¹H-NMR chart of a compound 1.

### DESCRIPTION OF THE PREFERRED EMBODIMENTS

Hereinafter, the present invention will be specifically described. The constituents described below will be explained based on representative embodiments or specific examples in some cases, but the present invention is not limited to the embodiments. In the present specification, a range of numerical values described using "to" means a range including the numerical values listed before and after "to" as a lower limit and an upper limit respectively.

In the present invention, unless otherwise specified, a hydrogen atom used in the description of each formula represents a hydrogen atom including an isotope (deuterium atom or the like). Furthermore, an atom constituting a substituent represents an atom including an isotope thereof.

### [Organic transistor]

An organic transistor of the present invention contains a compound represented by the following Formula (1-1) or (1-2) in a semiconductor active layer. 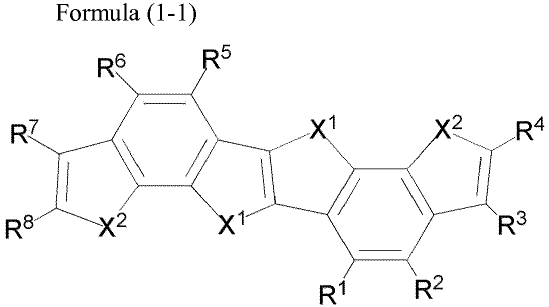 In Formula (1-1),
each X¹ independently represents a sulfur atom, an oxygen atom, a selenium atom, or NR⁹,
each X² independently represents a sulfur atom, an oxygen atom, or a selenium atom,
each of R¹ to R⁹ independently represents a hydrogen atom or a substituent, one or two of R¹, R², R³, R⁴, R⁵, R⁶, R⁷, R⁸, or R⁹ is a substituent represented by the following Formula (W); 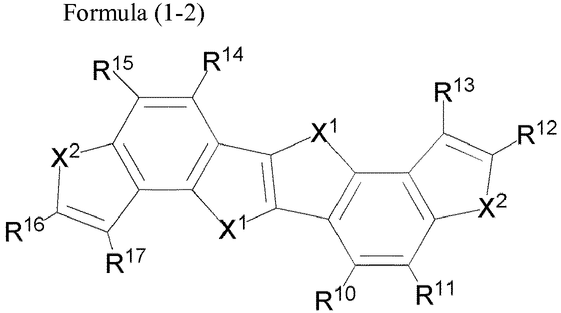
in Formula (1-2),
each X¹ independently represents a sulfur atom, an oxygen atom, a selenium atom, or NR⁹,
each X² independently represents a sulfur atom, an oxygen atom, or a selenium atom,
each of R⁹ to R¹⁷ independently represents a hydrogen atom or a substituent, and at least one of R⁹, R¹⁰, R¹¹, R¹², R¹³, R¹⁴, R¹⁵, R¹⁶, or R¹⁷ is a substituent represented by the following Formula (W);
-L-R Formula (W)
in Formula (W), L represents a divalent linking group represented by any of the following Formulae (L-1) to (L-25) or a divalent linking group in which two or more divalent linking groups represented by any of the following Formulae (L-1) to (L-25) are bonded to each other, and
R represents a substituted or unsubstituted alkyl group, a cyano group, a vinyl group, an ethynyl group, an oxyethylene group, an oligo-oxyethylene group in which a repetition number v of an oxyethylene unit is equal to or greater than 2, a siloxane group, an oligosiloxane group having two or more silicon atoms, or a substituted or unsubstituted trialkylsilyl group wherein the total number of carbon atoms contained in L and R is 5 to 12;

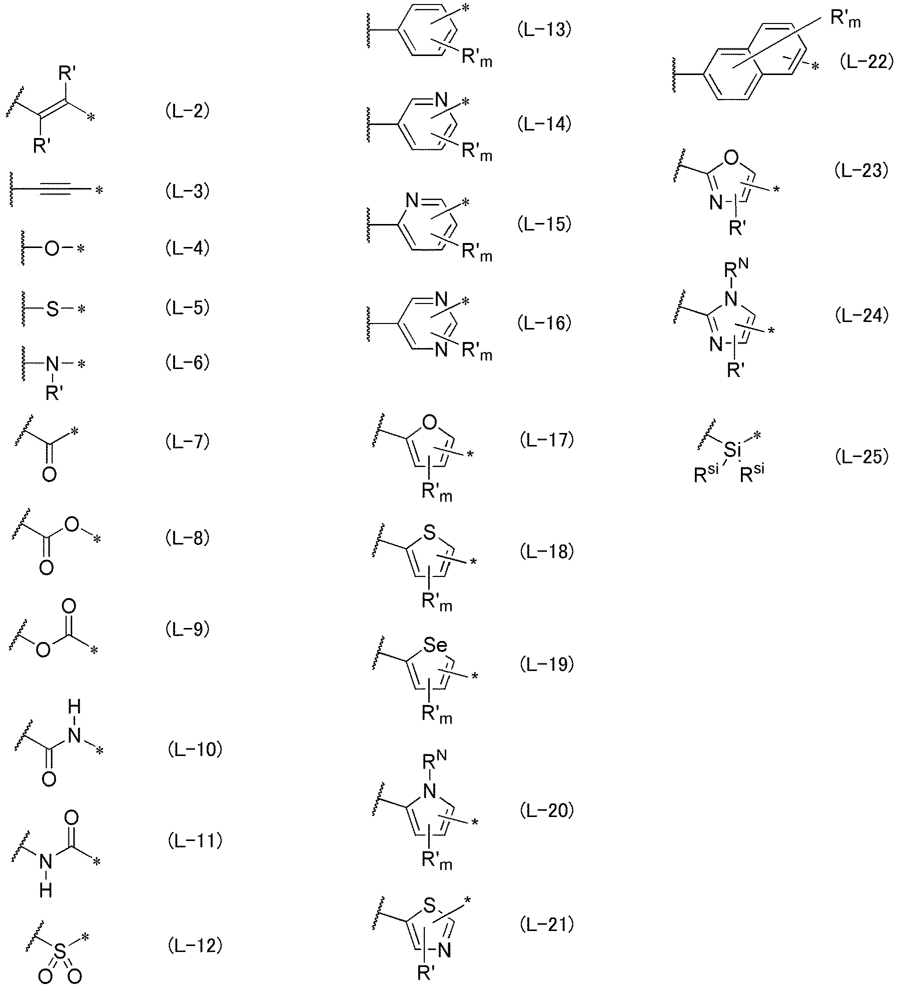

in Formulae (L-1) to (L-25),
the portion of a wavy line represents a position of bonding to a condensed cyclic skeleton,
m in Formula (L-13) represents 4, m in Formulae (L-14) and (L-15) represents 3, m in Formulae (L-16) to (L-20) represents 2, m in Formula (L-22) represents 6,
each R' in Formulae (L-1), (L-2), (L-6), and (L-13) to (L-24) independently represents a hydrogen atom or a substituent,
R^{N} represents a hydrogen atom or a substituent, and
each R^{si} independently represents a hydrogen atom, an alkyl group, an alkenyl group, or an alkynyl group.

The compound represented by Formula (1-1) and the compound represented by Formula (1-2) having the structure described above exhibit high solubility in an organic solvent. By containing the compound represented by Formula (1-1) or (1-2) in the semiconductor active layer, the organic transistor of the present invention has high carrier mobility.

Herein, WO2013/078407A describes an organic semiconductor compound having a polycyclic condensed structure whose structure seems to be similar to that of the compound of the present invention. However, due to the influence of the substituent on N, the intermolecular distance is increased in this compound, and as a result, overlapping of HOMO does not occur, and high carrier mobility is not obtained.

Furthermore, the compound described in US2008/0142792A1 differs from the compound of the present invention in terms of the position in which the heterocyclic ring is condensed on the terminal. Therefore, in the compound, the effect resulting from the interaction between heteroatoms is weak, overlapping of HOMO does not occur, and thus high carrier mobility is not obtained.

In contrast, each of the compounds of the present invention represented by Formulae (1-1) and (1-2) has a heterocyclic ring at the center and is condensed with two thiophene rings, furan rings, or selenophene rings in specific positions on the terminal. As a result, overlapping of HOMO occurs, and the crystallinity is improved. Therefore, by using the compound of the present invention in a semiconductor active layer, an organic transistor having high carrier mobility can be obtained.

In order to improve the solubility in a general organic solvent, it is effective to introduce a group represented by Formula (W) into the compound. It is preferable that the compound represented by Formula (1-1) or (1-2) exhibits high solubility in an organic solvent.

Furthermore, It is preferable that the organic transistor of the present invention using the compound represented by Formula (1-1) or (1-2) shows only a slight threshold voltage shift after repeated driving. In order to reduce the threshold voltage shift after repeated driving, HOMO of the organic semiconductor material needs not to be too shallow or too deep. Furthermore, the chemical stability (particularly, resistance against air oxidation and stability against oxidation and reduction) of the organic semiconductor material, the heat stability of the film state, the high film density which makes it difficult for air or moisture to permeate the film, the film quality by which the film has small defectiveness such that charge accumulation does not easily occur, and the like are required. It is considered that because the compound represented by the Formula (1-1) or (1-2) satisfies the aforementioned requirements, the organic transistor shows only a slight threshold voltage shift after repeated driving. That is, in the organic transistor showing only a slight threshold voltage shift after repeated driving, the semiconductor active layer has high chemical stability, high film density, and the like, and thus the organic transistor can effectively function as a transistor over a long period of time.

Being useful as an organic EL element material does not mean being useful as a semiconductor material for an organic transistor, because the characteristics required for the organic compound vary between the organic EL element and the organic transistor. In the organic EL element, charges need to be transported in the film thickness direction (generally, several nanometers to hundreds of nanometers) of a general film. In contrast, in the organic transistor, charges (carriers) need to be transported a long distance between electrodes (generally, several micrometers to hundreds of micrometers) in the film plane direction, and hence extremely high carrier mobility is required. Accordingly, as the semiconductor material for an organic transistor, an organic compound showing highly ordered molecular arrangement and having high crystallinity is required. Furthermore, for the expression of high carrier mobility, the π-conjugate plane thereof is preferably upright against a substrate. On the other hand, for the organic EL element, in order to improve the luminous efficiency, an element having high luminous efficiency and uniformly emitting light in plane is required. Generally, an organic compound having high crystallinity causes luminescence defectiveness such as nonuniform in-plane electric field intensity, nonuniform luminescence, and quenching of luminescence. Therefore, as the material for an organic EL element, those having low crystallinity but having high amorphousness are desirable. Consequently, the use of the organic compound constituting the organic EL element material as an organic semiconductor material does not ensure that excellent transistor characteristics can be obtained.

Hereinafter, preferred aspects of the compound and the organic transistor of the present invention will be described.

### <Compound represented by Formula (1-1) or (1-2)>

The organic transistor of the present invention contains the compound represented by Formula (1-1) or (1-2) in a semiconductor active layer which will be described later. Among compounds represented by Formula (1-1), a compound represented by Formula (1-1A) which will be described later and a compound represented by Formula (1-2) are novel compounds. The compound represented by Formula (1-1A) and the compound represented by Formula (1-2) are collectively called the compound of the present invention. The compound represented by Formula (1-1) or (1-2), particularly, the compound of the present invention is contained in a semiconductor active layer, which will be described later, in the organic transistor of the present invention. That is, the compound represented by Formula (1-1) or (1-2), particularly, the compound of the present invention can be used as a material for an organic transistor. Among the compound represented by Formula (1-1) and the compound represented by Formula (1-2), the compound represented by Formula (1-1) can be preferably used in a semiconductor active layer of the organic transistor.

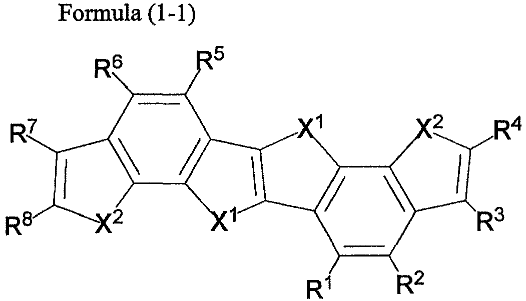

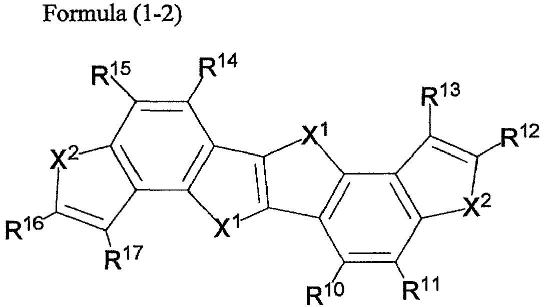

In Formulae (1-1) and (1-2), each X¹ independently represents a sulfur atom, an oxygen atom, a selenium atom, or NR⁹, and each X² independently represents a sulfur atom, an oxygen atom, or a selenium atom. From the viewpoint of the ease of synthesis, it is preferable that each of X¹ and X² is independently an oxygen atom or a sulfur atom. In contrast, from the viewpoint of improving the carrier mobility, it is preferable that at least one of X¹ or X² is a sulfur atom. X¹ and X² are preferably the same linking group. Both of X¹ and X² are more preferably a sulfur atom.

R⁹ represents a hydrogen atom, an alkyl group, an alkenyl group, an alkynyl group, an acyl group, or a group represented by the following Formula (W) which will be described later. R⁹ is preferably a hydrogen atom or an alkyl group, more preferably an alkyl group having 5 to 12 carbon atoms, and particularly preferably an alkyl group having 8 to 10 carbon atoms.

In a case where R⁹ represents an alkyl group, the alkyl group may be a linear, branched, or cyclic alkyl group. However, from the viewpoint of the overlapping of HOMO, R⁹ is preferably a linear alkyl group.

In Formula (1-1), each of R¹ to R⁹ independently represents a hydrogen atom or a substituent, and at least one of R¹, R², R³, R⁴, R⁵, R⁶, R⁷, R⁸, or R⁹ is a substituent represented by the following Formula (W).

Furthermore, in Formula (1-2), each of R⁹ to R¹⁷ represents a hydrogen atom or a substituent, and at least one of R⁹, R¹⁰, R¹¹, R¹², R¹³, R¹⁴, R¹⁵, R¹⁶, or R¹⁷ is a substituent represented by the following Formula (W).

-L-R Formula (W)

In Formula (W), L represents a divalent linking group represented by any of the following Formulae (L-1) to (L-25) or a divalent linking group in which two or more divalent linking groups represented by any of the following Formulae (L-1) to (L-25) are bonded to each other, and
R represents a substituted or unsubstituted alkyl group, a cyano group, a vinyl group, an ethynyl group, an oxyethylene group, an oligo-oxyethylene group in which a repetition number v of an oxyethylene unit is equal to or greater than 2, a siloxane group, an oligosiloxane group having two or more silicon atoms, or a substituted or unsubstituted trialkylsilyl group:

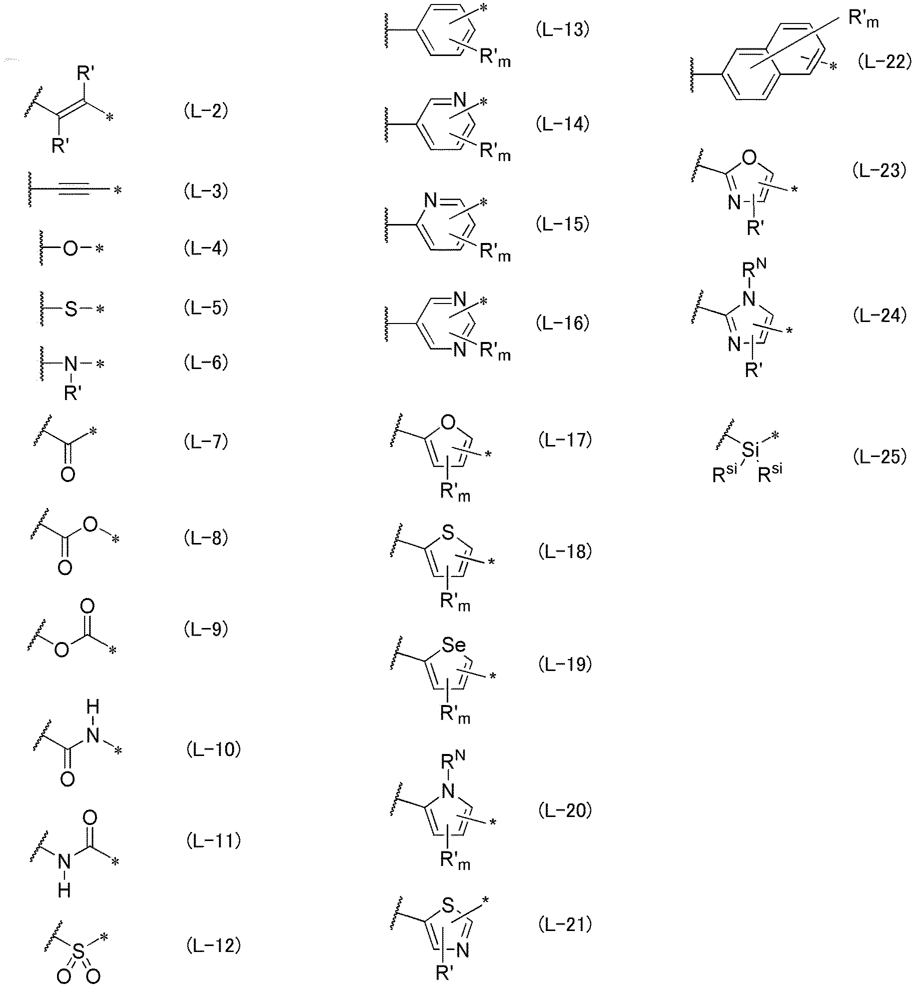

in Formulae (L-1) to (L-25), the portion of a wavy line represents a position of bonding to a condensed cyclic skeleton,
m in Formula (L-13) represents 4, m in Formulae (L-14) and (L-15) represents 3, m in Formulae (L-16) to (L-20) represents 2, m in Formula (L-22) represents 6,
each R' in Formulae (L-1), (L-2), (L-6), and (L-13) to (L-24) independently represents a hydrogen atom or a substituent,
R^{N} represents a hydrogen atom or a substituent, and
each R^{si} independently represents a hydrogen atom, an alkyl group, an alkenyl group, or an alkynyl group.

The substituent that each of R¹ to R⁹ in Formula (1-1) and each of R⁹ to R¹⁷ in Formula (1-2) can independently represent are a halogen atom, an alkyl group (including an alkyl group having 1 to 40 carbon atoms such as a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, or a pentadecyl group; here, the alkyl group also includes a 2,6-dimethyloctyl group, a 2-decyltetradecyl group, a 2-hexyldodecyl group, a 2-ethyloctyl group, a 2-decyltetradecyl group, a 2-butyldecyl group, a 1-octylnonyl group, a 2-ethyloctyl group, a 2-octyltetradecyl group, a 2-ethylhexyl group, a cycloalkyl group, a bicycloalkyl group, a tricycloalkyl group, and the like), an alkenyl group (including a 1-pentenyl group, a cycloalkenyl group, a bicycloalkenyl group, and the like), an alkynyl group (including a 1-pentynyl group, a trimethylsilylethynyl group, a triethylsilylethynyl group, a tri-i-propylsilylethynyl group, a 2-p-propylphenylethynyl group, and the like), an aryl group (including an aryl group having 6 to 20 carbon atoms such as a phenyl group, a naphthyl group, a p-pentylphenyl group, a 3,4-dipentylphenyl group, a p-heptoxyphenyl group, a 3,4-diheptoxyphenyl group, and the like), a hetero ring group (may be referred to as a heterocyclic group as well, including a 2-hexylfuranyl group and the like), a cyano group, a hydroxyl group, a nitro group, an acyl group (including a hexanoyl group, a benzoyl group, and the like), an alkoxy group (including a butoxy group and the like), an aryloxy group, a silyloxy group, a heterocyclic oxy group, an acyloxy group, a carbamoyloxy group, an amino group (including an anilino group), an acylamino group, an aminocarbonylamino group (including a ureide group), alkoxy- and aryloxycarbonylamino groups, alkyl- and aryl sulfonylamino groups, a mercapto group, alkyl- and arylthio groups (including a methylthio group, an octylthio group, and the like), a heterocyclic thio group, a sulfamoyl group, a sulfo group, alkyl- and aryl sulfinyl groups, alkyl- and aryl sulfonyl groups, alkyloxy- and aryloxycarbonyl groups, a carbamoyl group, aryl- and heterocyclic azo group, an imide group, a phosphino group, a phosphinyl group, a phosphinyloxy group, a phosphinylamino group, a phosphono group, a silyl group (a ditrimethylsiloxy methylbutoxy group), a hydrazino group, a ureide group, a boronic acid group (-B(OH)₂), a phosphate group (-OPO(OH)₂), a sulfate group (-OSO₃H), and other known substituents.

These substituents may further have the above substituents. In addition, in a case where the compound represented by Formula (1-1) or (1-2) is a polymer compound having a repeating structure, each of R¹ to R⁹ in Formula (1-1) and each of R⁹ to R¹⁷ in Formula (1-2) may have a group derived from a polymerizable group.

Among these, as the substituent that each of R¹ to R⁹ in Formula (1-1) and each of R⁹ to R¹⁷ in Formula (1-2) can independently represent, an alkyl group, an aryl group, an alkenyl group, an alkynyl group, a heterocyclic group, an alkoxy group, an alkylthio group, and a group represented by Formula (W) which will be described later are preferable; an alkyl group having 1 to 18 carbon atoms, an aryl group having 6 to 20 carbon atoms, an alkenyl group having 2 to 12 carbon atoms, an alkynyl group having 2 to 12 carbon atoms, an alkoxy group having 1 to 11 carbon atoms, a heterocyclic group having 5 to 12 carbon atoms, an alkylthio group having 1 to 12 carbon atoms, and a group represented by Formula (W) which will be described later are more preferable; a group having a linking group chain length, which will be described later, of equal to or less than 3.7 Å and a group represented by Formula (W) which will be described later are particularly preferable; and a group represented by Formula (W) which will be described later is more particularly preferable.

The group represented by Formula (W) will be described.

-L-R Formula (W)

In Formula (W), L represents a divalent linking group represented by any of the following Formulae (L-1) to (L-25) or a divalent linking group in which two or more divalent linking groups represented by any of the following Formulae (L-1) to (L-25), and
R represents a substituted or unsubstituted alkyl group, a cyano group, a vinyl group, an ethynyl group, an oxyethylene group, an oligo-oxyethylene group in which a repetition number v of an oxyethylene unit is equal to or greater than 2, a siloxane group, an oligosiloxane group having two or more silicon atoms, or a substituted or unsubstituted trialkylsilyl group:

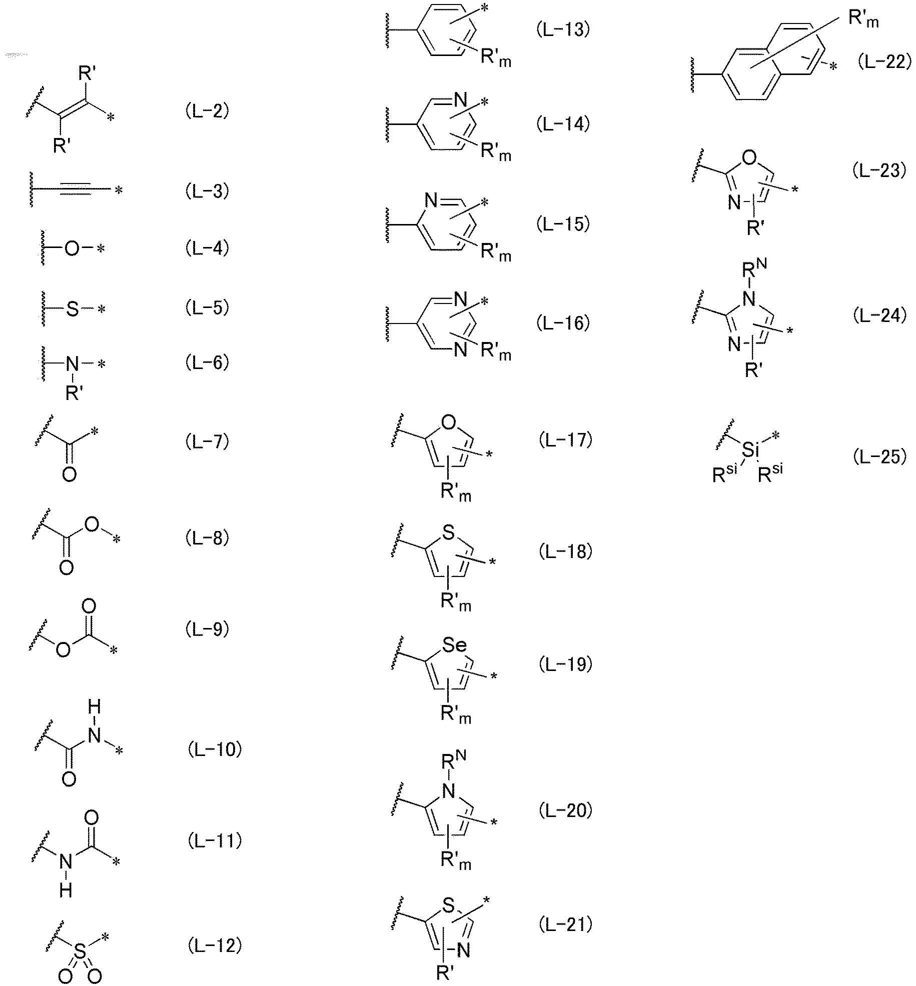

in Formulae (L-2) to (L-25), the portion of a wavy line represents a position of bonding to a condensed cyclic skeleton,
m in Formula (L-13) represents 4, m in Formulae (L-14) and (L-15) represents 3, m in Formulae (L-16) to (L-20) represents 2, m in Formula (L-22) represents 6,
each R' in Formulae (L-2), (L-6), and (L-13) to (L-24) independently represents a hydrogen atom or a substituent,
R^{N} represents a hydrogen atom or a substituent, and
each R^{si} independently represents a hydrogen atom, an alkyl group, an alkenyl group, or an alkynyl group.

In Formula (W), L represents a divalent linking group represented by any of the following Formulae (L-1) to (L-25) or a divalent linking group in which two or more divalent linking groups represented by any of the following Formulae (L-1) to (L-25) are bonded to each other.

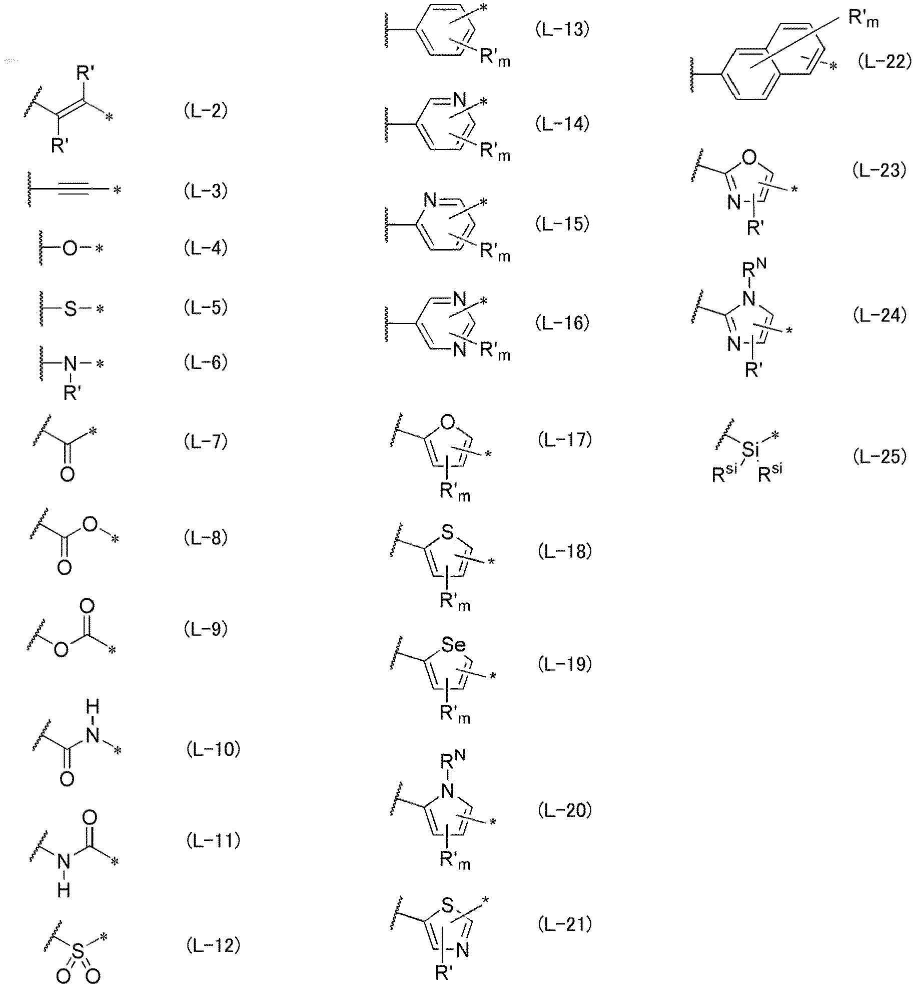

In Formulae (L-2) to (L-25), the portion of a wavy line represents a position of bonding to a condensed cyclic skeleton. Here, in a case where L represents a divalent linking group in which two or more divalent linking groups represented by any of Formulae (L-1) to (L-25) are bonded to each other, the portion of a wavy line represents a position of bonding to * of the divalent linking group represented by any of Formulae (L-1) to (L-25).
* represents a position of bonding to either the divalent linking group represented by any of Formulae (L-2) to (L-25) or R.
m in Formula (L-13) represents 4, m in Formulae (L-14) and (L-15) represents 3, m in Formulae (L-16) to (L-20) represents 2, and m in Formula (L-22) represents 6.
Each R' in Formulae (L-1), (L-2), (L-6), and (L-13) to (L-24) independently represents a hydrogen atom or a substituent.
R^{N} represents a hydrogen atom or a substituent.
Each R^{si} independently represents a hydrogen atom, an alkyl group, an alkenyl group, or an alkynyl group.
Each R' in Formulae (L-1) and (L-2) may form a condensed ring by being bonded to R adjacent to L.

The divalent linking group represented by any of Formulae (L-19) to (L-21), (L-23), and (L-24) is more preferably a divalent linking group represented by any of the following Formulae (L-19A) to (L-21A), (L-23A), and (L-24A).

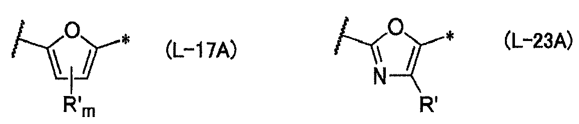

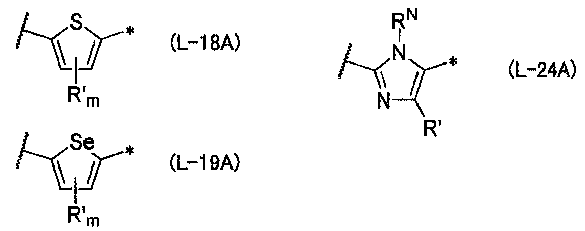

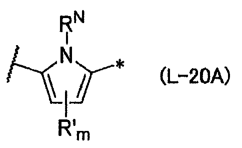

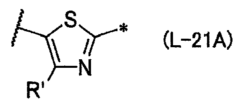

In a case where a substituted or unsubstituted alkyl group, an oxyethylene group, an oligo-oxyethylene group in which a repetition number v of an oxyethylene unit is equal to or greater than 2, a siloxane group, an oligosiloxane group having two or more silicon atoms, or a substituted or unsubstituted trialkylsilyl group is present on the terminal of the substituent represented by Formula (W), the substituent can be interpreted as either a substituent consisting of only -R in Formula (W) or a substituent consisting of -R-L in Formula (W).

In the present invention, in a case where a substituted or unsubstituted alkyl group whose main chain consists of N carbon atoms is present on the terminal of the substituent represented by Formula (W), the substituent is interpreted as -L-R in Formula (W) by including as much linking groups represented by -CR^{R}₂- as possible in R from the terminal of the substituent represented by Formula (W), and is not interpreted as a substituent composed solely of -R. R^{R} represents a hydrogen atom or a substituent. Specifically, the substituent is interpreted as a substituent in which "one (L-1) corresponding to L in Formula (W)" is bonded to "a substituted or unsubstituted alkyl group corresponding to R in Formula (W) having a main chain consisting of (N - 1) carbon atoms". For example, in a case where a n-octyl group as an alkyl group having 8 carbon atoms is present on the terminal of the substituent, the substituent is interpreted as a substituent in which one (L-1), wherein two R's are hydrogen atoms, is boned to a n-heptyl group having 7 carbon atoms. In a case where the substituent represented by Formula (W) is an alkyl group, for example, how to make a differentiation between L and R is as follows, and L represents a divalent linking group represented by Formula (L-1).

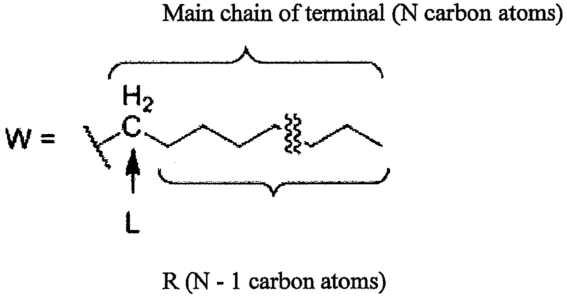

In a case where the substituent represented by Formula (W) is an alkoxy group having 8 carbon atoms, the alkoxy group is interpreted as a substituent in which one linking group represented by Formula (L-4) that is -O-, one linking group represented by Formula (L-1) in which two R's are hydrogen atoms, and a n-heptyl group having 7 carbon atoms are bonded to each other. In a case where the substituent represented by Formula (W) is a group other than an alkyl group, how to make a differentiation between L and R is as follows, and L represents a divalent linking group in which "a divalent linking group other than the linking group represented by Formula (L-1)" and "a divalent linking group represented by Formula (L-1)" are bonded to each other.

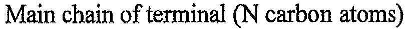

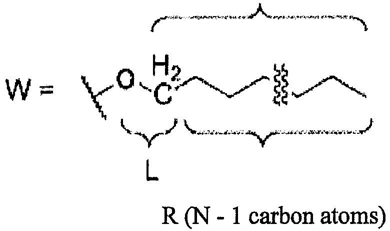

In contrast, in the present invention, in a case where an oxyethylene group, an oligo-oxyethylene group in which a repetition number v of an oxyethylene unit is equal to or greater than 2, a siloxane group, an oligosiloxane group having two or more silicon atoms, or a substituted or unsubstituted trialkylsilyl group is present on the terminal of the substituent represented by Formula (W), the substituent is interpreted as a substituent consisting solely of R in Formula (W) including as much linking groups as possible in R from the terminal of the substituent represented by Formula (W) but is not interpreted as -L-R. For example, in a case where a -(OCH₂CH₂)-(OCH₂CH₂)-(OCH₂CH₂)-OCH₃ group is present on the terminal of the substituent, the substituent is interpreted as a substituent consisting of only an oligo-oxyethylene group in which the repetition number v of an oxyethylene unit is 2. In a case where an oxyethylene group, an oligo-oxyethylene group in which a repetition number v of an oxyethylene unit is equal to or greater than 2, a siloxane group, an oligosiloxane group having two or more silicon atoms, or a substituted or unsubstituted trialkylsilyl group is present on the terminal of the substituent represented by Formula (W), how to make a differentiation between L and R is as follows, for example.

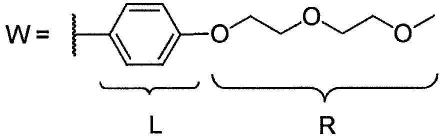

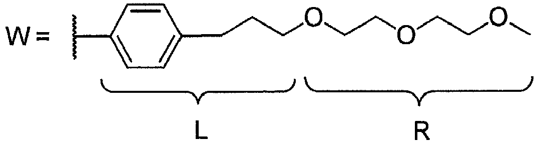

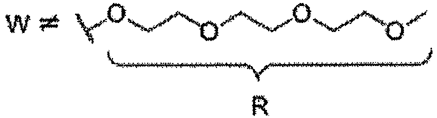

In a case where L forms a linking group in which divalent linking groups represented by any of Formulae (L-1) to (L-25) are bonded to each other, the number of the divalent linking groups bonded to each other represented by any of Formulae (L-1) to (L-25) is preferably 2 to 4, and more preferably 2 or 3.

Examples of the substituent R' in Formulae (L-1), (L-2), (L-6), and (L-13) to (L-24) include those exemplified above as the substituents that can be adopted as R¹ to R⁸ in Formula (1-1) or (1-2) described above. Among these, the substituent R' in Formula (L-6) is preferably an alkyl group. In a case where R' in (L-6) is an alkyl group, the number of carbon atoms of the alkyl group is preferably 1 to 18, more preferably 5 to 12 from the viewpoint of the chemical stability and the carrier transport properties, and even more preferably 8 to 10. In a case where R' in (L-6) is an alkyl group, the alkyl group is preferably a linear alkyl group because then the carrier mobility can be improved.

R^{N} represents a hydrogen atom or a substituent, and examples of R^{N} include those exemplified above as substituents that can be adopted as R¹ to R⁸ in Formula (1-1) or (1-2) described above. Among these, a hydrogen atom or a methyl group is preferable as R^{N}.

Each R^{si} independently represents a hydrogen atom, an alkyl group, an alkenyl group, or an alkynyl group, and is preferably an alkyl group. The alkyl group that can be adopted as R^{si} is not particularly limited, and the preferred range of the alkyl group that can be adopted as R^{si} is the same as the preferred range of an alkyl group that can be adopted as a silyl group in a case where R is a silyl group. The alkenyl group that can be adopted as R^{si} is not particularly limited. However, the alkenyl group is preferably a substituted or unsubstituted alkenyl group and more preferably a branched alkenyl group, and the number of carbon atoms of the alkenyl group is preferably 2 or 3. The alkynyl group that can be adopted as R^{si} is not particularly limited. However, the alkynyl group is preferably a substituted or unsubstituted alkynyl group and more preferably a branched alkynyl group, and the number of carbon atoms of the alkynyl group is preferably 2 or 3.

In a case where L includes divalent linking groups represented by Formulae (L-13) to (L-25), it is preferable that any one of the divalent linking groups represented by Formulae (L-13) to (L-25) is linked to R through a divalent linking group represented by Formula (L-1).

L is preferably a divalent linking group represented by any of Formulae (L-1) to (L-5), (L-13), (L-17), and (L-18) or a divalent linking group in which two or more divalent linking groups represented by any of Formulae (L-1) to (L-5), (L-13), (L-17), and (L-18) are bonded to each other, more preferably a divalent linking group represented by any of Formulae (L-1), (L-3), (L-13), and (L-18) or a divalent linking group in which two or more divalent linking groups represented by any of Formulae (L-1), (L-3), (L-13), and (L-18) are bonded to each other, and particularly preferably a divalent linking group represented by (L-1), (L-3), (L-13), or (L-18) or a divalent linking group in which a divalent linking group represented by one of Formulae (L-3), (L-13), and (L-18) is bonded to a divalent linking group represented by Formula (L-1). It is preferable that the divalent linking group represented by Formula (L-1) is bonded to the R side of a divalent linking group in which the divalent linking group represented by any one of Formulae (L-3), (L-13), and (L-18) is bonded to the divalent linking group represented by Formula (L-1).

From the viewpoint of the chemical stability and the carrier transport properties, L is particularly preferably a divalent linking group including the divalent linking group represented by Formula (L-1), more particularly preferably a divalent linking group represented by Formula (L-1). Even more particularly preferably, the substituent represented by Formula (W) is a group in which L is a divalent linking group represented by Formula (L-1), and R is a substituted or unsubstituted alkyl group.

In Formula (W), R represents a substituted or unsubstituted alkyl group, a cyano group, a vinyl group, an ethynyl group, an oxyethylene group, an oligo-oxyethylene group in which a repetition number v of an oxyethylene unit is equal to or greater than 2, a siloxane group, an oligosiloxane group having two or more silicon atoms, or a substituted or unsubstituted silyl group.

In Formula (W), in a case where L adjacent to R is a divalent linking group represented by Formula (L-1), R is preferably a substituted or unsubstituted alkyl group, an oxyethylene group, an oligo-oxyethylene group in which a repetition number v of an oxyethylene unit is equal to or greater than 2, a siloxane group, or an oligosiloxane group having two or more silicon atoms, and more preferably a substituted or unsubstituted alkyl group.

In Formula (W), in a case where L adjacent to R is a divalent linking group represented by any of Formulae (L-2) and (L-4) to (L-25), R is more preferably a substituted or unsubstituted alkyl group.

In Formula (W), in a case where L adjacent to R is a divalent linking group represented by Formula (L-3), R is preferably a substituted or unsubstituted alkyl group or a substituted or unsubstituted silyl group.

In a case where R is a substituted or unsubstituted alkyl group, the number of carbon atoms thereof is preferably 1 to 12, more preferably 5 to 12 from the viewpoint of the chemical stability and the carrier transport properties, and even more preferably 8 to 10. If R is a long-chain alkyl group within the above range, the alkyl group is particularly preferably a long-chain linear alkyl group because then the linearity of the molecule is improved, and hence the carrier mobility can be improved.

In a case where R represents an alkyl group, the alkyl group may be a linear, branched, or cyclic alkyl group. However, from the viewpoint of the overlapping of HOMO, the alkyl group is preferably a linear alkyl group.

Among these, from the viewpoint of improving the carrier mobility, R and L in Formula (W) are preferably combined such that L is a divalent linking group represented by Formula (L-1), and R is a linear alkyl group having 1 to 12 carbon atoms; or that L is a divalent linking group in which a divalent linking group represented by any one of Formulae (L-3), (L-13), and (L-18) is bonded to a divalent linking group represented by Formula (L-1), and R is a linear alkyl group.

In a case where L is a divalent linking group represented by Formula (L-1), and R is a linear alkyl group having 1 to 12 carbon atoms, from the view point of improving the carrier mobility, R is more preferably a linear alkyl group having 5 to 12 carbon atoms, and particularly preferably a linear alkyl group having 8 to 10 carbon atoms.

In a case where L is a divalent linking group in which a divalent linking group represented by any one of Formulae (L-3), (L-13), and (L-18) is bonded to a divalent linking group represented by Formula (L-1), and R is a linear alkyl group, R is a linear alkyl group having 1 to 12 carbon atoms, preferably a linear alkyl group having 5 to 12 carbon atoms from the viewpoint of the chemical stability and the carrier transport properties, and particularly preferably a linear alkyl group having 8 to 10 carbon atoms from the viewpoint of improving the carrier mobility.

In contrast, from the viewpoint of improving the solubility in an organic solvent, R is preferably a branched alkyl group.

In a case where R is an alkyl group having a substituent, examples of the substituent include a halogen atom and the like (at this time, R becomes a haloalkyl group), and among these, a fluorine atom is preferable. In a case where R is an alkyl group having a fluorine atom, a perfluoroalkyl group may be formed by the substitution of all of the hydrogen atoms of the alkyl group with the fluorine atom. Here, R is preferably a unsubstituted alkyl group.

In the present specification, in a case where the R is an ethyleneoxy group or an oligo-ethyleneoxy group in which a repetition number v of an oxyethylene group is equal to or greater than 2, the "oligo-oxyethylene group" represented by R refers to a group represented by -(OCH₂CH₂)ᵥOY (the repetition number v of an oxyethylene unit represents an integer of equal to or greater than 2, and Y on the terminal represents a hydrogen atom or a substituent). In a case where Y on the terminal of the oligo-oxyethylene group is a hydrogen atom, the terminal becomes a hydroxy group. The repetition number v of the oxyethylene unit is preferably 2 to 4, and more preferably 2 or 3. It is preferable that the hydroxy group on the terminal of the oligo-oxyethylene group is sealed. That is, it is preferable that Y represents a substituent. In this case, the hydroxy group is preferably sealed with an alkyl group having 1 to 3 carbon atoms. In other words, Y is preferably an alkyl group having 1 to 3 carbon atoms, more preferably a methyl group or an ethyl group, and particularly preferably a methyl group.

In a case where R is a siloxane group or an oligosiloxane group having two or more silicon atoms, the repetition number of the siloxane unit is preferably 2 to 4, and more preferably 2 or 3. Furthermore, it is preferable that hydrogen atoms or alkyl groups are boned to the Si atoms. In a case where alkyl groups are boned to the Si atoms, the number of carbon atoms of each alkyl group is preferably 1 to 3. For example, it is preferable that methyl groups or ethyl groups are bonded to the Si atoms. The same alkyl groups may be bonded to the Si atoms, or different alkyl groups or hydrogen atoms may be bonded to the Si atoms. In addition, all of the siloxane units constituting the oligosiloxane group may be the same as or different from each other, but it is preferable that they are the same as each other.

In a case where L adjacent to R is a divalent linking group represented by Formula (L-3), R is preferably a substituted or unsubstituted silyl group. In a case where R is a substituted or unsubstituted silyl group, a substituted silyl group is preferable as R. The substituent of the silyl group is not particularly limited. However, the substituent is preferably a substituted or unsubstituted alkyl group, and more preferably a branched alkyl group. In a case where R is a trialkylsilyl group, the number of carbon atoms of each alkyl group bonded to the Si atoms is preferably 1 to 3. For example, it is preferable that methyl groups, ethyl groups, or isopropyl groups are bonded to the Si atoms. The same alkyl groups or different alkyl groups may be bonded to the Si atoms. In a case where R is a trialkylsilyl group further having a substituent on the alkyl group, the substituent is not particularly limited.

In Formula (W), the total number of carbon atoms contained in L and R is 5 to 12. If the total number of carbon atoms contained in L and R is equal to or greater than the lower limit of the above range, the carrier mobility is improved, and the driving voltage is reduced. If the total number of carbon atoms contained in L and R is equal to or less than the upper limit of the above range, the solubility in an organic solvent is improved.

The total number of carbon atoms contained in L and R is preferably 6 to 12, and more particularly preferably 8 to 12.

In the compound represented by the Formula (1-1), the number of groups adopted as one of R¹ to R⁹ and represented by Formula (W) is 1 or 2, and preferably 2.

The group represented by the Formula (W) is positioned in any of R¹ to R⁹ without particular limitation. However, from the viewpoint of improving the carrier mobility and the solubility in an organic solvent, the group is at least positioned in R⁴ or R⁸, and preferably positioned in R⁴ and R⁸.

In the compound represented by the Formula (1-2), the number of groups adopted as R⁹ to R¹⁷ and represented by Formula (W) is preferably 1 to 4 from the viewpoint of improving the carrier mobility and the solubility in an organic solvent, more preferably 1 or 2, and particularly preferably 2.

The group represented by Formula (W) is positioned in any of R⁹ to R¹⁷ without particular limitation. However, from the viewpoint of improving the carrier mobility and the solubility in an organic solvent, the group is preferably positioned in R¹² or R¹⁶, and more preferably positioned in R¹² and R¹⁶.

The number of substituents that are adopted as R¹ to R⁹ in Formula (1-1) but other than the group represented by the Formula (W) is preferably 0 to 4, more preferably 0 to 2, particularly preferably 0 or 1, and more particularly preferably 0.

The number of substituents that are adopted as R⁹ to R¹⁷ in Formula (1-2) but other than the group represented by the Formula (W) is preferably 0 to 4, more preferably 0 to 2, particularly preferably 0 or 1, and more particularly preferably 0.

In a case where each of R¹ to R¹⁷ is a substituent other than the substituent represented by Formula (W), the substituent as each of R¹ to R¹⁷ is preferably a group having a linking group chain length of equal to or less than 3.7 Å, more preferably a group having a linking group chain length of 1.0 Å to 3.7 Å, and even more preferably a group having a linking group chain length of 1.0 Å to 2.1 Å.

The linking group chain length refers to a length from a C atom to the terminal of the substituent R⁰ in a C-R⁰ bond. Structural optimization calculation can be performed using a density functional method (Gaussian 03 (Gaussian Inc.)/basis function: 6-31G*, exchange-correlation functional: B3LYP/LANL2DZ). The molecular lengths of typical substituents are 4.6 Å for a propyl group, 4.6 Å for a pyrrole group, 4.5 Å for a propynyl group, 4.6 Å for a propenyl group, 4.5 Å for an ethoxy group, 3.7 Å for a methylthio group, 3.4 Å for an ethenyl group, 3.5 Å for an ethyl group, 3.6 Å for an ethynyl group, 3.3 Å for a methoxy group, 2.1 Å for a methyl group, and 1.0 Å for a hydrogen atom.

In a case where each of R¹ to R¹⁷ is a substituent other than the substituent represented by Formula (W), each of the substituents as R¹ to R¹⁷ is preferably independently a substituted or unsubstituted alkyl group having 2 or less carbon atoms, a substituted or unsubstituted alkynyl group having 2 or less carbon atoms, a substituted or unsubstituted alkenyl group having 2 or less carbon atoms, or a substituted or unsubstituted acyl group having two or less carbon atoms, and more preferably independently a substituted or unsubstituted alkyl group having 2 or less carbon atoms.

In a case where each of R¹ to R¹⁷ is a substituent other than the substituent represented by Formula (W), and each of the substituents as R¹ to R¹⁷ independently represents a substituted alkyl group having 2 or less carbon atoms, examples of the substituent that the alkyl group can have include a cyano group, a fluorine atom, a deuterium atom, and the like, and among these, a cyano group is preferable. In a case where each of R¹ to R¹⁷ is a substituent other than the substituent represented by Formula (W), the substituted or unsubstituted alkyl group having 2 or less carbon atoms that is represented by the substituent as each of R¹ to R¹⁷ is preferably a methyl group, an ethyl group, or a methyl group substituted with a cyano group, more preferably a methyl group or a methyl group substituted with a cyano group, and particularly preferably a methyl group substituted with a cyano group.

In a case where each of R¹ to R¹⁷ is a substituent other than the substituent represented by Formula (W), and each of the substituents as R¹ to R¹⁷ independently represents a substituted alkynyl group having 2 or less carbon atoms, examples of the substituent that the alkynyl group can have include a deuterium atom and the like. In a case where each of R¹ to R¹⁷ is a substituent other than the substituent represented by Formula (W), examples of the substituted or unsubstituted alkynyl group having 2 or less carbon atoms that is represented by the substituent as each of R¹ to R¹⁷ include an ethynyl group and an acetylene group substituted with a deuterium atom, and among these, an ethynyl group is preferable.

In a case where each of R¹ to R¹⁷ is a substituent other than the substituent represented by Formula (W), and each of the substituents as R¹ to R¹⁷ independently represents a substituted alkenyl group having 2 or less carbon atoms, examples of the substituent that the alkenyl group can have include a deuterium atom and the like. In a case where each of R¹ to R¹⁷ is a substituent other than the substituent represented by Formula (W), examples of a substituted or unsubstituted alkenyl group having 2 or less carbon atoms that is represented by the substituent as each of R¹ to R¹⁷ include an ethenyl group and an ethenyl group substituted with a deuterium atom, and among these, an ethenyl group is preferable.

In a case where each of R¹ to R¹⁷ is a substituent other than the substituent represented by Formula (W), and each of the substituents as R¹ to R¹⁷ independently represents a substituted acyl group having 2 or less carbon atoms, examples of the substituent that the acyl group can have include a fluorine atom and the like. In a case where each of R¹ to R¹⁷ is a substituent other than the substituent represented by Formula (W), examples of a substituted or unsubstituted acyl group having 2 or less carbon atoms that is represented by the substituent as each of R¹ to R¹⁷ include a formyl group, an acetyl group, and an acetyl group substituted with fluorine, and among these, a formyl group is preferable.

The compound represented by Formula (1-1) is preferably a compound represented by the following Formula (2-1-1) or (2-1-2), and is particularly preferably a compound represented by Formula (2-1-2) from the viewpoint of high mobility. 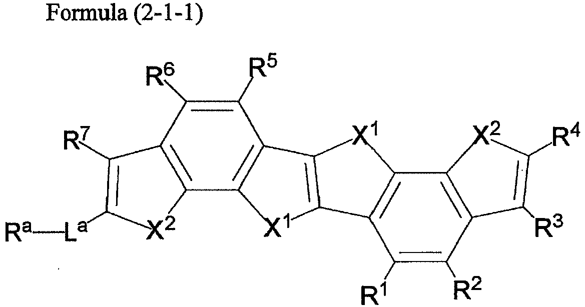 In Formula (2-1-1),
each X¹ independently represents a sulfur atom, an oxygen atom, a selenium atom, or NR⁹,
each X² independently represents a sulfur atom, an oxygen atom, or a selenium atom,
each of R¹ to R⁷ and R⁹ independently represents a hydrogen atom or a substituent, R⁴ is not a group represented by -L^{a}-R^{a},
L^{a} represents a divalent linking group represented by any of the following Formulae (L-1) to (L-25) or a divalent linking group in which two or more divalent linking groups represented by any of the following Formulae (L-1) to (L-25) are bonded to each other, and
R^{a} represents a substituted or unsubstituted alkyl group, a cyano group, a vinyl group, an ethynyl group, an oxyethylene group, an oligo-oxyethylene group in which a repetition number v of an oxyethylene unit is equal to or greater than 2, a siloxane group, an oilgosiloxane group having two or more silicon atoms, or a substituted or unsubstituted trialkylsilyl group; 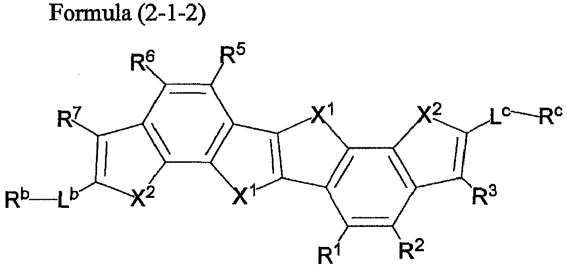
in Formula (2-1-2),
each X¹ independently represents a sulfur atom, an oxygen atom, a selenium atom, or NR⁹,
each X² independently represents a sulfur atom, an oxygen atom, or a selenium atom,
each of R¹ to R³, R⁵ to R⁷, and R⁹ independently represents a hydrogen atom or a substituent,
each of L^{b} and L^{c} independently represents a divalent linking group represented by any of the following Formulae (L-1) to (L-25) or a divalent linking group in which two or more divalent linking groups represented by any of the following Formulae (L-1) to (L-25) are bonded to each other, and
each of R^{b} and R^{c} independently represents a substituted or unsubstituted alkyl group, a cyano group, a vinyl group, an ethynyl group, an oxyethylene group, an oligo-oxyethylene group in which a repetition number v of an oxyethylene unit is equal to or greater than 2, a siloxane group, an oligosiloxane group having two or more silicon atoms, or a substituted or unsubstituted trialkylsilyl group;

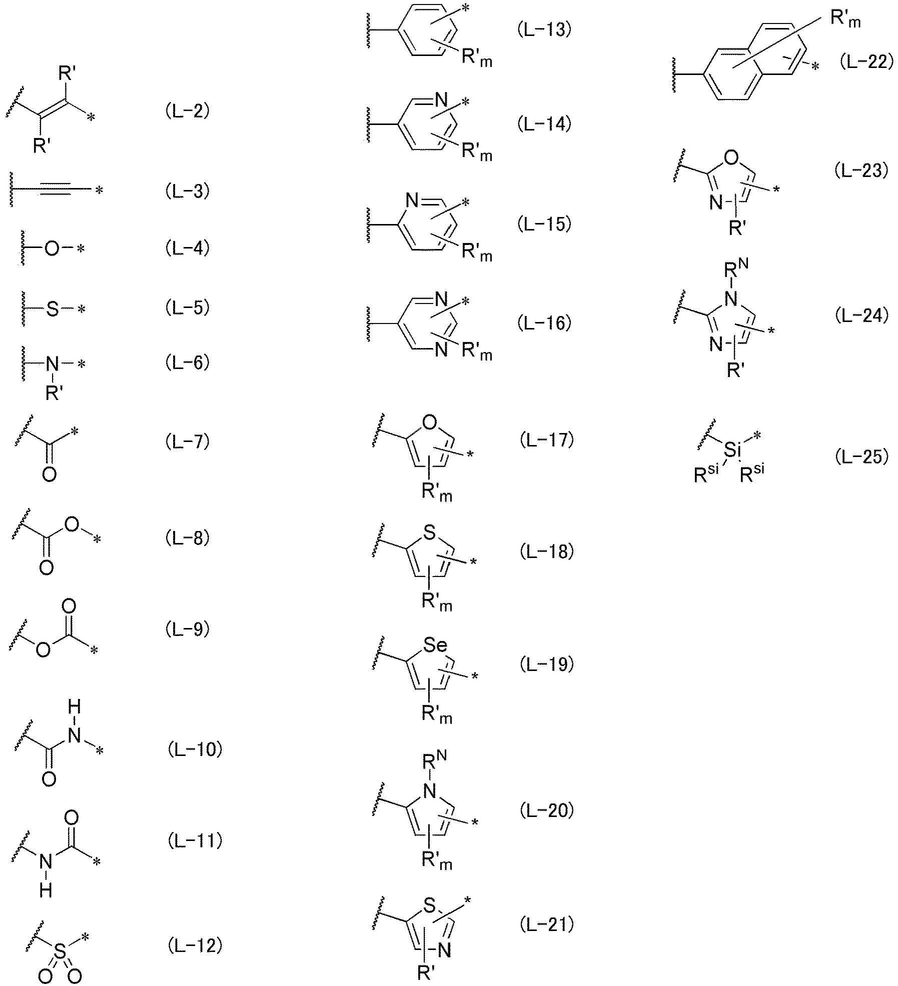

in Formulae (L-2) to (L-25), the portion of a wavy line represents a position of bonding to a condensed cyclic skeleton,
m in Formula (L-13) represents 4, m in Formulae (L-14) and (L-15) represents 3, m in Formulae (L-16) to (L-20) represents 2, m in Formula (L-22) represents 6,
each R' in Formulae (L-2), (L-6), and (L-13) to (L-24) independently represents a hydrogen atom or a substituent,
R^{N} represents a hydrogen atom or a substituent, and
each R^{si} independently represents a hydrogen atom, an alkyl group, an alkenyl group, or an alkynyl group.

Each of X¹ and X² in Formula (2-1-1) independently represents an oxygen atom or a sulfur atom. The preferred range of X¹ and X² in Formula (2-1-1) is the same as the preferred range of X¹ and X² in Formula (1-1).

Each of R¹ to R⁷ and R⁹ in Formula (2-1-1) independently represents a hydrogen atom or a substituent. Here, R⁴ is not a group represented by -L^{a}-R^{a}. In a case where each of R¹ to R⁷ and R⁸ in Formula (2-1-1) represents a substituent, the preferred range of the substituent is the same the preferred range of the substituent that is represented by each of R¹ to R⁹ in Formula (1-1) and other than the substituent represented by Formula (W).

L^{a} in Formula (2-1-1) represents a divalent linking group represented by any of Formulae (L-1) to (L-25) or a divalent linking group in which two or more divalent linking groups represented by any of the following Formulae (L-1) to (L-25) are bonded to each other. R^{a} represents a substituted or unsubstituted alkyl group, a cyano group, a vinyl group, an ethynyl group, an oxyethylene group, an oligo-oxyethylene group in which a repetition number v of an oxyethylene unit is equal to or greater than 2, a siloxane group, an oligosiloxane group having two or more silicon atoms, or a substituted or unsubstituted trialkylsilyl group. The preferred range of L^{a} and R^{a} in Formula (2-1-1) is the same as the preferred range of L and R in Formula (1-1).

Each of X¹ and X² in Formula (2-1-2) independently represents an oxygen atom or a sulfur atom. The preferred range of X¹ and X² in Formula (2-1-2) is the same as the preferred range of X¹ and X² in Formula (1-1).

Each of R¹ to R³, R⁵ to R⁷, and R⁹ in Formula (2-1-2) independently represents a hydrogen atom or a substituent. In a case where each of R¹ to R³, R⁵ to R⁷, and R⁹ in Formula (2-1-2) represents a substituent, the preferred range of the substituent is the same as the preferred range of the substituent that is represented by each of R¹ to R⁹ in Formula (1-1) and other than the substituent represented by Formula (W).

Each of L^{b} and L^{c} in Formula (2-1-2) represents a divalent linking group represented by any of Formulae (L-1) to (L-25) or a divalent linking group in which two or more divalent linking groups represented by any of the following Formulae (L-1) to (L-25) are bonded to each other. Each of R^{b} and R^{c} represents a substituted or unsubstituted alkyl group, a cyano group, a vinyl group, an ethynyl group, an oxyethylene group, an oligo-oxyethylene group in which a repetition number v of an oxyethylene unit is equal to or greater than 2, a siloxane group, an oligosiloxane group having two or more silicon atoms, or a substituted or unsubstituted trialkylsilyl group. The preferred range of L^{b}, L^{c}, R^{b}, and R^{c} in Formula (2-1-2) is the same as the preferred range of L and R in Formula (1-1).

The compound represented by Formula (1-2) is preferably a compound represented by the following Formula (2-2-1) or (2-2-2), and particularly preferably a compound represented by Formula (2-2-2) from the viewpoint of high mobility. 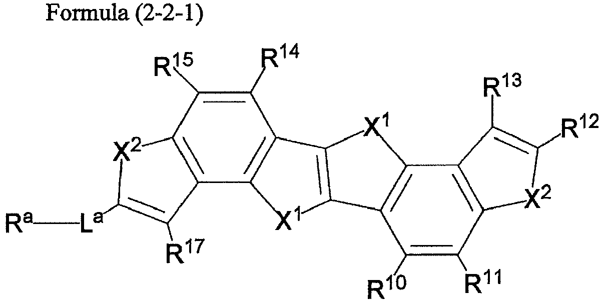 In Formula (2-2-1),
each X¹ independently represents a sulfur atom, an oxygen atom, a selenium atom, or NR⁹,
each X² independently represents a sulfur atom, an oxygen atom, or a selenium atom,
each of R¹⁰, R¹² to R¹⁵, and R¹⁷ independently represents a hydrogen atom or a substituent, R¹² is not a group represented by -L^{a}-R^{a},
L^{a} represents a divalent linking group represented by any of the following Formulae (L-1) to (L-25) or a divalent linking group in which two or more divalent linking groups represented by any of the following Formulae (L-1) to (L-25) are bonded to each other, and
R^{a} represents a substituted or unsubstituted alkyl group, a cyano group, a vinyl group, an ethynyl group, an oxyethylene group, an oligo-oxyethylene group in which a repetition number v of an oxyethylene unit is equal to or greater than 2, a siloxane group, an oilgosiloxane group having two or more silicon atoms, or a substituted or unsubstituted trialkylsilyl group; 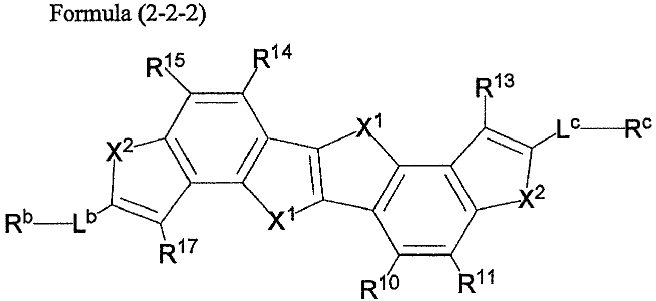
in Formula (2-2-2),
each X¹ independently represents a sulfur atom, an oxygen atom, a selenium atom, or NR⁹,
each X² independently represents a sulfur atom, an oxygen atom, or a selenium atom,
each of R¹⁰, R¹¹, R¹³ to R¹⁵, and R¹⁷ independently represents a hydrogen atom or a substituent,
each of L^{b} and L^{c} independently represents a divalent linking group represented by any of the following Formulae (L-1) to (L-25) or a divalent linking group in which two or more divalent linking groups represented by any of the following Formulae (L-1) to (L-25) are bonded to each other, and
each of R^{b} and R^{c} independently represents a substituted or unsubstituted alkyl group, a cyano group, a vinyl group, an ethynyl group, an oxyethylene group, an oligo-oxyethylene group in which a repetition number v of an oxyethylene unit is equal to or greater than 2, a siloxane group, an oligosiloxane group having two or more silicon atoms, or a substituted or unsubstituted trialkylsilyl group;

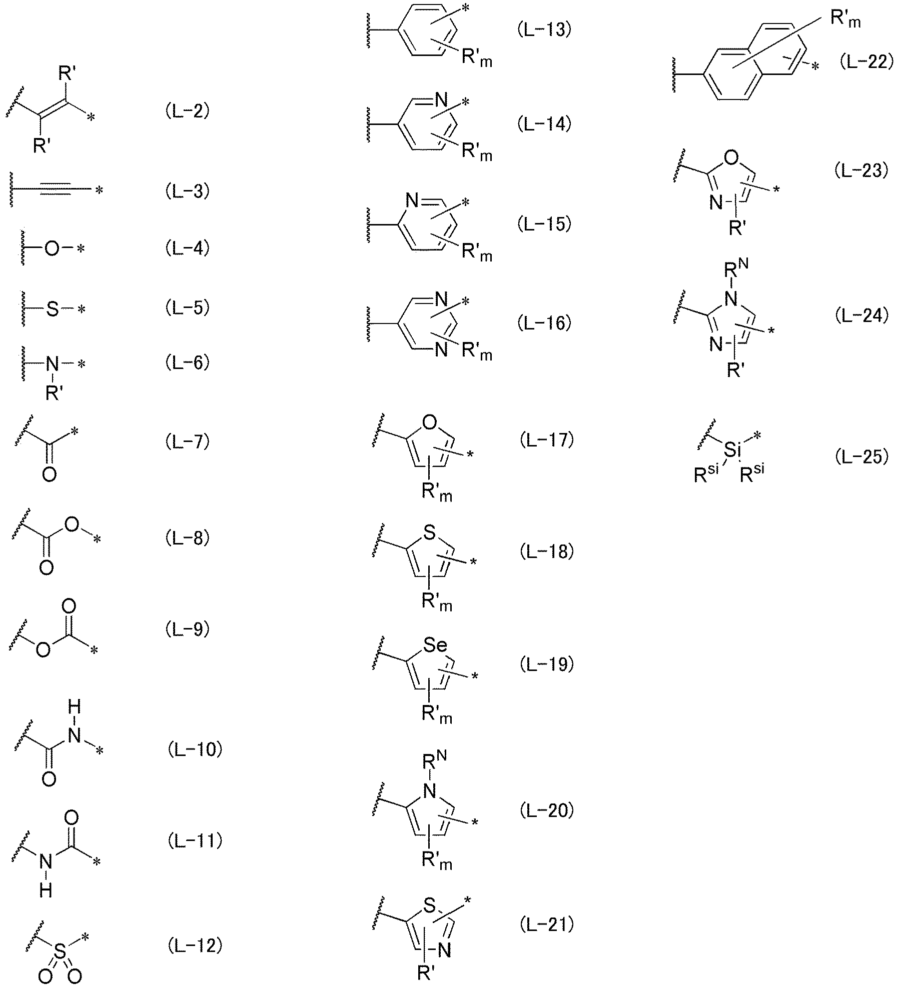

in Formulae (L-2) to (L-25), the portion of a wavy line represents a position of bonding to a condensed cyclic skeleton,
m in Formula (L-13) represents 4, m in Formulae (L-14) and (L-15) represents 3, m in Formulae (L-16) to (L-20) represents 2, m in Formula (L-22) represents 6,
each R' in Formulae (L-2), (L-6), and (L-13) to (L-24) independently represents a hydrogen atom or a substituent,
R^{N} represents a hydrogen atom or a substituent, and
each R^{si} independently represents a hydrogen atom, an alkyl group, an alkenyl group, or an alkynyl group.

Each of X¹ and X² in Formula (2-2-1) independently represents an oxygen atom or a sulfur atom. The preferred range of X¹ and X² in Formula (2-1-1) is the same as the preferred range of X¹ and X² in Formula (1-2).

Each of R¹⁰, R¹² to R¹⁵, and R¹⁷ in Formula (2-2-1) independently represents a hydrogen atom or a substituent. Here, R¹² is not a group represented by -L^{a}-R^{a}. In a case where each of R¹⁰, R¹² to R¹⁵, and R¹⁷ in Formula (2-2-1) represents a substituent, the preferred range of the substituent is the same as the preferred range of the substituent that is represented by each of R⁹ to R¹⁷ in Formula (1-2) and is other than the substituent represented by Formula (W).

L^{a} in Formula (2-2-1) represents a divalent linking group represented by any of Formulae (L-1) to (L-25) or a divalent linking group in which two or more divalent linking groups represented by any of the following Formulae (L-1) to (L-25) are bonded to each other. R^{a} represents a substituted or unsubstituted alkyl group, a cyano group, a vinyl group, an ethynyl group, an oxyethylene group, an oligo-oxyethylene group in which a repetition number v of an oxyethylene unit is equal to or greater than 2, a siloxane group, an oligosiloxane group having two or more silicon atoms, or a substituted or unsubstituted trialkylsilyl group. The preferred range of L^{a} and R^{a} in Formula (2-2-1) is the same as the preferred range of L and R in Formula (1-2).

Each of X¹ and X² in Formula (2-2-2) independently represents an oxygen atom or a sulfur atom. The preferred range of X¹ and X² in Formula (2-2-2) is the same as the preferred range of X¹ and X² in Formula (1-2).

Each of R¹⁰, R¹¹, R¹³ to R¹⁵, and R¹⁷ in Formula (2-2-2) independently represents a hydrogen atom or a substituent. In a case where each of R¹⁰, R¹¹, R¹³ to R¹⁵, and R¹⁷ in Formula (2-2-2) represents a substituent, the preferred range of the substituent is the same as the preferred range of the substituent that is represented by each of R⁹ to R¹⁷ in Formula (1-2) and other than the substituent represented by Formula (W).

Each of L^{b} and L^{c} in Formula (2-2-2) represents a divalent linking group represented by any of Formulae (L-1) to (L-25) or a divalent linking group in which two or more divalent linking groups represented by any of the following Formulae (L-1) to (L-25) are bonded to each other. Each of R^{b} and R^{c} represents a substituted or unsubstituted alkyl group, a cyano group, a vinyl group, an ethynyl group, an oxyethylene group, an oligo-oxyethylene group in which a repetition number v of an oxyethylene unit is equal to or greater than 2, a siloxane group, an oligosiloxane group having two or more silicon atoms, or a substituted or unsubstituted trialkylsilyl group. The preferred range of L^{b}, L^{c}, R^{b}, and R^{c} in Formula (2-2-2) is the same as the preferred range of L and R in Formula (1-2).

Specific examples of the compound represented by the Formula (1-1) or (1-2) are shown below, but the compound represented by Formula (1-1) or (1-2) that can be used in the present invention is not limited to the specific examples.

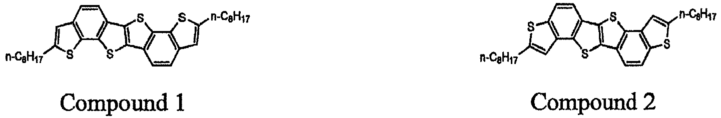

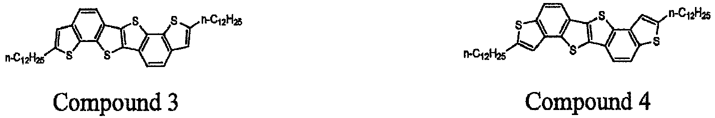

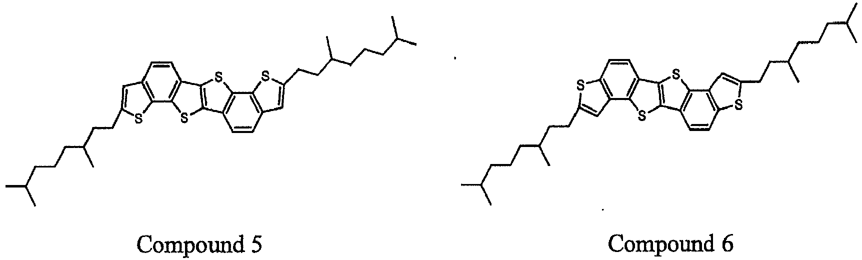

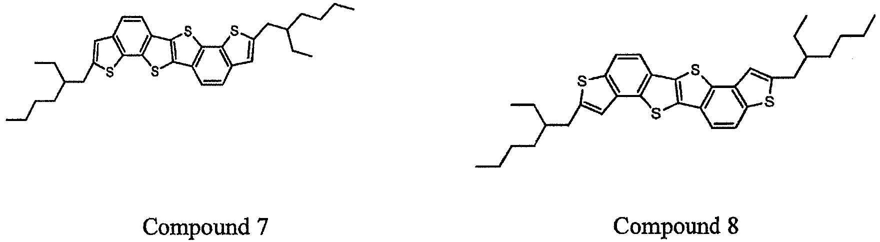

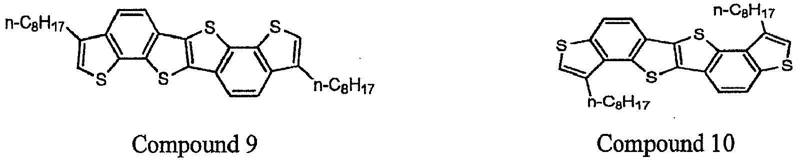

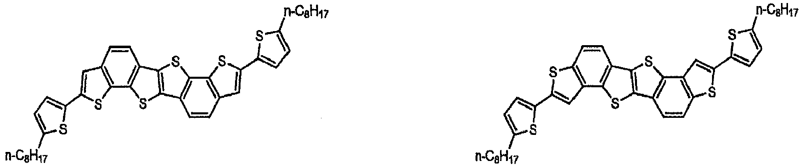

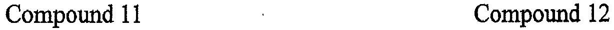

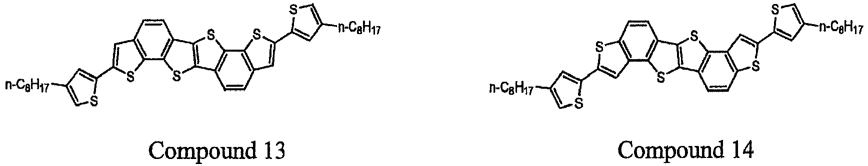

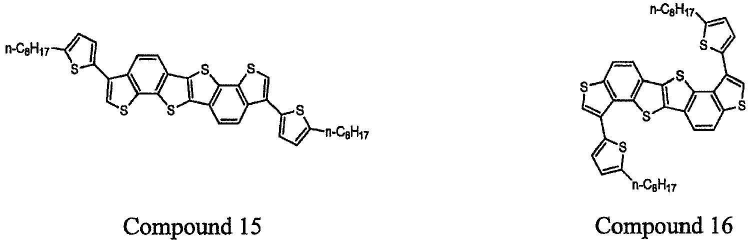

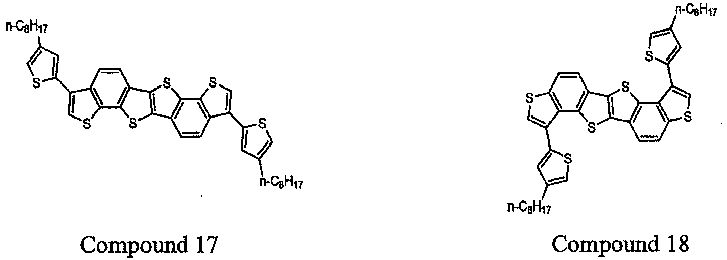

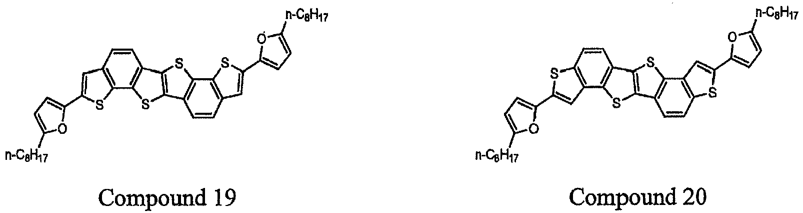

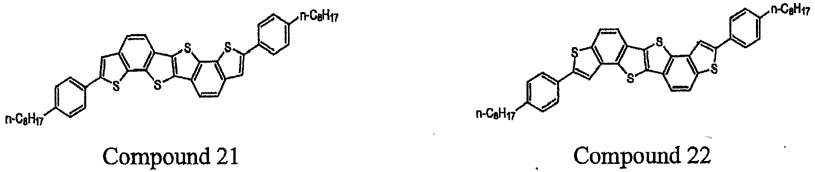

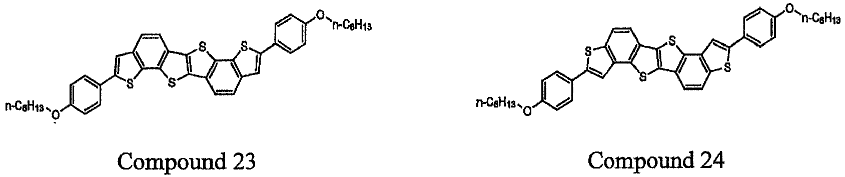

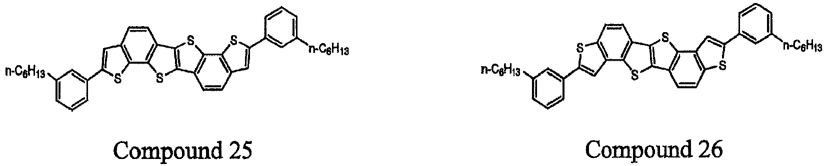

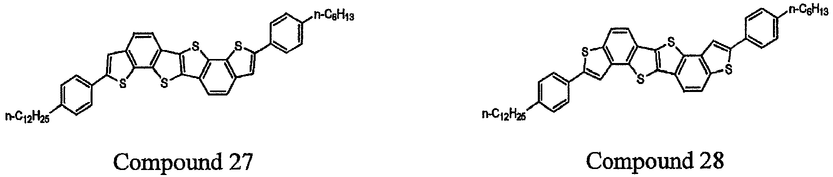

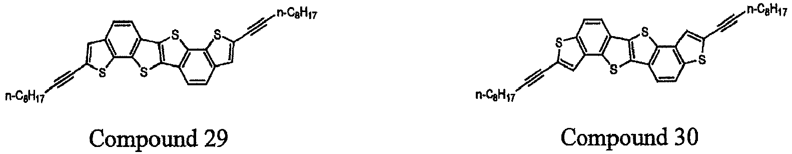

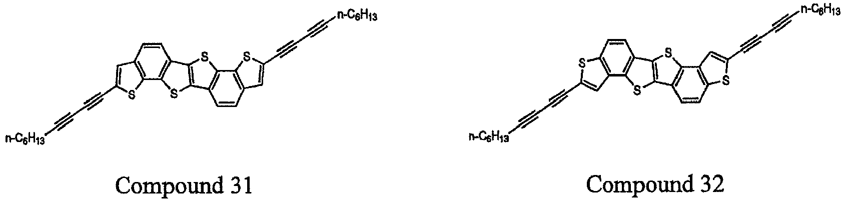

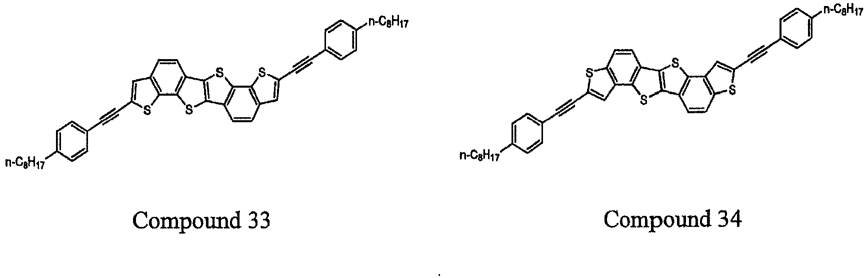

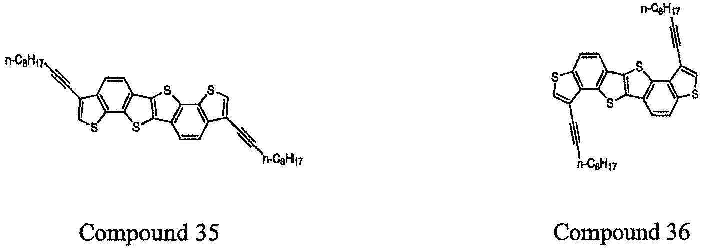

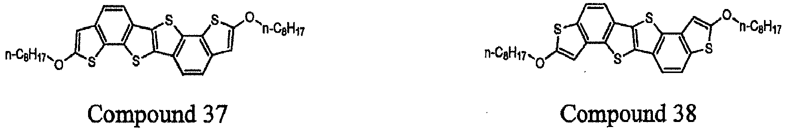

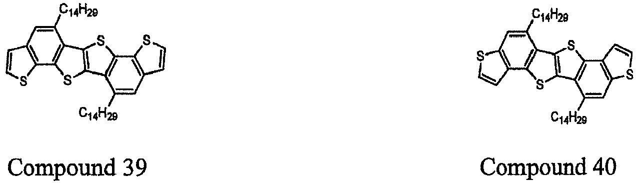

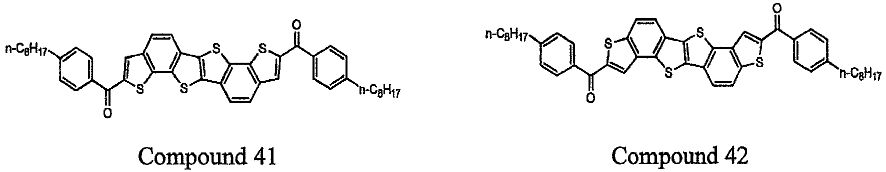

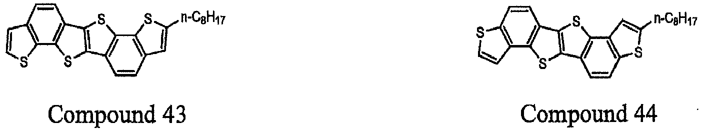

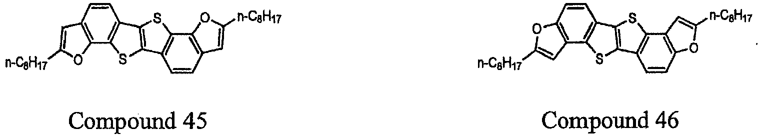

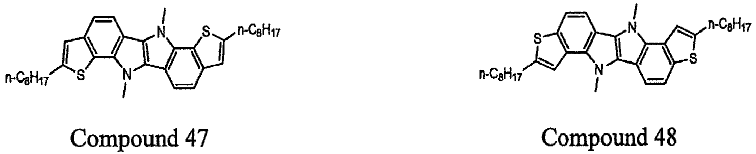

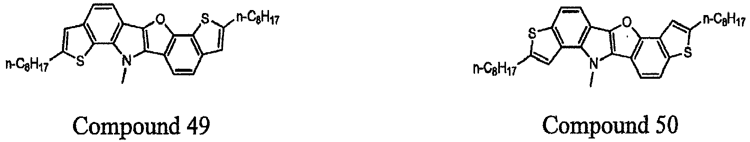

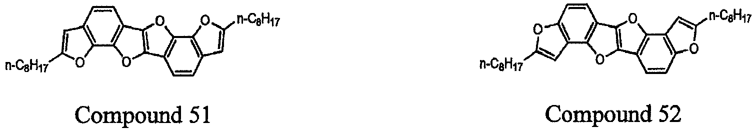

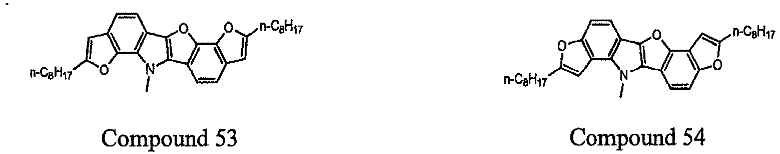

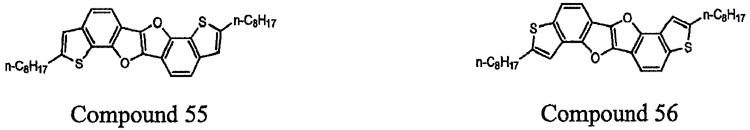

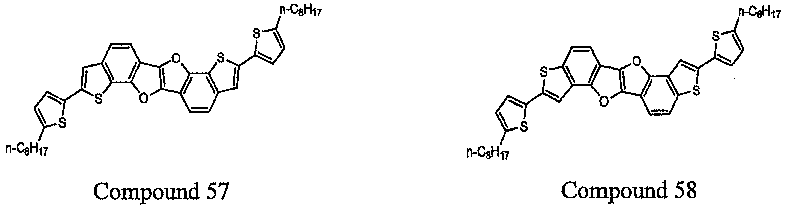

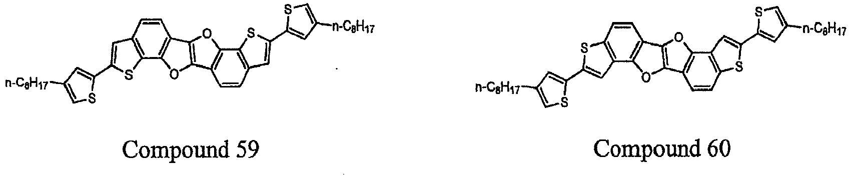

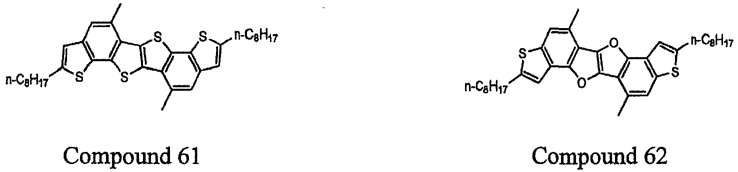

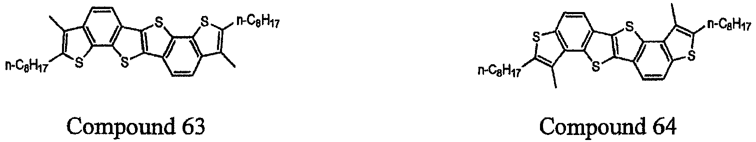

The compound represented by the Formula (1-1) or (1-2) may have a repeating structure and may be a low-molecular weight compound or a polymer compound. In a case where the compound represented by the Formula (1-1) or (1-2) is a low-molecular weight compound, the molecular weight thereof is preferably equal to or less than 3,000, more preferably equal to or less than 2,000, even more preferably equal to or less than 1,000, and particularly preferably equal to or less than 850. It is preferable that the molecular weight is equal to or less than the upper limit described above because then the solubility in a solvent can be improved.

In contrast, from the viewpoint of the stability of the film quality, the molecular weight is preferably equal to or greater than 400, more preferably equal to or greater than 450, and even more preferably equal to or greater than 500.

Furthermore, in a case where the compound represented by Formula (1-1) or (1-2) is a polymer compound having a repeating structure, the weight average molecular weight thereof is preferably equal to or greater than 30,000, more preferably equal to or greater than 50,000, and even more preferably equal to or greater than 100,000. In a case where the compound represented by Formula (1-1) or (1-2) is a polymer compound having a repeating structure, it is preferable that the weight average molecular weight thereof is equal to or greater than the lower limit described above because then the intermolecular interaction can be enhanced, and hence high mobility is obtained.

Examples of the polymer compound having a repeating structure include a π-conjugated polymer having a repeating structure in which the compound represented by Formula (1-1) or (1-2) represents at least one or more arylene groups or heteroarylene groups (thiophene or bithiophene), and a pendant-type polymer in which the compound represented by Formula (1-1) or (1-2) is bonded to the main chain of the polymer via the side chain. As the main chain of the polymer, polyacrylate, polyvinyl, polysiloxane, or the like is preferable, and as the side chain, an alkylene group, a polyethylene oxide group, or the like is preferable.

The compound represented by the Formula (1-1) can be synthesized with reference to known documents (Macromolecules, 2010, 43, 6264-6267, J. Am. Chem. Soc. 2012, 134, 16548-16550) by using, as a starting material, a compound a described in scheme 1 which will be described later.

Furthermore, the compound represented by Formula (1-2) can be synthesized with reference to the known documents described above by using, as a starting material, a compound a described in scheme 2 which will be described later.

In synthesizing the compound of the present invention, any of reaction conditions may be used. As a reaction solvent, any solvent may be used. Furthermore, in order to accelerating a ring forming reaction, an acid or a base is preferably used, and a base is particularly preferably used. The optimal reaction conditions vary with the structure of the intended compound, but can be set with reference to the specific reaction conditions described in the above documents.

### <Intermediate compound>

The synthetic intermediate having various substituents can be synthesized using known reactions in combination. Furthermore, various substituents may be introduced into the intermediate at any stage. After the intermediate is synthesized, it is preferable to purify the intermediate by column chromatography, recrystallization, or the like and then further purify it by sublimation. By the sublimation purification, it is possible to separate organic impurities and to effectively remove an inorganic salt, a residual solvent, and the like.

The present invention also relates to a compound represented by the following Formula (3-1) and a compound represented by the following Formula (3-2). 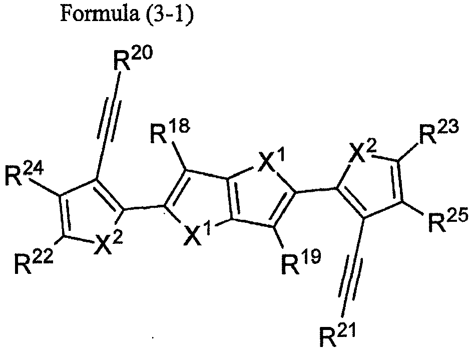 In Formula (3-1),
each X¹ independently represents a sulfur atom, an oxygen atom, a selenium atom, or NR⁹,
each X² independently represents a sulfur atom, an oxygen atom, or a selenium atom,
each of R⁹ and R¹⁸ to R²⁵ independently represents a hydrogen atom or a substituent, and at least either R²² or R²³ represents an alkyl group, an alkenyl group, an alkynyl group, a substituted or unsubstituted aryl group, a substituted or unsubstituted heteroaryl group, an alkylcarbonyl group, or a substituted or unsubstituted arylcarbonyl group; 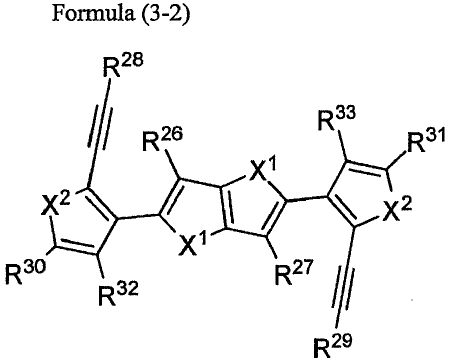
in Formula (3-2),
each X¹ independently represents a sulfur atom, an oxygen atom, a selenium atom, or NR⁹,
each X² independently represents a sulfur atom, an oxygen atom, or a selenium atom,
each of R⁹ and R²⁶ to R³³ independently represents a hydrogen atom or a substituent, and at least either R³⁰ or R³¹ represents an alkyl group, an alkenyl group, an alkynyl group, a substituted or unsubstituted aryl group, a substituted or unsubstituted heteroaryl group, an alkylcarbonyl group, or a substituted or unsubstituted arylcarbonyl group.

It is preferable that the compound represented by Formula (3-1) described above is an intermediate compound of the compound represented by Formula (1-1) described above. Furthermore, it is preferable that the compound represented by Formula (3-2) described above is an intermediate compound of the compound represented by Formula (1-2) described above.

The compound represented by Formula (1-1) described above can be synthesized according to the scheme 1, which will be described later, through a process in which the compound represented by Formula (3-1) described above is synthesized as an intermediate compound thereof.

In addition, the compound represented by Formula (1-2) described above can be synthesized according to the scheme 2, which will be described later, through a process in which the compound represented by Formula (3-2) described above is synthesized as an intermediate compound thereof.

Each of X¹ and X² in Formula (3-1) has the same definition as each of X¹ and X² in Formula (1-1), and the preferred range thereof is also the same.

Each of R⁹ and R¹⁸ to R²³ in Formula (3-1) independently represents a hydrogen atom or a substituent. At least either R²² or R²³ represents an alkyl group, an alkenyl group, an alkynyl group, an alkylcarbonyl group, or a substituted or unsubstituted arylcarbonyl group.

In a case where each of R⁹ and R¹⁸ to R²³ in Formula (3-1) represents a substituent, the preferred range of the substituent is the same as the preferred range of the substituent that is represented by each of R¹ to R⁸ in Formula (1-1) and other than the substituent represented by Formula (W).

In a case where each of R²² and R²³ in Formula (3-1) represents an alkyl group, the alkyl group is preferably an alkyl group having 1 to 18 carbon atoms, more preferably an alkyl group having 5 to 12 carbon atoms, and particularly preferably an alkyl group having 8 to 10 carbon atoms.

In a case where each of R²² and R²³ in Formula (3-1) represents an alkenyl group, the alkenyl group is preferably an alkenyl group having 2 to 18 carbon atoms, and more preferably an alkenyl group having 5 to 12 carbon atoms.

In a case where each of R²² and R²³ in Formula (3-1) represents an alkynyl group, the alkynyl group is preferably an alkynyl group having 2 to 18 carbon atoms, and more preferably an alkynyl group having 5 to 12 carbon atoms.

In a case where each of R²² and R²³ in Formula (3-1) represents a substituted or unsubstituted aryl group, examples of the aryl group include a benzene ring, a naphthalene ring, an anthracene ring, a tetracene ring, a pentacene ring, a phenanthrene ring, a chrysene ring, a triphenylene ring, a pyrene ring, and the like. Among these, an aryl group having 6 to 18 carbon atoms is preferable, an aryl group having 6 to 10 carbon atoms is more preferable, and an aryl group having 6 carbon atoms is particularly preferable. Examples of the substituent that the substituted aryl group represented by R²² and R²³ may have include the substituent that can be adopted as R¹ to R⁹ in Formula (1-1).

In a case where each of R²² and R²³ in Formula (3-1) represents a substituted or unsubstituted heteroaryl group, examples of the heteroaryl group include rings formed when carbon atoms forming the aforementioned aromatic rings are partially or totally substituted with a heteroatom such as an oxygen atom, a nitrogen atom, or a sulfur atom, a furan ring, a thiophene ring, a pyrrole ring, and the like. Furthermore, the aforementioned aromatic rings or heterocyclic aromatic rings may be condensed with each other or may be further condensed with an oxazole ring, a thiazole ring, an imidazole ring, a thiadiazole ring, and the like. Examples of the heterocyclic aromatic ring as a condensed heterocyclic aromatic ring include a quinoline ring, an isoquinoline ring, a naphthyridine ring, a quinoxaline ring, an acridine ring, a phenazine ring, a benzothiazole ring, a naphthobisthiadiazole ring, a thieno[3,2-b]thiophene ring, a carbazole ring, and the like. Among these, a heteroaryl group having 5 to 18 carbon atoms is preferable, a heteroaryl group having 5 to 10 carbon atoms is more preferable, and a heteroaryl group having 5 carbon atoms is particularly preferable. Examples of the substituent that the substituted heteroaryl group represented by R²² and R²³ may have include the substituents that can be adopted as R¹ to R⁹ in Formula (1-1).

In a case where each of R²² and R²³ in Formula (3-1) represents an alkylcarbonyl group, the alkylcarbonyl group is preferably an alkylcarbonyl group having 2 to 18 carbon atoms, more preferably an alkylcarbonyl group having 5 to 12 carbon atoms, and particularly preferably an alkylcarbonyl group having 8 to 10 carbon atoms.

In a case where each of R²² and R²³ in Formula (3-1) represents an aryl carbonyl group, the arylcarbonyl group is preferably an arylcarbonyl group having 7 to 25 carbon atoms, more preferably an arylcarbonyl group having 12 to 19 carbon atoms, and particularly preferably an arylcarbonyl group having 15 to 17 carbon atoms.

Each of X¹ and X² in Formula (3-2) has the same definition as each of X¹ and X² in Formula (1-2), and the preferred range thereof is also the same.

Each of R⁹ and R²⁶ to R³³ in Formula (3-2) independently represents a hydrogen atom or a substituent. At least either R³⁰ or R³¹ represents an alkyl group, an alkenyl group, an alkynyl group, a substituted or unsubstituted aryl group, a substituted or unsubstituted heteroaryl group, an alkylcarbonyl group, or a substituted or unsubstituted arylcarbonyl group.

In a case where each of R⁹ and R²⁶ to R³³ in Formula (3-2) represents a substituent, the preferred range of the substituent is the same as the preferred range of the substituent that is represented by each of R¹⁰ to R¹⁷ in Formula (1-2) and other than the substituent represented by Formula (W).

In a case where each of R³⁰ and R³¹ in Formula (3-2) represents an alkyl group, an alkenyl group, an alkynyl group, a substituted or unsubstituted aryl group, a substituted or unsubstituted heteroaryl group, an alkylcarbonyl group, or a substituted or unsubstituted arylcarbonyl group, the specific examples and preferred range of these groups are the same as the specific examples and preferred range of the groups represented by R²² and R²³ in Formula (3-1).

### <Structure of organic transistor>

The organic transistor of the present invention has a semiconductor active layer containing the compound represented by the Formula (1-1) or (1-2).

The organic transistor of the present invention may further have layers other than the semiconductor active layer.

The organic transistor of the present invention is preferably used as an organic field effect transistor (FET), and is more preferably used as an insulated gate-type FET in which the gate is insulated from channels.

Hereinafter, preferred structural aspects of the organic transistor of the present invention will be specifically described by using drawings, but the present invention is not limited to the aspects.

### (Lamination structure)

The lamination structure of an organic field effect transistor is not particularly limited, and various known structures can be adopted.

For example, the organic transistor of the present invention can adopt a structure (bottom gate/top contact type) in which an electrode, an insulator layer, a semiconductor active layer (organic semiconductor layer), and two electrodes are arranged in this order on the upper surface of a substrate which is a lower most layer. In this structure, the electrode on the upper surface of the substrate as the lower most layer is provided in a portion of the substrate, and the insulator layer is so disposed that it comes into contact with the substrate in a portion other than the electrode. The two electrodes provided on the upper surface of the semiconductor active layer are arranged in a state of being separated from each other.

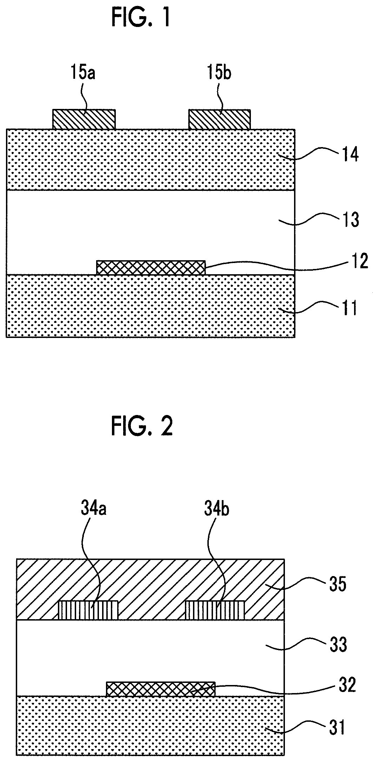

Fig. 1 shows the constitution of a bottom gate/top contact-type element. Fig. 1 is a schematic view showing a section of an exemplary structure of the organic transistor of the present invention. In the organic transistor shown in Fig. 1, a substrate 11 is disposed as a lower most layer, an electrode 12 is provided in a portion of the upper surface thereof, and an insulator layer 13 is provided such that it covers the electrode 12 and comes into contact with the substrate 11 in a portion other than the electrode 12. On the upper surface of the insulator layer 13, a semiconductor active layer 14 is provided, and in a portion of the upper surface thereof, two electrodes 15a and 15b separated from each other are arranged.

In the organic transistor shown in Fig. 1, the electrode 12 is a gate, and the electrode 15a and the electrode 15b are a drain and a source respectively. The organic transistor shown in Fig. 1 is an insulated gate-type FET in which a channel as a path of electric currents between the drain and the source is insulated from the gate.

As an example of the structure of the organic transistor of the present invention, a bottom gate/bottom contact-type element can be exemplified.

Fig. 2 shows the constitution of the bottom gate/bottom contact-type element. Fig. 2 is a schematic view showing a section of the structure of an organic transistor manufactured as a substrate for measuring FET characteristics in examples of the present invention. In the organic transistor shown in Fig. 2, a substrate 31 is disposed as a lower most layer, an electrode 32 is provided in a portion of the upper surface thereof, and an insulator layer 33 is provided such that it covers the electrode 32 and comes into contact with the substrate 31 in a portion other than the electrode 32. Furthermore, a semiconductor active layer 35 is provided on the upper surface of the insulator layer 33, and electrodes 34a and 34b are in a lower portion of the semiconductor active layer 35.

In the organic transistor shown in Fig. 2, the electrode 32 is a gate, and the electrode 34a and the electrode 34b are a drain and a source respectively. The organic transistor shown in Fig. 2 is an insulated gate-type FET in which a channel as a path of electric currents between the drain and the source is insulated from the gate.

As the structure of the organic transistor of the present invention, a top gate/top contact-type element in which an insulator and a gate electrode are in the upper portion of a semiconductor active layer or a top gate/bottom contact-type element can also be preferably used.

### (Thickness)

In a case where the organic transistor of the present invention needs to be a thinner transistor, the total thickness of the transistor is preferably, for example, 0.1 µm to 0.5 µm.

### (Sealing)

In order to improve the preservation stability of the organic transistor element by blocking the organic transistor element from the atmosphere or moisture, the entirety of the organic transistor element may be sealed with a metal sealing can, glass, an inorganic material such as silicon nitride, a polymer material such as parylene, a low-molecular weight material, or the like.

Hereinafter, preferred aspects of the respective layers of the organic transistor of the present invention will be described, but the present invention is not limited to the aspects.

### <Substrate>

### (Material)

The organic transistor of the present invention preferably includes a substrate.

The material of the substrate is not particularly limited, and known materials can be used. Examples of the material include a polyester film such as polyethylene naphthalate (PEN) or polyethylene terephthalate (PET), a cycloolefin polymer film, a polycarbonate film, a triacetylcellulose (TAC) film, a polyimide film, a material obtained by bonding these polymer films to extremely thin glass, ceramics, silicon, quartz, glass, and the like. Among these, silicon is preferable.

### <Electrode>

### (Material)

The organic transistor of the present invention preferably includes an electrode.

As the material constituting the electrode, known conductive materials such as a metal material like Cr, Al, Ta, Mo, Nb, Cu, Ag, Au, Pt, Pd, In, Ni, or Nd, an alloy material of these, a carbon material, and a conductive polymer can be used without particular limitation.

### (Thickness)

The thickness of the electrode is not particularly limited, but is preferably 10 nm to 50 nm.

A gate width (or a channel width) W and a gate length (or a channel length) L are not particularly limited. However, a ratio of W/L is preferably equal to or greater than 10, and more preferably equal to or greater than 20.

### <Insulating layer>

### (Material)

The material constituting the insulating layer is not particularly limited as long as an insulating effect is obtained as required. Examples of the material include silicon dioxide, silicon nitride, a fluorine polymer-based insulating material such as PTFE or CYTOP, a polyester insulating material, a polycarbonate insulating material, an acryl polymer-based insulating material, an epoxy resin-based insulating material, a polyimide insulating material, a polyvinyl phenol resin-based insulating material, a poly p-xylylene resin-based insulating material, and the like.

A surface treatment may be performed on the upper surface of the insulating layer. For example, it is possible to preferably use an insulating layer in which the silicon dioxide surface thereof is subjected to the surface treatment by being coated with hexamethyldisilazane (HMDS) or octadecyltrichlorosilane (OTS).

### (Thickness)

The thickness of the insulating layer is not particularly limited. However, in a case where the film needs to be thinned, the thickness of the insulating layer is preferably 10 nm to 400 nm, more preferably 20 nm to 200 nm, and particularly preferably 50 nm to 200 nm.

### <Semiconductor active layer>

### (Material)

In the organic transistor of the present invention, the semiconductor active layer contains a compound represented by Formula (1-1) or (1-2), that is, the compound of the present invention.

The semiconductor active layer may be a layer consisting of the compound of the present invention or a layer further containing a polymer binder, which will be described later, in addition to the compound of the present invention. Furthermore, the semiconductor active layer may contain a residual solvent used at the time of forming a film.

The content of the polymer binder in the semiconductor active layer is not particularly limited. However, the content of the polymer binder is preferably within a range of 0% by mass to 95% by mass, more preferably within a range of 10% by mass to 90% by mass, even more preferably within a range of 20% by mass to 80% by mass, and particularly preferably within a range of 30% by mass to 70% by mass.

### (Thickness)

The thickness of the semiconductor active layer is not particularly limited. However, in a case where the film needs to be thinned, the thickness of the semiconductor active layer is preferably 10 nm to 400 nm, more preferably 10 nm to 200 nm, and particularly preferably 10 nm to 100 nm.

### [Organic semiconductor material for non-light-emitting organic semiconductor device]

The present invention also relates to an organic semiconductor material for a non-light-emitting organic semiconductor device containing the compound represented by Formula (1-1) or (1-2), that is, the compound of the present invention.

### (Non-light-emitting organic semiconductor device)

In the present specification, a "non-light-emitting organic semiconductor device" refers to a device which is not used for the purpose of emitting light. The non-light-emitting organic semiconductor device preferably uses an electronic element having a layered structure consisting of films. The non-light-emitting organic semiconductor device includes an organic transistor, an organic photoelectric conversion element (a solid-state imaging element used for a photosensor, a solar cell used for energy conversion, or the like), a gas sensor, an organic rectifying element, an organic inverter, an information recording element, and the like. The organic photoelectric conversion element can be used for both a photosensor (solid-state imaging element) and for energy conversion (a solar cell). Among these, an organic photoelectric conversion element and an organic transistor are preferable, and an organic transistor is more preferable. That is, the organic semiconductor material for a non-light-emitting organic semiconductor device of the present invention is preferably a material for an organic transistor as described above.

### (Organic semiconductor material)

In the present specification, the "organic semiconductor material" is an organic material showing characteristics of a semiconductor. Just as the semiconductor composed of an inorganic material, the organic semiconductor is classified into a p-type (hole-transporting) organic semiconductor material conducting holes as carriers and an n-type (electron-transporting) organic semiconductor material conducting electrons as carriers.

The compound of the present invention may be used as any of the p-type organic semiconductor material and the n-type organic semiconductor material, but is preferably used as the p-type. The ease with which the carriers flow in the organic semiconductor is represented by a carrier mobility µ. The higher the carrier mobility µ, the better. The carrier mobility µ is preferably equal to or greater than 1 × 10⁻³ cm²/Vs, more preferably equal to or greater than 5 × 10⁻³ cm²/Vs, particularly preferably equal to or greater than 1 × 10⁻² cm²/Vs, more particularly preferably equal to or greater than 5 × 10⁻² cm²/Vs, even more particularly preferably equal to or greater than 1 × 10⁻¹ cm²/Vs, and still more particularly preferably equal to or greater than 3 × 10⁻¹ cm²/Vs. The carrier mobility µ can be determined by the characteristics of the prepared field effect transistor (FET) element or by a time-of-flight (TOF) measurement method.

### [Organic semiconductor film for non-light-emitting organic semiconductor device]

### (Material)

The present invention also relates to an organic semiconductor film for a non-light-emitting organic semiconductor device containing the compound represented by Formula (1-1) or (1-2) described above, that is, the compound of the present invention.

As the organic semiconductor film for a non-light-emitting organic semiconductor device of the present invention, an aspect is also preferable in which the organic semiconductor film contains the compound represented by Formula (1-1) or (1-2) and does not contain a polymer binder.

Furthermore, the organic semiconductor film for a non-light-emitting organic semiconductor device of the present invention may contain the compound represented by Formula (1-1) or (1-2) and a polymer binder.

Examples of the polymer binder include an insulating polymer such as polystyrene, polycarbonate, polyarylate, polyester, polyamide, polyimide, polyurethane, polysiloxane, polysulfone, polymethyl methacrylate, polymethyl acrylate, cellulose, polyethylene, or polypropylene, a copolymer of these, a photoconductive polymer such as polyvinylcarbazole or polysilane, a conductive polymer such as polythiophene, polypyrrole, polyaniline, or poly p-phenylenevinylene, and a semiconductor polymer.

One kind of the aforementioned polymer binder may be used singly, or plural kinds thereof may be used concurrently.

The organic semiconductor material may be uniformly mixed with the polymer binder. Alternatively, the organic semiconductor material and the polymer binder may be totally or partially in a phase separation state. From the viewpoint of the charge mobility, a structure, in which the organic semiconductor and the binder are in a phase separation state along the film thickness direction in the film, is the most preferable because then the binder does not hinder the organic semiconductor from moving a charge.

Considering the mechanical strength of the film, a polymer binder having a high glass transition temperature is preferable. Furthermore, considering the charge mobility, a polymer binder having a structure not containing a polar group, a photoconductive polymer, and a conductive polymer are preferable.

The amount of the polymer binder used is not particularly limited. However, in the organic semiconductor film for a non-light-emitting organic semiconductor device of the present invention, the amount of the polymer binder used is preferably within a range of 0% by mass to 95% by mass, more preferably within a range of 10% by mass to 90% by mass, even more preferably within a range of 20% by mass to 80% by mass, and particularly preferably within a range of 30% by mass to 70% by mass.

In the present invention, by adopting the aforementioned structure as the structure of the compound, an organic film having excellent film quality can be obtained. Specifically, because the compound obtained in the present invention has excellent crystallinity, a sufficient film thickness can be obtained, and the obtained organic semiconductor film for a non-light-emitting organic semiconductor device of the present invention has excellent quality.

### (Film forming method)

The compound of the present invention may be formed into a film on a substrate by any method.

At the time of forming the film, the substrate may be heated or cooled. By varying the temperature of the substrate, it is possible to control the film quality or the packing of molecules in the film. The temperature of the substrate is not particularly limited. However, it is preferably between 0°C to 200°C, more preferably between 15°C to 100°C, and particularly preferably between 20°C to 95°C.

The compound of the present invention can be formed into a film on a substrate by a vacuum process or a solution process, and both of the processes are preferable.

Specific examples of the film forming method by a vacuum process include a physical vapor deposition method such as a vacuum vapor deposition method, a sputtering method, an ion plating method, or a molecular beam epitaxy (MBE) method and a chemical vapor deposition (CVD) method such as plasma polymerization, and it is particularly preferable to use a vacuum vapor deposition method.

Herein, the film forming method by a solution process refers to a method of dissolving an organic compound in a solvent which can dissolve the compound and forming a film by using the solution. Specifically, it is possible to use general methods like a coating method such as a casting method, a dip coating method, a die coater method, a roll coater method, a bar coater method, or a spin coating method, various printing methods such as an ink jet method, a screen printing method, a gravure printing method, a flexographic printing method, an offset printing method, or a micro-contact printing method, and a Langmuir-Blodgett (LB) method. It is particularly preferable to use a casting method, a spin coating method, an ink jet method, a gravure printing method, a flexographic printing method, an offset printing method, or a micro-contact printing method.

The organic semiconductor film for a non-light-emitting organic semiconductor device of the present invention is preferably prepared by a solution coating method. In a case where the organic semiconductor film for a non-light-emitting organic semiconductor device of the present invention contains a polymer binder, it is preferable to prepare a coating solution by dissolving or dispersing a material, which will be formed into a layer, and a polymer binder in an appropriate solvent and to form the organic semiconductor film by various coating methods.

Hereinafter, a coating solution for a non-light-emitting organic semiconductor device of the present invention that can be used for forming a film by a solution process will be described.

### [Coating solution for non-light-emitting organic semiconductor device]

The present invention also relates to a coating solution for a non-light-emitting organic semiconductor device containing the compound represented by Formula (1-1) or (1-2).

In a case where a film is formed on a substrate by using a solution process, a material which will be formed into a layer is dissolved or dispersed in either or both of an appropriate organic solvent (for example, a hydrocarbon-based solvent such as hexane, octane, decane, toluene, xylene, mesitylene, ethylbenzene, decalin, or 1-methylnaphthalene, a ketone-based solvent such as acetone, methyl ethyl ketone, methyl isobutyl ketone, or cyclohexanone, a halogenated hydrocarbon-based solvent such as dichloromethane, chloroform, tetrachloromethane, dichloroethane, trichloroethane, tetrachloroethane, chlorobenzene, dichlorobenzene, or chlorotoluene, an ester-based solvent such as ethyl acetate, butyl acetate, or amyl acetate, an alcohol-based solvent such as methanol, propanol, butanol, pentanol, hexanol, cyclohexanol, methyl cellosolve, ethyl cellosolve, or ethylene glycol, an ether-based solvent such as dibutylether, tetrahydrofuran, dioxane, or anisole, an amide·imide-based solvent such as N,N-dimethylformamide, N,N-dimethylacetamide, 1-methyl-2-pyrrolidone, or 1-methyl-2-imidazolidinone, a sulfoxide-based solvent such as dimethyl sulfoxide, or a nitrile-based solvent such as acetonitrile) and water so as to obtain a coating solution, and a film can be formed by various coating methods by using the coating solution. One kind of the solvent may be used singly, or plural kinds thereof may be used in combination. Among these, a hydrocarbon-based solvent, a halogenated hydrocarbon-based solvent, and an ether-based solvent are preferable, toluene, xylene, mesitylene, tetralin, dichlorobenzene, and anisole are more preferable, and toluene, xylene, tetralin, and anisole are particularly preferable. The concentration of the compound represented by Formula (1-1) or (1-2) in the coating solution is preferably 0.1% by mass to 80% by mass, more preferably 0.1% by mass to 10% by mass, and particularly preferably 0.5% by mass to 10% by mass. In this way, a film having an arbitrary thickness can be formed.

In order to form a film by a solution process, the material needs to dissolve in the solvent exemplified above, but simply dissolving in a solvent is not good enough. Generally, even the material formed into a film by a vacuum process can dissolve in a solvent to some extent. The solution process includes a step of coating a substrate with a material by dissolving the material in a solvent and then forming a film by evaporating the solvent, and many of the materials not suitable for being formed into a film by the solution process have high crystallinity. Therefore, the material is inappropriately crystallized (aggregated) in the aforementioned step, and hence it is difficult to form an excellent film. The compound represented by Formula (1-1) or (1-2) is also excellent in the respect that it is not easily crystallized (aggregated).

As the coating solution for a non-light-emitting organic semiconductor device of the present invention, an aspect is also preferable in which the coating solution contains the compound represented by Formula (1-1) or (1-2) and does not contain a polymer binder.

Furthermore, the coating solution for a non-light-emitting organic semiconductor device of the present invention may contain the compound represented by Formula (1-1) or (1-2) and a polymer binder. In this case, a material, which will be formed into a layer, and a polymer binder are dissolved or dispersed in an appropriate solvent described above so as to prepare a coating solution, and by using the coating solution, a film can be formed by various coating methods. The polymer binder can be selected from those described above.

### Examples

Hereinafter, the characteristics of the present invention will be more specifically explained by describing examples and comparative examples. The materials, the amount thereof used, the proportion thereof, the content of treatment, the treatment procedure, and the like described in the following examples can be appropriately modified within a range that does not depart from the gist of the present invention. Accordingly, the scope of the present invention is not limited to the following specific examples.

### [Example 1]

### <Synthesis example 1> Synthesis of compounds 1, 3, 5, 9, 29, 37, 39, 43, and 51 and an intermediate compound g

According to a specific synthesis procedure shown in the following scheme 1, compounds 1, 3, 5, 29, 37, 39, 43, and 51 as the compound represented by Formula (1-1) and intermediate compound g were synthesized.

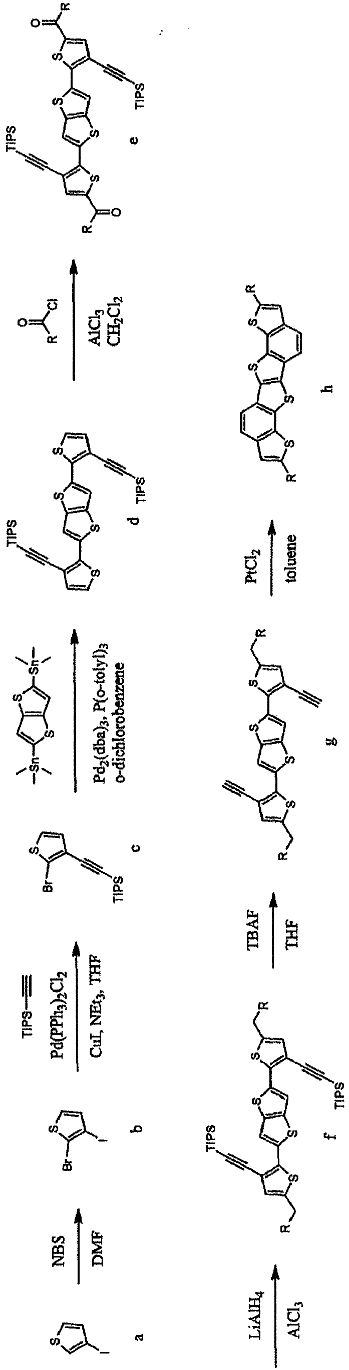

A process will be specifically described in which an intermediate compound g represented by Formula (3-1) and then a compound h represented by Formula (1-1) are synthesized from the compound a described above. In the following synthesis example, a case where R in the scheme 1 is a n-octyl group, that is, a case where the compound h is the compound 1 will be specifically described.

### Synthesis of compound b;

4.66 g of N-bromosuccinimide was added to 120 mL of a dimethylformamide solution containing 5.00 g of the compound a, followed by stirring for 12 hours at room temperature. Then, distilled water was added to the mixture, the mixture was moved to a liquid-separating funnel, and an organic layer was extracted using hexane. Subsequently, the organic layer was dried over sodium sulfate and concentrated under reduced pressure. Thereafter, the obtained residue was separated by silica gel chromatography (SiO₂; hexane). In this way, 6.70 g of a product (compound b, yield: 93%) was obtained.

### Synthesis of compound c;

In a nitrogen atmosphere, a mixed solution of 4.00 g of the compound b, 3.41 mL of triisopropylsilyl acetylene, 0.39 g of bis(triphenylphosphine)palladium (II) dichloride, 0.11 g of copper iodide, 40 mL of triethylamine, and 15 mL of THF was stirred for 12 hours at room temperature. Then, distilled water was added to the mixture, the mixture was moved to a liquid-separating funnel, and an organic layer was extracted using hexane. Subsequently, the organic layer was dried over sodium sulfate and concentrated under reduced pressure. Thereafter, the obtained residue was separated by silica gel chromatography (SiO₂; hexane). In this way, 3.90 g of a product (compound c, yield: 82%) was obtained.

### Synthesis of compound d;

In a nitrogen atmosphere, 30 mL of a chlorobenzene solution containing 1.80 g of the compound c, 1.2 g of 2,5-bis(trimethylstannyl)thieno[3,2-b]thiophene, 0.058 g of tris(dibenzylideneacetone)dipalladium (0), and 0.078 g of tris(2-methylphenyl)phosphine was heated to 130°C. Then, distilled water was added to the mixture, the mixture was moved to a liquid-separating funnel, and an organic layer was extracted using chloroform. Subsequently, the organic layer was dried over sodium sulfate and concentrated under reduced pressure. Thereafter, the obtained residue was separated by silica gel chromatography (SiO₂; hexane:dichloromethane = 100:2). In this way, 3.90 g of a product (compound d, yield: 82%) was obtained.

### Synthesis of compound e;

In a nitrogen atmosphere, 0.59 g of n-octanoyl chloride was added to 15 mL of a dichloromethane solution containing 1.00 g of the compound d and 0.95 g of aluminum chloride at room temperature, followed by stirring at room temperature. After 4 hours, methanol, water, and dilute hydrochloric acid (1 N) were added thereto in this order so as to stop the reaction, and the precipitated solid was separated by filtration. The obtained solid was separated by silica gel chromatography (SiO₂; hexane:toluene = 5:1). In this way, 0.91 g of a product (compound e, yield: 66%) was obtained.

### Synthesis of compound f;

In a nitrogen atmosphere, 0.66 g of lithium aluminum hydride was added to 30 mL of a tetrahydrofuran solution containing 0.80 g of the compound e and 0.61 g of aluminum chloride, followed by stirring for 6 hours at room temperature. Then, methanol, water, and dilute hydrochloric acid (1 N) were added thereto in this order so as to stop the reaction, and the precipitated solid was separated by filtration. The obtained solid was separated by silica gel chromatography (SiO₂; hexane:toluene = 10:1). In this way, 0.70 g of a product (compound f, yield: 90%) was obtained.

### Synthesis of compound g;

0.70 g of the compound f was added to 15 mL of a tetrahydrofuran solution containing tetra-n-butylammonium fluoride, and the resulting mixture was heated to 60°C for 1 hour. Then, distilled water was added to the mixture, the mixture was moved to a liquid-separating funnel, and an organic layer was extracted using chloroform. In this way, 0.42 g of a product (compound g, yield: 92%) was obtained.

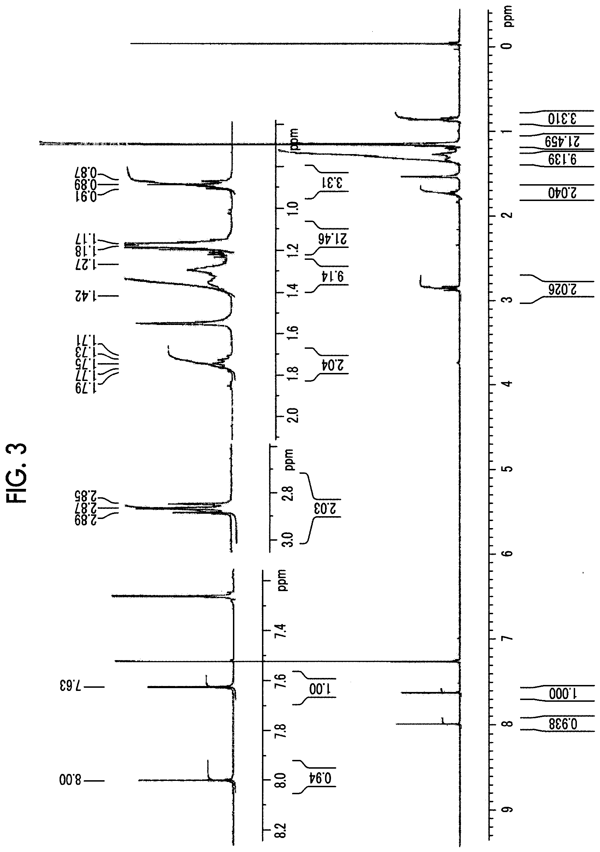

The structure of the compound g was identified by ¹H-NMR. The result is shown in Fig. 3.

### Synthesis of compound h;

In a nitrogen atmosphere, a toluene solution containing 0.42 g of the compound g and 0.038 g of platinum (II) chloride was heated to 90°C for 12 hours. Then, the distilled water was added to the mixture, the mixture was moved to a liquid-separating funnel, and an organic layer was extracted using chloroform. The obtained solid was separated by silica gel chromatography (SiO₂; hexane:dichloromethane = 9:1). In this way, 0.20 g of a product (compound h, yield: 47%) was obtained.

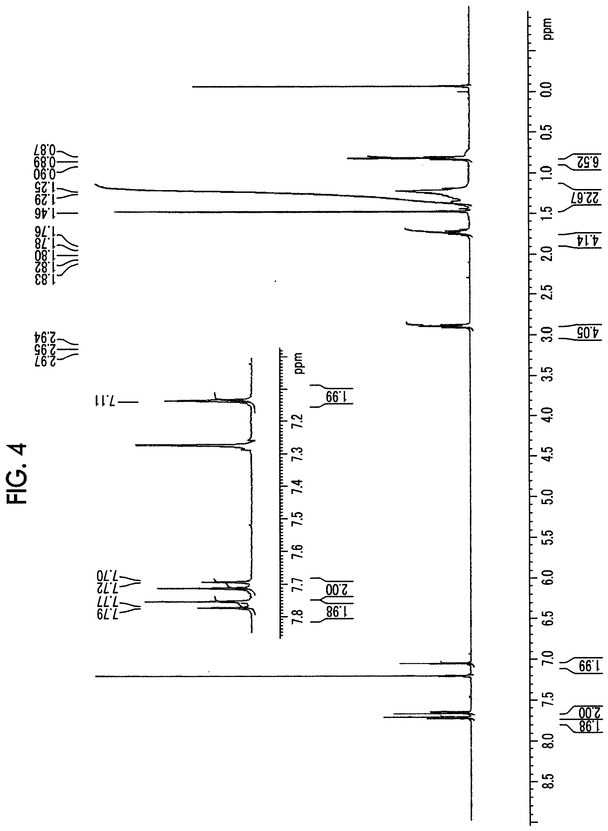

The compound h in which R represents a n-octyl group was denoted as a compound 1. The structure of the compound 1 was identified by ¹H-NMR. The result is shown in Fig. 4.

The compounds represented by Formula (1-1) other than the compound 1 were synthesized in the same manner as used for synthesizing the compound 1. That is, through the compound g, the compounds 3, 5, 29, 37, 39, 43, and 51 represented by Formula (1-1) were synthesized according to the scheme 1 described above.

Each of the obtained compounds represented by Formula (1-1) was identified by elementary analysis, NMR, and MASS spectrometry.

### <Synthesis example 2> Synthesis of compounds 2, 4, 30, 38, 40, and 44 and intermediate compound 2g

According to a specific synthesis procedure shown in the following scheme 2, compounds 2, 4, 30, 38, 40, and 44 as the compound represented by Formula (1-2) and an intermediate compound 2g were synthesized.

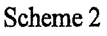

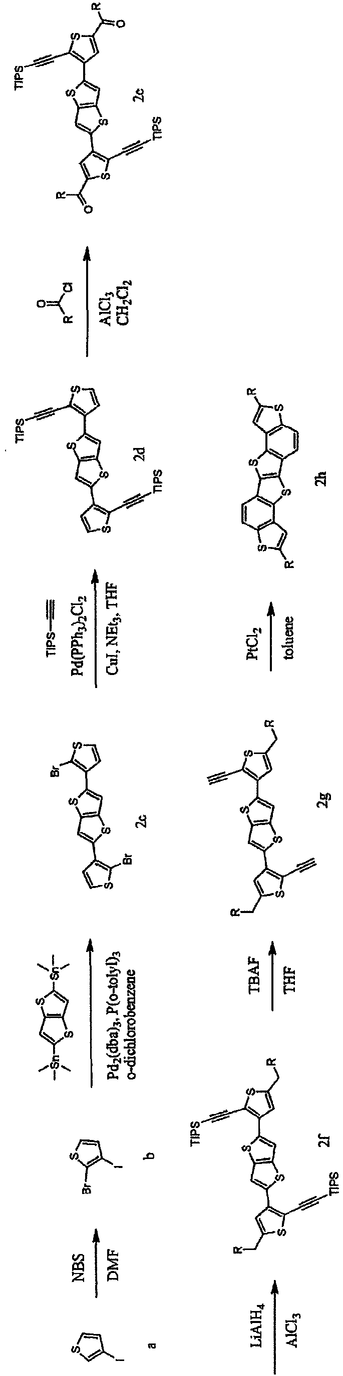

A process will be specifically described in which an intermediate compound 2g represented by Formula (3-2) and then a compound 2h represented by Formula (1-2) are synthesized from the compound b described above. In the following synthesis example, a case where R in the scheme 2 is a n-octyl group, that is, a case where the compound 2h is the compound 2 will be specifically described.

### Synthesis of compound 2c;

In a nitrogen atmosphere, 300 mL of a dimethylacetamide solution containing 8.60 g of the compound b, 7.00 g of 2,5-bis(trimethylstannyl)thieno[3,2-b]thiophene, and 1.05 g of bis(triphenylphosphine)palladium (II) dichloride was heated to 60°C. Then, distilled water was added to the mixture, the mixture was moved to a liquid-separating funnel, and an organic layer was extracted using ethyl acetate. Subsequently, the organic layer was dried over sodium sulfate and concentrated under reduced pressure. Thereafter, the obtained residue was separated by silica gel chromatography (SiO₂; hexane:dichloromethane = 100:5). In this way, 3.00 g of a product (compound 2c, yield: 43%) was obtained.

### Synthesis of compound 2d;

In a nitrogen atmosphere, a mixed solution of 3.00 g of the compound 2c, 3.19 mL of triisopropylsilyl acetylene, 0.36 g of bis(triphenylphosphine)palladium (II) dichloride, 0.99 g of copper iodide, 40 mL of triethylamine, and 15 mL of THF was stirred for 12 hours at room temperature. Then, distilled water was added to the mixture, the mixture was moved to a liquid-separating funnel, and an organic layer was extracted using hexane. Subsequently, the organic layer was dried over sodium sulfate and concentrated under reduced pressure. Thereafter, the obtained residue was separated by silica gel chromatography (SiO₂; hexane). In this way, 3.10 g of a product (compound 2d, yield: 71%) was obtained.

### Synthesis of compound 2e;

In a nitrogen atmosphere, 1.76 g of n-octanoyl chloride was added to 45 mL of a dichloromethane solution containing 3.00 g of the compound 2d and 2.86 g of aluminum chloride at room temperature, followed by stirring at room temperature. After 4 hours, methanol, water, and dilute hydrochloric acid (1 N) were added thereto in this order so as to stop the reaction, and the precipitated solid was separated by filtration. The obtained solid was separated by silica gel chromatography (SiO₂; hexane:toluene = 5:1). In this way, 2.48 g of a product (compound 2e, yield: 60%) was obtained.

### Synthesis of compound 2f;

In a nitrogen atmosphere, 1.66 g of lithium aluminum hydride was added to 80 mL of a tetrahydrofuran solution containing 2.00 g of the compound 2e and 1.29 g of aluminum chloride, followed by stirring for 6 hours at room temperature. Then, methanol, water, and dilute hydrochloric acid (1 N) were added thereto in this order so as to stop the reaction, and the precipitated solid was separated by filtration. The obtained solid was separated by silica gel chromatography (SiO₂; hexane:toluene = 10:1). In this way, 1.71 g of a product (compound 2f, yield: 88%) was obtained.

### Synthesis of compound 2g;

1.50 g of the compound 2f was added to 30 mL of a tetrahydrofuran solution containing tetra-n-butylammonium fluoride, and the resulting mixture was heated to 60°C for 1 hour. Then, distilled water was added to the mixture, the mixture was moved to a liquid-separating funnel, and an organic layer was extracted using chloroform. In this way, 0.89 g of a product (compound 2g, yield: 92%) was obtained.

### Synthesis of compound 2h;

In a nitrogen atmosphere, a toluene solution containing 0.80 g of the compound g and 0.073 g of platinum (II) chloride was heated to 90°C for 12 hours. Then, distilled water was added to the mixture, the mixture was moved to a liquid-separating funnel, and an organic layer was extracted using chloroform. The obtained solid was separated by silica gel chromatography (SiO₂; hexane:dichloromethane = 9:1). In this way, 0.32 g of a product (compound 2h, yield: 40%) was obtained.

The compound 2h in which R represents a n-octyl group was denoted as a compound 2.

The compounds represented by Formula (1-2) other than the compound 2 were synthesized in the same manner as used for synthesizing the compound 2. That is, through the compound 2g, the compounds 4, 30, 38, 40, and 44 represented by Formula (1-2) were synthesized according to the scheme 2 described above.

Each of the obtained compounds represented by Formula (1-2) was identified by elementary analysis, NMR, and MASS spectrometry.

### <Synthesis of comparative compounds>

The structures of comparative compounds 1 to 4 used in the semiconductor active layer (organic semiconductor layer) of comparative elements are shown below.

The comparative compound 3 is Bis-InBTBT (16) described in WO2013/078407A, and the comparative compounds 1 and 3 were synthesized according to the method described in WO2013/078407A.

The comparative compound 4 is a compound 5 used in Example 1 of US2008/0142792A, and the comparative compounds 2 and 4 were synthesized as described in US2008/0142792A.

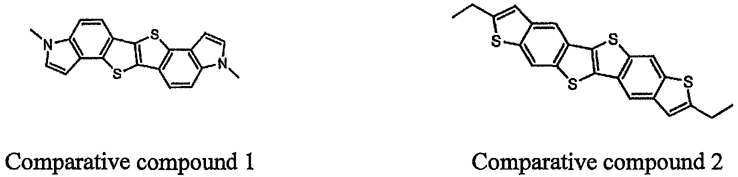

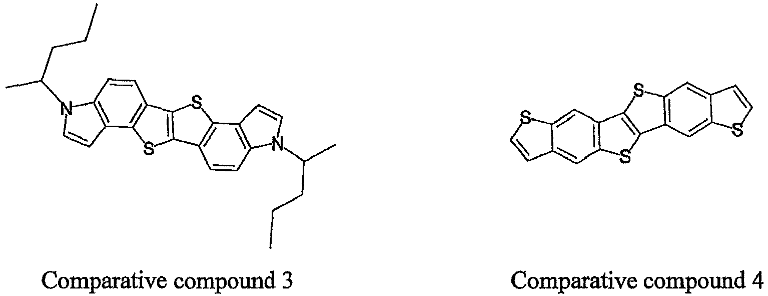

### [Example 2]

### <Preparation/evaluation of element>

All of the materials used for preparing elements were purified by sublimation. Through high-performance liquid chromatography (TOSOH CORPORATION, TSKgel ODS-100Z), it was confirmed that the materials had purity (area ratio for absorption intensity at 254 nm) of equal to or higher than 99.5%.

### <Formation of semiconductor active layer (organic semiconductor layer) by using compound alone>

Each of the compounds of the present invention or the comparative compounds (1 mg each) was mixed with toluene (1 mL) and heated to 100°C, thereby obtaining a coating solution for a non-light-emitting organic semiconductor device. In a nitrogen atmosphere, the coating solution was cast onto a substrate for measuring FET characteristics that was heated to 90°C, thereby forming an organic semiconductor film for a non-light-emitting organic semiconductor device. In this way, an organic transistor element of Example 2 for measuring FET characteristics was obtained. As the substrate for measuring FET characteristics, a silicon substrate comprising a bottom gate/bottom contact structure was used which included chromium/gold (gate width W = 100 mm, gate length L = 100 µm) arranged to form a comb pattern as source and drain electrodes and included SiO₂ (film thickness: 200 nm) as an insulating layer (the structure is schematically shown in Fig. 2).

### [Evaluation]

By using a semiconductor parameter analyzer (4156C manufactured by Agilent Technologies) connected to a semi-automatic prober (AX-2000 manufactured by Vector Semiconductor Co., Ltd.), the FET characteristics of the organic transistor element of Example 2 were evaluated under a normal pressure/nitrogen atmosphere, from the viewpoint of the carrier mobility and the threshold voltage shift after repeated driving.

The obtained results are shown in the following Table 1.

### (a) Carrier mobility

Between the source electrode and the drain electrode of each organic film transistor element (FET element), a voltage of -80 V was applied, and the gate voltage was varied within a range of 20 V to -100 V. In this way, a carrier mobility µ was calculated using the following equation showing a drain current I_{d}. ${I}_{d} = \left(w/2L\right) {μC}_{i} {\left({V}_{g}-{V}_{th}\right)}^{2}$

In the equation, L represents a gate length, W represents a gate width, Cᵢ represents a capacity of the insulating layer per unit area, V_{g} represents a gate voltage, and Vₜₕ represents a threshold voltage.

Herein, because the characteristics of the element having a carrier mobility of less than 1 × 10⁻⁵ cm²/Vs were too poor, the element was not subjected to the evaluation of (b) threshold voltage shift after repeated driving described below.

### (b) Threshold voltage shift after repeated driving

Between the source electrode and the drain electrode of each organic transistor element (FET element), a voltage of -80 V was applied, and the element was repeatedly driven 100 times by varying the gate voltage within a range of +20 V to -100 V. In this way, the element was measured in the same manner as in the section (a), and a difference between a threshold voltage V_{before} before the repeated driving and a threshold voltage V_{after} after the repeated driving (| V_{after} - V_{before} |) was evaluated into 3 levels as below. The smaller the difference, the higher the stability of the element against repeated driving. Therefore, the smaller the difference, the more preferable.
A: | V_{after} - V_{before} | ≤ 5 V
B: 5 V <| V_{after} - V_{before} | ≤ 10 V
C: | V_{after} - V_{before} | > 10 V

### (c) Solubility

Each of the compounds of the present invention or the comparative examples (20 mg each) was mixed with toluene (1 mL), and the solubility was evaluated into 2 levels as shown below.
A: Completely dissolved.
B: Incompletely dissolved.

**[Table 1]**

| Element No. | Organic semiconductor material | Carrier mobility (cm²/Vs) | Threshold voltage shift after repeated driving | Solubility | Note |
|---|---|---|---|---|---|
| Element 1 | Compound 1 | 0.49 | A | A | Present invention |
| Element 2 | Compound 2 | 0.32 | A | A | Present invention |
| Element 3 | Compound 3 | 0.25 | A | A | Present invention |
| Element 4 | Compound 4 | 0.16 | A | A | Present invention |
| Element 5 | Compound 5 | 7.9 × 10⁻² | A | A | Present invention |
| Element 6 | Compound 29 | 1.5 × 10⁻² | A | A | Present invention |
| Element 7 | Compound 30 | 1.5 × 10⁻² | A | A | Present invention |
| Element 8 | Compound 37 | 8.7 × 10⁻³ | A | A | Present invention |
| Element 9 | Compound 38 | 5.0 × 10⁻³ | A | A | Present invention |
| Element 10 | Compound 39 | 4.4 × 10⁻³ | A | A | Present invention |
| Element 11 | Compound 40 | 4.2 × 10⁻³ | A | A | Present invention |
| Element 12 | Compound 43 | 4.0 × 10⁻³ | A | A | Present invention |
| Element 13 | Compound 44 | 3.8 × 10⁻³ | A | A | Present invention |
| Element 14 | Compound 51 | 3.1 × 10⁻³ | A | A | Present invention |
| Comparative element 1 | Comparative compound 1 | 4.4 × 10⁻⁴ | C | B | Comparative example |
| Comparative element 2 | Comparative compound 2 | 8.4 × 10⁻⁵ | B | B | Comparative example |
| Comparative element 3 | Comparative compound 3 | 1.2 × 10⁻⁶ | C | B | Comparative example |
| Comparative element 4 | Comparative compound 4 | 4.8 × 10⁻⁵ | B | B | Comparative example |

As is evident from the above Table 1, the compound of the present invention exhibits excellent solubility in an organic solvent. Furthermore, it was understood that the organic transistor element using the compound of the present invention has high carrier mobility. Therefore, it was understood that the compound of the present invention can be preferably used as an organic semiconductor material for a non-light-emitting organic semiconductor device.

In contrast, the organic transistor elements using the comparative compounds 1 to 4 had low carrier mobility.

Herein, the organic transistor element using the compound of the present invention showed only a slight threshold voltage shift after repeated driving, and the compound of the present invention exhibited high solubility in an organic solvent.

### [Example 3]

### <Formation of semiconductor active layer (organic semiconductor layer) by using compound and binder together>

Each of the compounds of the present invention or the comparative compounds (1 mg each) was mixed with 1 mg of PαMS (poly(α-methylstyrene, Mw = 300,000), manufactured by Sigma-Aldrich Co, LLC.) and toluene (1 mL), and the mixture was heated to 100°C, thereby obtaining a coating solution. Organic transistor elements for measuring FET characteristics were prepared and evaluated in the same manner as in Example 2, except that the coating solution obtained as above was used.

The obtained results are shown in the following Table 2.

**[Table 2]**

| Element No. | Organic semiconductor material | Carrier mobility (cm²/Vs) | Threshold voltage shift after repeated driving | Note |
|---|---|---|---|---|
| Element 15 | Compound 1 | 0.63 | A | Present invention |
| Element 16 | Compound 2 | 0.42 | A | Present invention |
| Element 17 | Compound 3 | 0.32 | A | Present invention |
| Element 18 | Compound 4 | 0.25 | A | Present invention |
| Element 19 | Compound 5 | 8.6 × 10⁻² | A | Present invention |
| Element 20 | Compound 29 | 2.5 × 10⁻² | A | Present invention |
| Element 21 | Compound 30 | 2.0 × 10⁻² | A | Present invention |
| Comparative element 5 | Comparative compound 1 | 6.3 × 10⁻⁴ | C | Comparative example |
| Comparative element 6 | Comparative compound 2 | 1.0 × 10⁻⁴ | B | Comparative example |

From the above Table 2, it was understood that the organic transistor element including a semiconductor active layer formed using the compound of the present invention with a binder has high carrier mobility. Therefore, it was understood that the compound of the present invention can be preferably used as an organic semiconductor material for a non-light-emitting organic semiconductor device.

In contrast, the organic transistor element including a semiconductor active layer formed using the comparative compounds 1 and 2 with a binder had low carrier mobility.

Herein, the organic transistor element using the compound of the present invention showed only a slight threshold voltage shift after repeated driving.

Each of the organic transistor elements obtained in Example 3 was observed by unaided eyes and an optical microscope. As a result, it was understood that all of the films using PαMS as a binder have extremely high smoothness and uniformity.

From the above results, it was understood that in a case where the semiconductor active layer for the comparative element is formed using a binder and a comparative compound in combination, the carrier mobility becomes extremely low; however, in a case where the semiconductor active layer for the organic transistor element of the present invention is formed using the compound of the present invention with a binder, excellent carrier mobility is exhibited, and preferably, it is possible to obtain an element which shows only a slight threshold voltage shift after repeated driving and has extremely high smoothness/uniformity of the film.

### [Example 4]

### <Formation of semiconductor active layer (organic semiconductor layer)>

The surface of a silicon wafer, which comprised SiO₂ (film thickness: 370 nm) as a gate insulating film, was treated with octyltrichlorosilane.

Each of the compounds of the present invention or the comparative compounds (1 mg each) was mixed with toluene (1 mL), and the mixture was heated to 100°C, thereby preparing a coating solution for a non-light-emitting organic semiconductor device. In a nitrogen atmosphere, the coating solution was cast onto the silicon wafer which had been heated to 90°C and undergone surface treatment with octylsilane, thereby forming an organic semiconductor film for a non-light-emitting organic semiconductor device.

Furthermore, gold was deposited onto the surface of the film by using a mask so as to prepare source and drain electrodes, thereby obtaining an organic transistor element having a bottom gate/top contact structure with a gate width W = 5 mm and a gate length L = 80 µm (the structure is schematically shown in Fig. 1).

By using a semiconductor parameter analyzer (4156C manufactured by Agilent Technologies) connected to a semi-automatic prober (AX-2000 manufactured by Vector Semiconductor Co., Ltd.), the FET characteristics of the organic transistor element of Example 4 were evaluated in the same manner as in Example 2 under a normal pressure/nitrogen atmosphere.

The obtained results are shown in the following Table 3.

**[Table 3]**

| Element No. | Organic semiconductor material | Carrier mobility (cm²/Vs) | Threshold voltage shift after repeated driving | Note |
|---|---|---|---|---|
| Element 22 | Compound 1 | 0.64 | A | Present invention |
| Element 23 | Compound 2 | 0.44 | A | Present invention |
| Element 24 | Compound 5 | 9.1 × 10⁻² | A | Present invention |
| Element 25 | Compound 29 | 3.3 × 10⁻² | A | Present invention |
| Element 26 | Compound 30 | 2.4 × 10⁻² | A | Present invention |
| Comparative element 7 | Comparative compound 1 | 4.8 × 10⁻⁵ | C | Comparative example |

From the above Table 3, it was understood that the organic transistor element using the compound of the present invention has high carrier mobility. Therefore, it was understood that the compound of the present invention can be preferably used as an organic semiconductor material for a non-light-emitting organic semiconductor device.

In contrast, the organic transistor element using the comparative compound 1 had low carrier mobility.

Herein, the organic transistor element using the compound of the present invention showed only a slight threshold voltage shift after repeated driving.

### Explanation of References

11: substrate
12: electrode
13: insulator layer
14: semiconductor active layer (organic substance layer, organic semiconductor layer)
15a, 15b: electrode
31: substrate
32: electrode
33: insulator layer
34a, 34b: electrode
35: semiconductor active layer (organic substance layer, organic semiconductor layer)

## Claims

1. A compound represented by the following Formula (1-1), (1-2), (3-1) or (3-2); 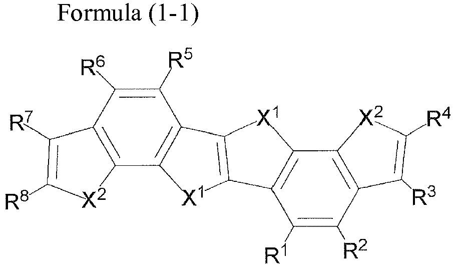 in Formula (1-1),
each X¹ independently represents a sulfur atom, an oxygen atom, a selenium atom, or NR⁹,
each X² independently represents a sulfur atom, an oxygen atom, or a selenium atom,
each of R¹ to R⁹ independently represents a hydrogen atom or a substituent, one or two of R¹, R², R³, R⁴, R⁵, R⁶, R⁷, R⁸, and R⁹ is a substituent represented by the following Formula (W) and at least one of R⁴ and R⁸ is a substituent represented by the Formula (W); 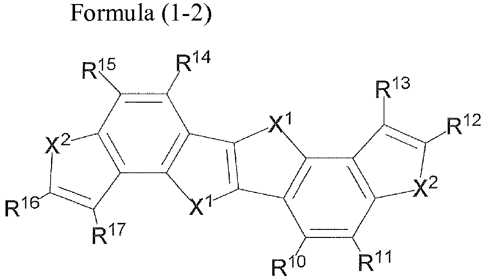
in Formula (1-2),
each X¹ independently represents a sulfur atom, an oxygen atom, a selenium atom, or NR⁹,
each X² independently represents a sulfur atom, an oxygen atom, or a selenium atom,
each of R⁹ R¹⁰ to R¹⁷ independently represents a hydrogen atom or a substituent, and at least one of R⁹, R¹⁰, R ¹¹, R¹², R¹³, R¹⁴, R¹⁵, R¹⁶, and R¹⁷ is a substituent represented by the following Formula (W);
wherein the substituent of R¹ to R¹⁷ is a group selected from a halogen atom, an alkyl group having 1 to 40 carbon atoms, an alkenyl group, an alkynyl group, a trimethylsilylethynyl group, a triethylsilylethynyl group, a tri-i-propylsilylethynyl group, an aryl group having 6 to 20 carbon atoms, a hetero ring group, a cyano group, a hydroxyl group, a nitro group, an acyl group, an alkoxy group, an aryloxy group, a silyloxy group, a heterocyclic oxy group, an acyloxy group, a carbamoyloxy group, an amino group, an acylamino group, an aminocarbonylamino group, alkoxy- and aryloxycarbonylamino groups, alkyl- and aryl sulfonylamino groups, a mercapto group, alkyl- and arylthio groups, a heterocyclic thio group, a sulfamoyl group a sulfo group, alkyl- and aryl sulfinyl groups, alkyl- and aryl sulfonyl groups, alkoxyloxy- and aryloxycarbonyl groups, a carbamoyl group, aryl- and heterocyclic azo group, an imide group, a phosphino group, a phosphinyl group, a phosphinyloxy group, a phosphinylamino group, a phosphono group, a silyl group, a hydrazino group, a ureide group, a boronic acid group (-B(OH)₂), a phosphate group (-OPO(OH)₂), a sulfate group (-OSO₃H), wherein these substituents may further have the above substituents,
-L-R Formula (W)
in Formula (W), L represents a divalent linking group represented by any of the following Formulae (L-2) to (L-25), and
R represents a substituted or unsubstituted alkyl group, a cyano group, a vinyl group, an ethynyl group, an oxyethylene group, an oligo-oxyethylene group in which a repetition number v of an oxyethylene unit is equal to or greater than 2, a siloxane group, an oligosiloxane group having two or more silicon atoms, or a substituted or unsubstituted trialkylsilyl group;
wherein the total number of carbon atoms contained in L and R is 5 to 12; 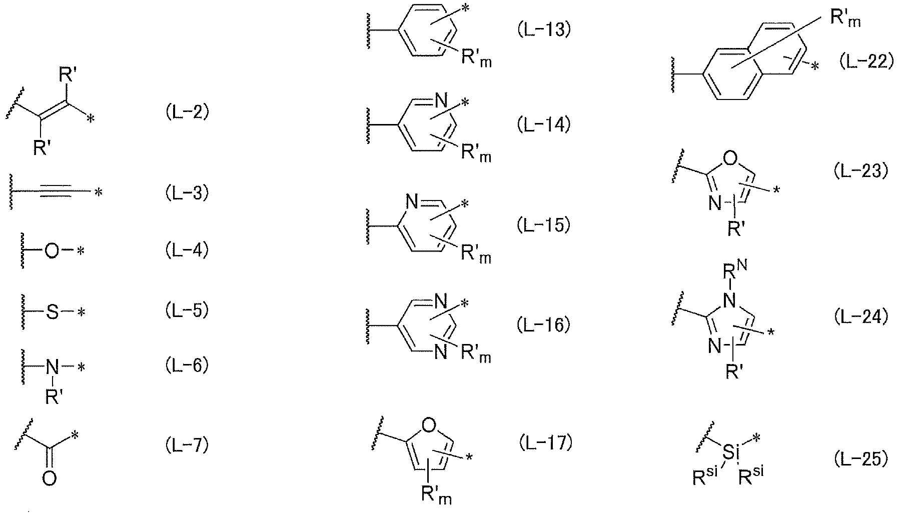 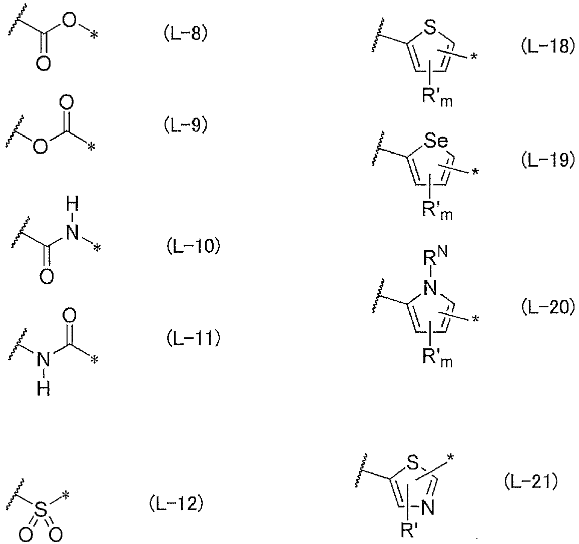
in Formulae (L-2) to (L-25),
the portion of a wavy line represents a position of bonding to a condensed cyclic skeleton,
m in Formula (L-13) represents 4, m in Formulae (L-14) and (L-15) represents 3, m in Formulae (L-16) to (L-20) represents 2, m in Formula (L-22) represents 6,
each R' in Formulae (L-2), (L-6), and (L-13) to (L-24) independently represents a hydrogen atom or a substituent,
R^{N} represents a hydrogen atom or a substituent, and
each R^{si} independently represents a hydrogen atom, an alkyl group, an alkenyl group, or an alkynyl group, 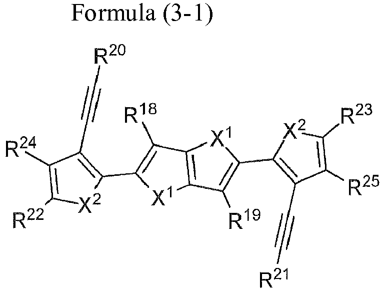
in Formula (3-1),
X¹ and X² have the same meaning as defined above,
each of R⁹ and R¹⁸ to R²⁵ independently represents a hydrogen atom or a substituent, one or two of R⁹, R¹⁸, R¹⁹, R²⁰, R²¹, R²², R²³, R²⁴, and R²⁵ is a substituent represented by the Formula (W) as defined above and at least one of R²² and R²³ represents an alkyl group, an alkenyl group, an alkynyl group, a substituted or unsubstituted aryl group, a substituted or unsubstituted heteroaryl group, an alkylcarbonyl group, or a substituted or unsubstituted arylcarbonyl group; 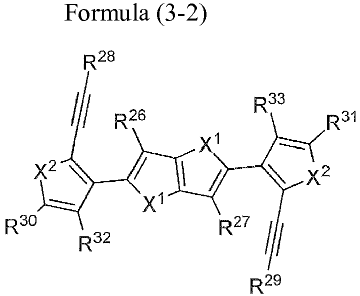
in Formula (3-2),
each X¹ independently represents a sulfur atom, an oxygen atom, a selenium atom, or NR⁹,
each X² independently represents a sulfur atom, an oxygen atom, or a selenium atom,
each of R⁹ and R²⁶ to R³³ independently represents a hydrogen atom or a substituent, and at least one of R³⁰ and R³¹ represents an alkyl group, an alkenyl group, an alkynyl group, a substituted or unsubstituted aryl group, a substituted or unsubstituted heteroaryl group, an alkylcarbonyl group, or a substituted or unsubstituted arylcarbonyl group.

2. The compound according to claim 1,
wherein the compound represented by the Formula (1-1) or (1-2) is a compound represented by any of the following Formulae (2-1-1), (2-1-2), (2-2-1) or (2-2-2); 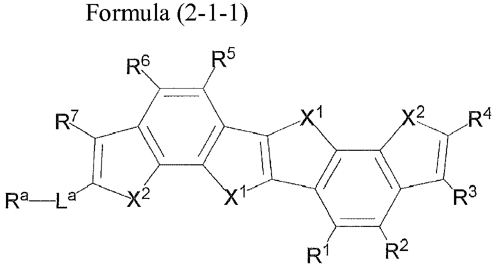 in Formula (2-1-1),
each X¹, X², R¹ to R⁷ and R⁹ are as defined in claim 1, and when R¹, R², R³, R⁴, R⁵, R⁶, R⁷ or R⁹ is a substituent, then the substituent is a substituent other than the substituents represented by the Formula (W), R⁴ is not a group represented by -L^{a}-R^{a},
L^{a} and R^{a} have the same meaning as L and R as defined in claim 1; 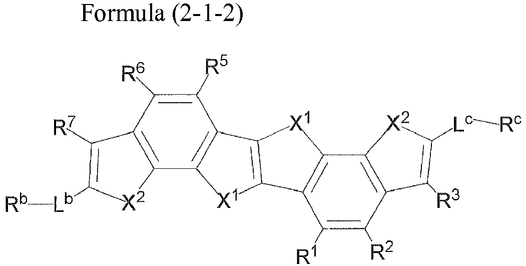
in Formula (2-1-2),
X¹, X² and R¹ to R³, R⁵ to R⁷, and R⁹ have the same meaning as defined in claim 1, and when R¹, R², R³, R⁵, R⁶, R⁷ or R⁹ is a substituent, then the substituent is a substituent other than the substituents represented by the Formula (W),
L^{b}, L^{c} R^{b} and R^{c} have the same meaning as L and R as defined in claim 1, 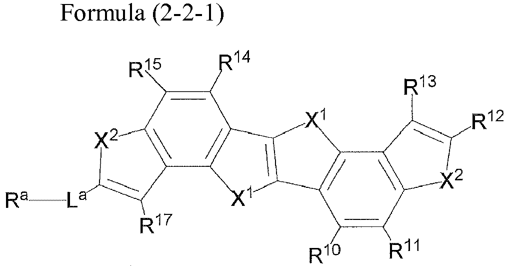
in Formula (2-2-1),
X¹ and X² have the same meaning as defined in claim 1,
each of R¹⁰, R¹² to R¹⁵, and R¹⁷ independently represents a hydrogen atom or a substituent, R¹² is not a group represented by -L^{a}-R^{a},
L^{a} and R^{a} have the same meaning as L and R as defined in claim 1; 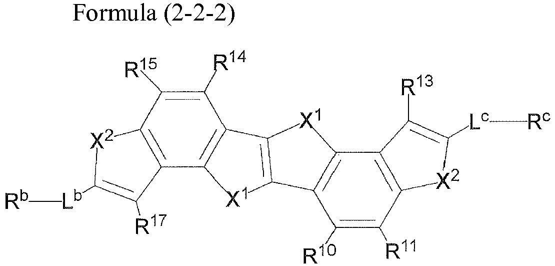
in Formula (2-2-2),
X¹ and X² have the same meaning as defined in claim 1,
each of R¹⁰, R¹¹, R¹³ to R¹⁵, and R¹⁷ independently represents a hydrogen atom or a substituent,
L^{b} and L^{c}, R^{b} and R^{c} have the same meaning as L and R as defined in claim 1.

3. The compound according to claim 1 or 2,
wherein in the Formulae (1-2), (1-1), (2-1-1), (2-1-2), (2-2-1), and (2-2-2), each of L, L^{a}, L^{b}, and L^{c} is independently a divalent linking group represented by any of the Formulae (L-2) to (L-5), (L-13), (L-17), and (L-18).

4. The compound according to any one of claims 1 to 3,
wherein in the Formulae (1-2), (1-1), (2-1-1), (2-1-2), (2-2-1), and (2-2-2), each of L, L^{a}, L^{b}, and L^{c} is a divalent linking group represented by any of the Formulae (L-3), (L-13), and (L-18).

5. The compound according to any one of the claims 1 to 4, wherein the substituents of each of R¹ to R³ in formula (1-1) and each of R¹⁰ to R¹⁷ in formula (1-2) are an alkyl group having 1 to 18 carbon atoms, an aryl group having 6 to 20 carbon atoms, an alkenyl group having 2 to 12 carbon atoms, an alkynyl group having 2 to 12 carbon atoms, an alkoxy group having 1 to 11 carbon atoms, a heterocyclic group having 5 to 12 carbon atoms, an alkyl thio group having 1 to 12 carbon atoms or the group of formula (W).

6. An organic transistor or an organic semiconductor material for a non-light emitting organic semiconductor device or an organic semiconductor film for a non-light emitting organic semiconductor device, each comprising a compound represented by the Formula (1-1) or (1-2) as defined in any one of the claims 1 to 5, wherein in the organic transistor said compound is comprised in a semiconductor active layer.

7. A use for an organic transistor of a compound represented by the Formula (1-1) or (1-2) as defined in any one of the claims 1 to 5.

8. A coating solution for a non-light-emitting organic semiconductor device, comprising a compound represented by the Formula (1-1) or (1-2) as defined in any one of the claims 1 to 5.

## Patentansprüche

1. Verbindung mit der folgenden Formel (1-1), (2-2), (3-1) oder (3-2); 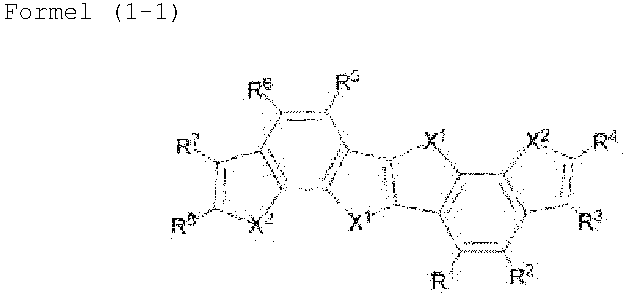
worin in der Formel (1-1) jedes X¹ unabhängig ein Schwefelatom, Sauerstoffatom, Selenatom oder NR⁹ ist,
jedes X² unabhängig ein Schwefelatom, Sauerstoffatom oder Selenatom ist,
jedes von R¹ bis R⁹ unabhängig ein Wasserstoffatom oder ein Substituent ist, ein oder zwei von R¹, R², R³, R⁴, R⁵, R⁶, R⁷, R⁸ und R⁹ ein Substituent ist, dargestellt durch die folgende Formel (W) und zumindest eines von R⁴ und R⁸ ein Substituent mit der Formel (W) ist; 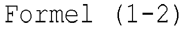 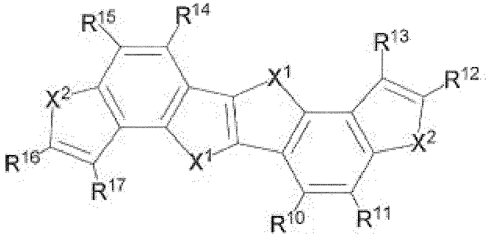
worin in der Formel (1-2) jedes X¹ unabhängig ein Schwefelatom, Sauerstoffatom, Selenatom oder NR⁹ ist,
jedes X² unabhängig ein Schwefelatom, Sauerstoffatom oder Selenatom ist,
jedes von R⁹, R¹⁰ bis R¹⁷ unabhängig ein Wasserstoffatom oder ein Substituent sind und zumindest eines von R⁹, R¹⁰, R¹¹, R¹², R¹³, R¹⁴, R¹⁵, R¹⁶ und R¹⁷ ein Substituent mit der folgenden Formel (W) ist,
worin der Substituent von R¹ bis R¹⁷ eine Gruppe ist, ausgewählt aus einem Halogenatom, einer Alkylgruppe mit 1 bis 40 Kohlenstoffatomen, Alkenylgruppe, Alkinylgruppe, Trimethylsilylethinylgruppe, Triethylsilylethinylgruppe, Tri-i-propylsilylethinylgruppe, Arylgruppe mit 6 bis 20 Kohlenstoffatomen, Heteroringgruppe, Cyanogruppe, Hydroxylgruppe, Nitrogruppe, Acylgruppe, Alkoxygruppe, Aryloxygruppe, Silyloxygruppe, heterocyclischen Oxygruppe, Acyloxygruppe, Carbamoyloxygruppe, Aminogruppe, Acylaminogruppe, Aminocarbonylaminogruppe, Alkoxy- und Aryloxycarbonylaminogruppen, Alkyl- und Arylsulfonylaminogruppen, Mercaptogruppe, Alkyl- und Arylthiogruppen, heterocyclischen Thiogruppe, Sulfamoxylgruppe, Sulfogruppe, Alkyl- und Arylsulfinylgruppen, Alkyl- und Arylsulfonylgruppen, Alkoxyloxy- und Aryloxycarbonylgruppen, Carbamoylgruppe, Aryl- und heterocyclischen Azogruppe, Imidgruppe, Phosphingruppe, Phosphinylgruppe, Phosphinyloxygruppe, Phosphinylaminogruppe, Phosphongruppe, Silylgruppe, Hydrazinogruppe, Ureidgruppe, Borsäuregruppe (-B(OH)₂), Phosphatgruppe (-OPO(OH)2), Sulfatgruppe (-OSO₃H), worin diese Substituenten weiterhin die obigen Substituenten haben können,
-L-R Formel (W)
worin in der Formel (W) L eine bivalente Bindegruppe ist, dargestellt durch eine der folgenden Formeln (L-2) bis (L-25), und
R eine substituierte oder unsubstituierte Alkylgruppe, Cyanogruppe, Vinylgruppe, Ethinylgruppe, Oxyethylengruppe, Oligooxiethylengruppe, worin eine Wiederholungszahl v einer Oxyethyleneinheit gleich oder größer als 2 ist, Siloxangruppe, Oligosiloxangruppe mit zwei oder mehr Siliciumatomen oder eine substituierte oder unsubstituierte Trialkylsilylgruppe ist,
worin die gesamte Zahl der Kohlenstoffatome, die in L und R enthalten sind, 5 bis 12 ist, 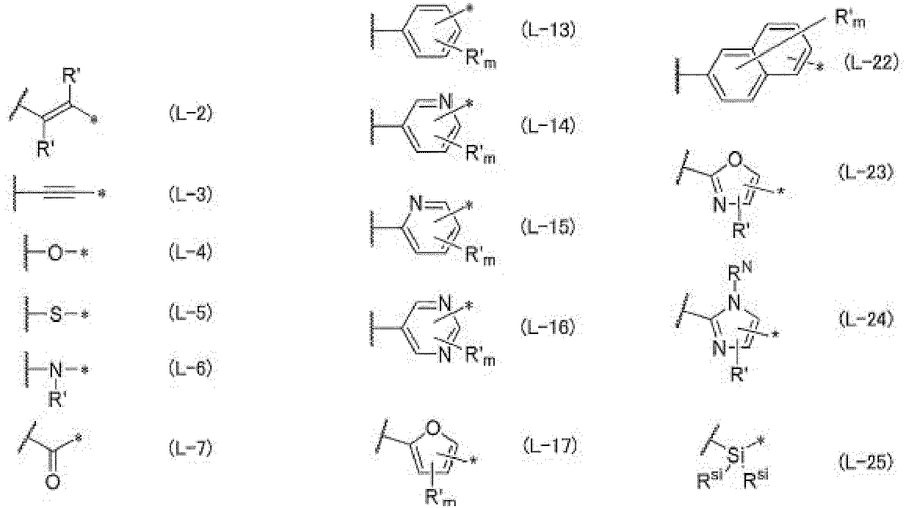 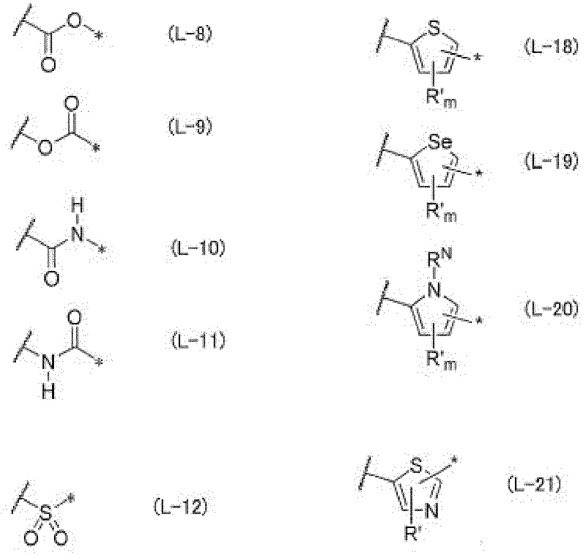
worin in den Formeln (L-2) bis (L-25) der Bereich einer Wellenlinie eine Position der Bindung an ein kondensiertes cyclisches Gerüst ist,
m in der Formel (L-13) 4 ist, m in den Formeln (L-14) und (L-15) 3 ist, m in den Formeln (L-16) bis (L-20) 2 ist, m in der Formel (L-22) 6 ist,
jedes R' in den Formeln (L-2), (L-6) und (L-13) bis (L-24) unabhängig ein Wasserstoffatom oder ein Substituent sind,
R^{N} ein Wasserstoffatom oder ein Substituent ist und
jedes R^{si} unabhängig ein Wasserstoffatom, eine Alkylgruppe, Alkenylgruppe oder Alkinylgruppe ist, 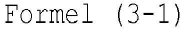 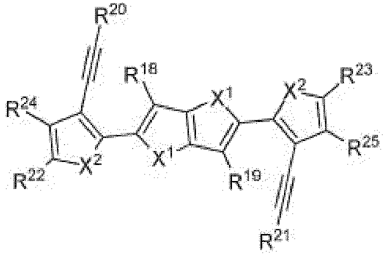
worin in der Formel (3-1) X¹ und X² die gleiche Bedeutung wie oben definiert aufweisen,
jedes von R⁹ und R¹⁸ bis R²⁵ unabhängig ein Wasserstoffatom oder ein Substituent sind, ein oder zwei von R⁹, R¹⁸, R¹⁹, R²⁰, R²¹, R²², R²³, R²⁴ und R²⁵ ein Substituent sind, dargestellt durch die Formel (W), wie oben definiert, und zumindest eines von R²² und R²³ eine Alkylgruppe, Alkenylgruppe, Alkinylgruppe, substituierte oder unsubstituierte Arylgruppe, substituierte oder unsubstituierte Heteroarylgruppe, Alkylcarbonylgruppe oder substituierte oder unsubstituierte Arylcarbonylgruppe sind, 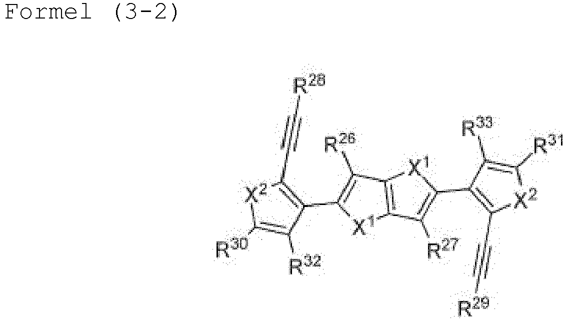
worin in der Formel (3-2) jedes X¹ unabhängig ein Schwefelatom, Sauerstoffatom, Selenatom oder NR⁹ ist,
jedes X² unabhängig ein Schwefelatom, Sauerstoffatom oder Selenatom ist,
jedes von R⁹ und R²⁶ bis R³³ unabhängig ein Wasserstoffatom oder ein Substituent sind, und zumindest eines von R³⁰ und R³¹ eine Alkylgruppe, Alkenylgruppe, Alkinylgruppe, substituierte oder unsubstituierte Arylgruppe, substituierte oder unsubstituierte Heteroarylgruppe, Alkylcarbonylgruppe oder substituierte oder unsubstituierte Arylcarbonylgruppe sind.

2. Verbindung gemäß Anspruch 1, worin die Verbindung mit der Formel (1-1) oder (1-2) eine Verbindung ist, die durch eine der folgenden Formeln (2-1-1), (2-1-2), (2-2-1) oder (2-2-2) dargestellt ist; 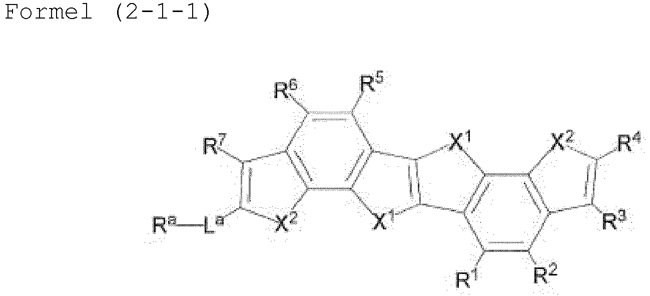
worin in der Formel (2-1-1) jedes X¹, X², R¹ bis R⁷ und R⁹ wie in Anspruch 1 definiert sind und wenn R¹, R², R³, R⁴, R⁵, R⁶, R⁷ oder R⁹ ein Substituent sind, der Substituent ein anderer Substituent ist als die Substituenten mit der Formel (W), R⁴ keine Gruppe ist, dargestellt durch -L^{a}-R^{a},
L^{a} und R^{a} die gleiche Bedeutung wie L und R wie in Anspruch 1 definiert aufweisen, 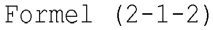 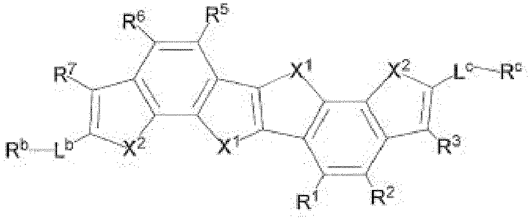
worin in der Formel (2-1-2) X¹, X² und R¹ bis R³, R⁵ bis R⁷ und R⁹ die gleiche Bedeutung, wie in Anspruch 1 definiert, aufweisen und wenn R¹, R², R³, R⁵, R⁶, R⁷ oder R⁹ ein Substituent ist, der Substituent ein anderer Substituent ist als die Substituenten, dargestellt durch die Formel (W),
L^{b}, L^{c}, R^{b} und R^{c} die gleiche Bedeutung wie L und R haben, wie in Anspruch 1 definiert, 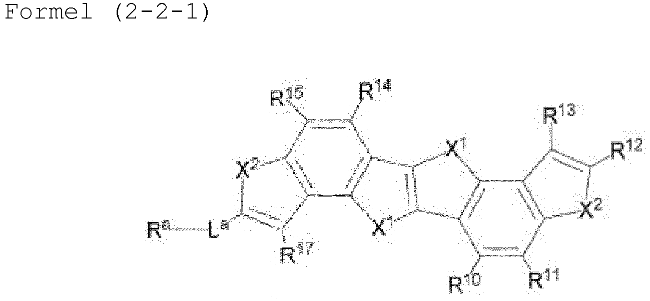
worin in der Formel (2-2-1) X¹ und X² die gleiche Bedeutung, wie in Anspruch 1 definiert, haben
jedes von R¹⁰, R¹² bis R¹⁵ und R¹⁷ unabhängig ein Wasserstoffatom oder ein Substituent sind, R¹² keine Gruppe ist, dargestellt durch -L^{a}-R^{a},
L^{a} und R^{a} die gleiche Bedeutung wie L und R haben, wie in Anspruch 1 definiert, 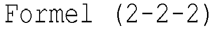 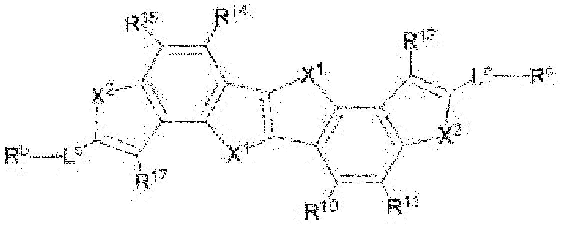
worin in der Formel (2-2-2) X¹ und X² die gleiche Bedeutung haben wie in Anspruch 1 definiert,
jedes von R¹⁰, R¹¹, R¹³ bis R¹⁵ und R¹⁷ unabhängig ein Wasserstoffatom oder ein Substituent sind,
L^{b} und L^{c}, R^{b} und R^{c} die gleiche Bedeutung wie L und R haben, wie in Anspruch 1 definiert.

3. Verbindung gemäß Anspruch 1 oder 2, worin den Formeln (1-2), (1-1), (2-1-1), (2-1-2), (2-2-1) und (2-2-2) jedes von L, L^{a}, L^{b} und L^{c} unabhängig eine bivalente Gruppe ist, dargestellt durch eine der Formeln (L-2) bis (L-5), (L-13), (L-17) und (L-18).

4. Verbindung gemäß einem der Ansprüche 1 bis 3, worin in den Formeln (1-2), (1-1), (2-1-1), (2-1-2), (2-2-1) und (2-2-2) jedes von L, L^{a}, L^{b} und L^{c} eine bivalente Bindegruppe ist, dargestellt durch eine der Formeln (L-3), (L-13) und (L-18).

5. Verbindung gemäß einem der Ansprüche 1 bis 4, worin die Substituenten von jedem von R¹ bis R³ in der Formel (1-1) und jedem von R¹⁰ bis R¹⁷ in der Formel (1-2) eine Alkylgruppe mit 1 bis 18 Kohlenstoffatomen, Arylgruppe mit 6 bis 20 Kohlenstoffatomen, Alkenylgruppe mit 2 bis 12 Kohlenstoffatomen, Alkinylgruppe mit 2 bis 12 Kohlenstoffatomen, Alkoxygruppe mit 1 bis 11 Kohlenstoffatomen, heterocyclische Gruppe mit 5 bis 12 Kohlenstoffatomen, Alkylthiogruppe mit 1 bis 12 Kohlenstoffatomen oder die Gruppe der Formel (W) sind.

6. Organischer Transistor oder organisches Halbleitermaterial für eine nicht-lichtemittierende organische Halbleitervorrichtung oder ein organischer Halbleiterfilm für eine nicht-lichtemittierende organische Halbleitervorrichtung, jeweils enthaltend eine Verbindung mit der Formel (1-1) oder (1-2), wie in einem der Ansprüche 1 bis 5 definiert, worin in dem organischen Transistor die Verbindung in einer Halbleiter-Aktivschicht enthalten ist.

7. Verwendung einer Verbindung mit der Formel (1-1) oder (1-2), wie in einem der Ansprüche 1 bis 5 definiert, für einen organischen Transistor.

8. Beschichtungslösung für eine nicht-lichtemittierende organische Halbleitervorrichtung, enthaltend eine Verbindung mit der Formel (1-1) oder (1-2), wie in einem der Ansprüche 1 bis 5 definiert.

## Revendications

1. Composé représenté par la formule (1-1), (1-2), (3-1) ou (3-2) suivante ; 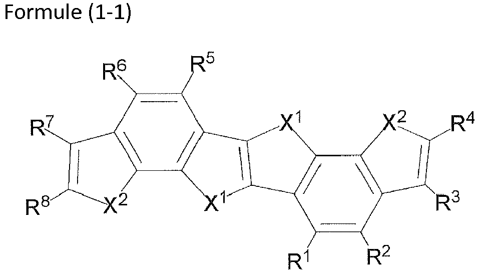 dans la formule (1-1),
chaque X¹ représente indépendamment un atome de soufre, un atome d'oxygène, un atome de sélénium ou NR⁹,
chaque X² représente indépendamment un atome de soufre, un atome d'oxygène ou un atome de sélénium,
chacun parmi R¹ à R⁹ représente indépendamment un atome d'hydrogène ou un substituant, un ou deux parmi R¹, R², R³, R⁴, R⁵, R⁶, R⁷, R⁸ et R⁹ sont un substituant représenté par la formule (W) suivante et au moins l'un parmi R⁴ et R⁸ est un substituant représenté par la formule (W) ; 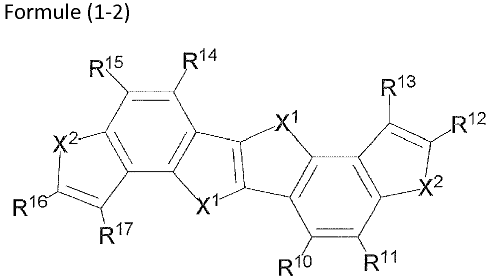
dans la formule (1-2),
chaque X¹ représente indépendamment un atome de soufre, un atome d'oxygène, un atome de sélénium ou NR⁹,
chaque X² représente indépendamment un atome de soufre, un atome d'oxygène ou un atome de sélénium,
chacun parmi R⁹ R¹⁰ à R¹⁷ représente indépendamment un atome d'hydrogène ou un substituant, et au moins l'un parmi R⁹, R¹⁰, R¹¹, R¹², R¹³, R¹⁴, R¹⁵, R¹⁶ et R¹⁷ est un substituant représenté par la formule (W) suivante ;
dans lequel le substituant de R¹ à R¹⁷ est un groupe sélectionné à partir d'un atome d'halogène, d'un groupe alkyle ayant 1 à 40 atomes de carbone, d'un groupe alcényle, d'un groupe alcynyle, d'un groupe triméthylsilyléthynyle, d'un groupe triéthylsilyléthynyle, d'un groupe tri-i-propylsilyléthynyle, d'un groupe aryle ayant 6 à 20 atomes de carbone, d'un groupe hétérocyclique, d'un groupe cyano, d'un groupe hydroxyle, d'un groupe nitro, d'un groupe acyle, d'un groupe alcoxy, d'un groupe aryloxy, d'un groupe silyloxy, d'un groupe oxy hétérocyclique, d'un groupe acyloxy, d'un groupe carbamoyloxy, d'un groupe amino, d'un groupe acylamino, d'un groupe aminocarbonylamino, des groupes alcoxy- et aryloxycarbonylamino, des groupes alkyl-et aryl-sulfonylamino, un groupe mercapto, des groupes alkyl- et aryl-thio, d'un groupe thio hétérocyclique, d'un groupe sulfamoyle un groupe sulfo, des groupes alkyl- et aryl-sulfinyle, des groupes alkyl- et aryl-sulfonyle, des groupes alcoxyloxy- et aryloxy-carbonyle, d'un groupe carbamoyle, d'un groupe aryl- et hétérocyclique azo, d'un groupe imide, d'un groupe phosphino, d'un groupe phosphinyle, d'un groupe phosphinyloxy, d'un groupe phosphinylamino, d'un groupe phosphono, d'un groupe silyle, d'un groupe hydrazino, d'un groupe uréide, d'un groupe acide boronique (-B(OH)₂), d'un groupe phosphate (-OPO(OH)₂), d'un groupe sulfate (-OSO₃H), dans lequel ces substituants peuvent en outre avoir les substituants ci-dessus,
-L-R Formule (W)
dans la formule (W), L représente un groupe de liaison divalent représenté par l'une quelconque des formules (L-2) à (L-25) suivantes, et
R représente un groupe alkyle substitué ou non substitué, un groupe cyano, un groupe vinyle, un groupe éthynyle, un groupe oxyéthylène, un groupe oligo-oxyéthylène dans lequel un nombre de répétitions v d'un motif oxyéthylène est égal ou supérieur à 2, un groupe siloxane, un groupe oligosiloxane ayant deux atomes de silicium ou plus, ou un groupe trialkylsilyle substitué ou non substitué ;
dans lequel le nombre total d'atomes de carbone contenus dans L et R va de 5 à 12; 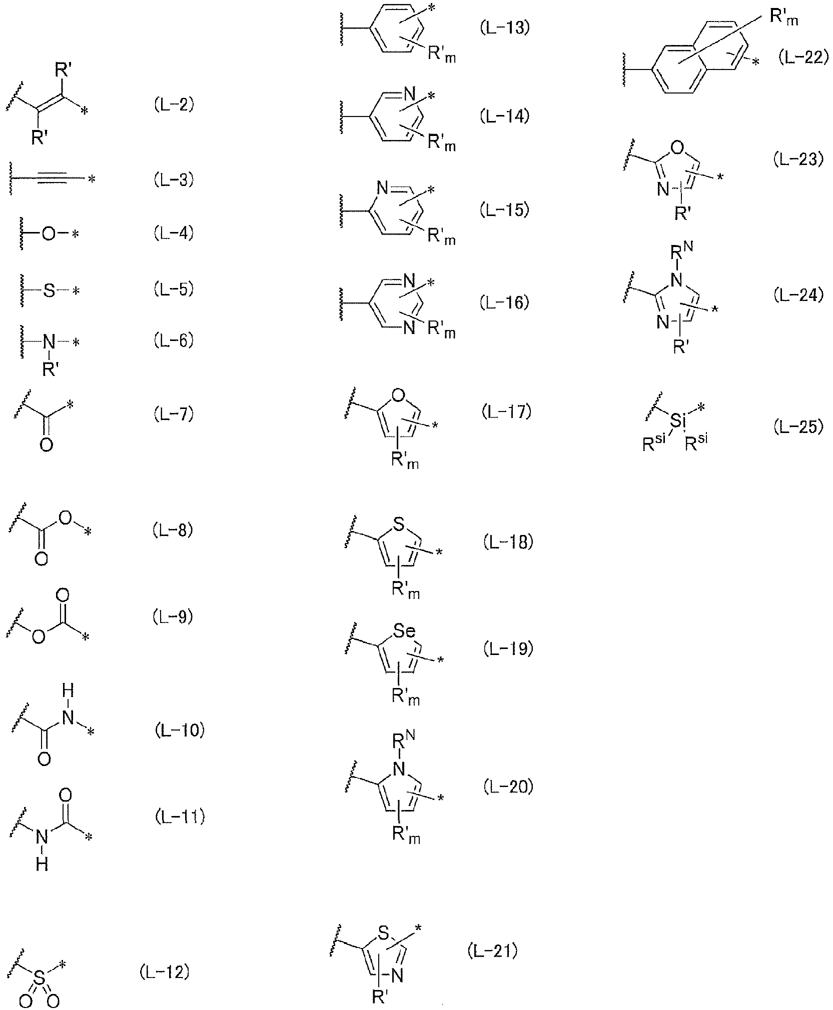
dans les formules (L-2) à (L-25),
la partie d'une ligne ondulée représente une position de liaison à un squelette cyclique condensé,
m dans la formule (L-13) représente 4, m dans les formules (L-14) et (L-15) représente 3, m dans les formules (L-16) à (L-20) représente 2, m dans la formule (L-22) représente 6,
chaque R' dans les formules (L-2), (L-6) et (L-13) à (L-24) représente indépendamment un atome d'hydrogène ou un substituant,
R^{N} représente un atome d'hydrogène ou un substituant, et
chaque R^{si} représente indépendamment un atome d'hydrogène, un groupe alkyle, un groupe alcényle, ou un groupe alcynyle, 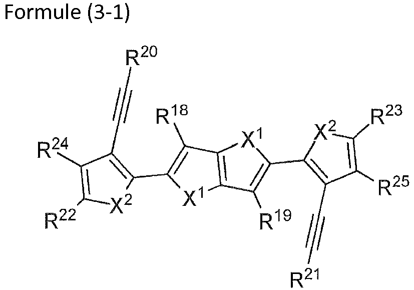
dans la formule (3-1),
X¹ et X² ont la même signification que celle définie ci-dessus,
chacun parmi R⁹ et R¹⁸ à R²⁵ représente indépendamment un atome d'hydrogène ou un substituant,
un ou deux parmi R⁹, R¹⁸, R¹⁹, R²⁰, R²¹, R²², R²³, R²⁴ et R²⁵ sont un substituant représenté par la formule (W) telle que définie précédemment et au moins l'un parmi R²² et R²³ représente un groupe alkyle, un groupe alcényle, un groupe alcynyle, un groupe aryle substitué ou non substitué, un groupe hétéroaryle substitué ou non substitué, un groupe alkylcarbonyle, ou un groupe arylcarbonyle substitué ou non substitué ; 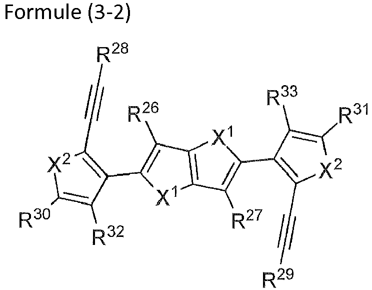
dans la formule (3-2),
chaque X¹ représente indépendamment un atome de soufre, un atome d'oxygène, un atome de sélénium ou NR⁹,
chaque X² représente indépendamment un atome de soufre, un atome d'oxygène ou un atome de sélénium,
chacun parmi R⁹ et R²⁶ à R³³ représente indépendamment un atome d'hydrogène ou un substituant, et au moins l'un parmi R³⁰ et R³¹ représente un groupe alkyle, un groupe alcényle, un groupe alcynyle, un groupe aryle substitué ou non substitué, un groupe hétéroaryle substitué ou non substitué, un groupe alkylcarbonyle, ou un groupe arylcarbonyle substitué ou non substitué.

2. Composé selon la revendication 1,
dans lequel le composé représenté par la formule (1-1) ou (1-2) est un composé représenté par l'une quelconque des formules (2-1-1), (2-1-2), (2-2-1) ou (2-2-2) suivantes ; 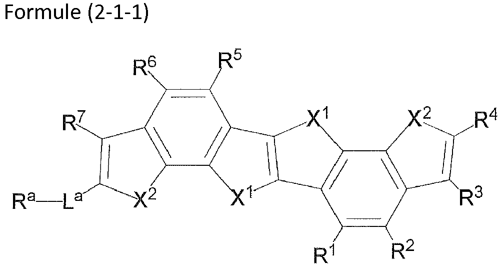 dans la formule (2-1-1),
chaque X¹, X², R¹ à R⁷ et R⁹ est tel que défini dans la revendication 1, et lorsque R¹, R², R³, R⁴, R⁵, R⁶, R⁷ ou R⁹ est un substituant, alors le substituant est un substituant autre que les substituants représentés par la formule (W), R⁴ n'est pas un groupe représenté par -L^{a}-R^{a},
L^{a} et R^{a} ont la même signification que L et R tels que définis dans la revendication 1 ; 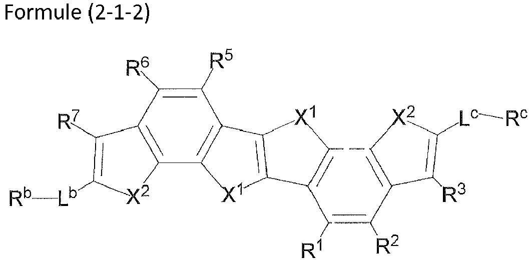
dans la formule (2-1-2),
X¹, X² et R¹ à R³, R⁵ à R⁷ et R⁹ ont la même signification que celle définie la revendication 1, et lorsque R¹, R², R³, R⁵, R⁶, R⁷ ou R⁹ est un substituant, alors le substituant est un substituant autre que les substituants représentés par la formule (W),
L^{b}, L^{c} R^{b} et R^{c} ont la même signification que L et R tels que définis dans la revendication 1, 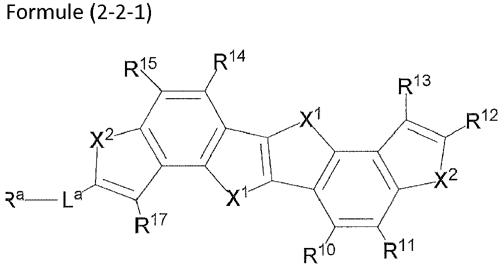
dans la formule (2-2-1),
X¹ et X² ont la même signification que celle définie dans la revendication 1,
chacun parmi R¹⁰, R¹² à R¹⁵, et R¹⁷ représente indépendamment un atome d'hydrogène ou un substituant, R¹² n'est pas un groupe représenté par -L^{a}-R^{a},
L^{a} et R^{a} ont la même signification que L et R tels que définis dans la revendication 1 ; 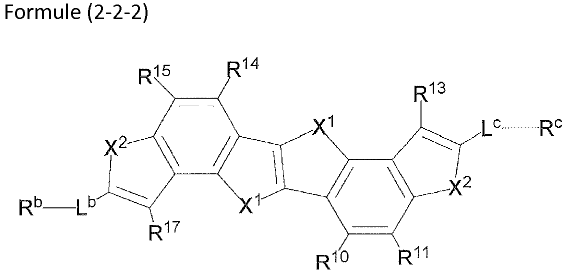
dans la formule (2-2-2),
X¹ et X² ont la même signification que celle définie dans la revendication 1,
chacun parmi R¹⁰, R¹¹, R¹³ à R¹⁵, et R¹⁷ représente indépendamment un atome d'hydrogène ou un substituant,
L^{b} et L^{c}, R^{b} et R^{c} ont la même signification que L et R tels que définis dans la revendication 1.

3. Composé selon la revendication 1 ou 2,
dans lequel dans les formules (1-2), (1-1), (2-1-1), (2-1-2), (2-2-1) et (2-2-2), chacun parmi L, L^{a}, L^{b} et L^{c} est indépendamment un groupe de liaison divalent représenté par l'une quelconque des formules (L-2) à (L-5), (L-13), (L-17) et (L-18).

4. Composé selon l'une quelconque des revendications 1 à 3,
dans lequel dans les formules (1-2), (1-1), (2-1-1), (2-1-2), (2-2-1) et (2-2-2), chacun parmi L, L^{a}, L^{b} et L^{c} est un groupe de liaison divalent représenté par l'une quelconque des formules (L-3), (L-13) et (L-18).

5. Composé selon l'une quelconque des revendications 1 à 4, dans lequel les substituants de chacun parmi R¹ à R³ dans la formule (1-1) et chacun parmi R¹⁰ à R¹⁷ dans la formule (1-2) sont un groupe alkyle ayant 1 à 18 atomes de carbone, un groupe aryle ayant 6 à 20 atomes de carbone, un groupe alcényle ayant 2 à 12 atomes de carbone, un groupe alcynyle ayant 2 à 12 atomes de carbone, un groupe alcoxy ayant 1 à 11 atomes de carbone, un groupe hétérocyclique ayant 5 à 12 atomes de carbone, un groupe alkyl-thio ayant 1 à 12 atomes de carbone ou le groupe de formule (W).

6. Transistor organique ou matériau semi-conducteur organique pour un dispositif semi-conducteur organique non luminescent ou un film semi-conducteur organique pour un dispositif semi-conducteur organique non luminescent, comprenant chacun un composé représenté par la formule (1-1) ou (1-2) tel que défini dans l'une quelconque des revendications 1 à 5, dans lequel dans le transistor organique ledit composé est compris dans une couche active semi-conductrice.

7. Utilisation pour un transistor organique d'un composé représenté par la formule (1-1) ou (1-2) tel que défini dans l'une quelconque des revendications 1 à 5.

8. Solution de revêtement pour un dispositif semi-conducteur organique non luminescent, comprenant un composé représenté par la formule (1-1) ou (1-2) tel que défini dans l'une quelconque des revendications 1 à 5.
